# EUROPEAN PATENT SPECIFICATION

(11) **EP 3 689 890 B1**
(45) Date of publication and mention of the grant of the patent: **08.03.2023**
(21) Application number: 20165603.0
(22) Date of filing: 02.01.2018
(51) Int. Cl.: C07F 15/00, C09K 11/06, H01L 51/00, H01L 51/50, H01L 51/52

(54) **ORGANIC ELECTROLUMINESCENT MATERIALS AND DEVICES**
ORGANISCHE ELEKTROLUMINESZENTE MATERIALIEN UND VORRICHTUNGEN
MATÉRIAUX ET DISPOSITIFS ÉLECTROLUMINESCENTS ORGANIQUES

(30) Priority: 09.01.2017 US 201762443908 P; 11.04.2017 US 201762484004 P; 15.12.2017 US 201715843710
(43) Date of publication of application: 05.08.2020
(62) Divisional of application: 18150061.2
(73) Proprietor: Universal Display Corporation, Ewing, NJ 08618 (US)
(72) Inventor: BOUDREAULT, Pierre-Luc T, Ewing, NJ 08618 (US); ALLEYNE, Bert, Ewing, NJ 08618 (US); JOSEPH, Scott, Ewing, NJ 08618 (US); WENDT, Harvey, Ewing, NJ 08618 (US)
(74) Representative: Maiwald GmbH

(56) References cited:
- US-A1- 2007 278 936

## Description

### CROSS-REFERENCE TO RELATED APPLICATIONS

### FIELD

The present invention relates to compounds for use as emitters, and devices, such as organic light emitting diodes, including the same.

### BACKGROUND

Opto-electronic devices that make use of organic materials are becoming increasingly desirable for a number of reasons. Many of the materials used to make such devices are relatively inexpensive, so organic opto-electronic devices have the potential for cost advantages over inorganic devices. In addition, the inherent properties of organic materials, such as their flexibility, may make them well suited for particular applications such as fabrication on a flexible substrate. Examples of organic opto-electronic devices include organic light emitting diodes/devices (OLEDs), organic phototransistors, organic photovoltaic cells, and organic photodetectors. For OLEDs, the organic materials may have performance advantages over conventional materials. For example, the wavelength at which an organic emissive layer emits light may generally be readily tuned with appropriate dopants.

OLEDs make use of thin organic films that emit light when voltage is applied across the device. OLEDs are becoming an increasingly interesting technology for use in applications such as flat panel displays, illumination, and backlighting. Several OLED materials and configurations are described in U.S. Pat. Nos. 5,844,363, 6,303,238, and 5,707,745.

One application for phosphorescent emissive molecules is a full color display. Industry standards for such a display call for pixels adapted to emit particular colors, referred to as "saturated" colors. In particular, these standards call for saturated red, green, and blue pixels. Alternatively the OLED can be designed to emit white light. In conventional liquid crystal displays emission from a white backlight is filtered using absorption filters to produce red, green and blue emission. The same technique can also be used with OLEDs. The white OLED can be either a single EML device or a stack structure. Color may be measured using CIE coordinates, which are well known to the art.

One example of a green emissive molecule is tris(2-phenylpyridine) iridium, denoted Ir(ppy)₃, which has the following structure:

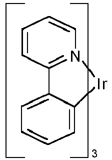

In this, and later figures herein, we depict the dative bond from nitrogen to metal (here, Ir) as a straight line.

As used herein, the term "organic" includes polymeric materials as well as small molecule organic materials that may be used to fabricate organic opto-electronic devices. "Small molecule" refers to any organic material that is not a polymer, and "small molecules" may actually be quite large. Small molecules may include repeat units in some circumstances. For example, using a long chain alkyl group as a substituent does not remove a molecule from the "small molecule" class. Small molecules may also be incorporated into polymers, for example as a pendent group on a polymer backbone or as a part of the backbone. Small molecules may also serve as the core moiety of a dendrimer, which consists of a series of chemical shells built on the core moiety. The core moiety of a dendrimer may be a fluorescent or phosphorescent small molecule emitter. A dendrimer may be a "small molecule," and it is believed that all dendrimers currently used in the field of OLEDs are small molecules.

As used herein, "top" means furthest away from the substrate, while "bottom" means closest to the substrate. Where a first layer is described as "disposed over" a second layer, the first layer is disposed further away from substrate. There may be other layers between the first and second layer, unless it is specified that the first layer is "in contact with" the second layer. For example, a cathode may be described as "disposed over" an anode, even though there are various organic layers in between.

As used herein, "solution processible" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

As used herein, and as would be generally understood by one skilled in the art, a first "Highest Occupied Molecular Orbital" (HOMO) or "Lowest Unoccupied Molecular Orbital" (LUMO) energy level is "greater than" or "higher than" a second HOMO or LUMO energy level if the first energy level is closer to the vacuum energy level. Since ionization potentials (IP) are measured as a negative energy relative to a vacuum level, a higher HOMO energy level corresponds to an IP having a smaller absolute value (an IP that is less negative). Similarly, a higher LUMO energy level corresponds to an electron affinity (EA) having a smaller absolute value (an EA that is less negative). On a conventional energy level diagram, with the vacuum level at the top, the LUMO energy level of a material is higher than the HOMO energy level of the same material. A "higher" HOMO or LUMO energy level appears closer to the top of such a diagram than a "lower" HOMO or LUMO energy level.

As used herein, and as would be generally understood by one skilled in the art, a first work function is "greater than" or "higher than" a second work function if the first work function has a higher absolute value. Because work functions are generally measured as negative numbers relative to vacuum level, this means that a "higher" work function is more negative. On a conventional energy level diagram, with the vacuum level at the top, a "higher" work function is illustrated as further away from the vacuum level in the downward direction. Thus, the definitions of HOMO and LUMO energy levels follow a different convention than work functions.

More details on OLEDs, and the definitions described above, can be found in US Pat. No. 7,279,704. An example for a red emitter complex of Ir(III) is described in US 2007/278936.

### SUMMARY

Disclosed herein are novel ligands for metal complexes that are useful as phosphorescent emitters in OLEDs. The ligands contain an aryl covalently bonded to the coordinating metal. This aryl group has at least two different alkyl side chains that are linked *ortho and para* compared to the linkage of the coordinating metal on that aryl group. The particular linkage will provide a better emission line shape as well as better external quantum efficiency of the emitters that are synthesized from those ligands.

A compound having a formula M(L_{A})ₓ(L_{B})_{y}(L_{C})_{z} is disclosed. In the compound, the ligand L_{A} has Formula I, 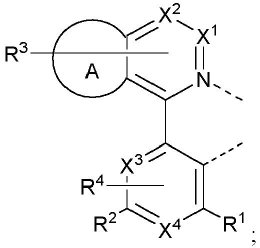 the ligand L_{B} has Formula II, 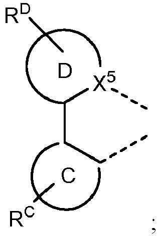 and the ligand L_{C} has Formula III, 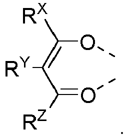 M is a metal having an atomic weight greater than 40. x is 1, 2, or 3. y is 0, 1, or 2. z is 0, 1, or 2. x+y+z is the oxidation state of the metal M. X¹ to X⁵ are each independently a carbon or nitrogen. Ring A is a 5- or 6-membered aromatic ring fused to the ring having X¹. Rings C and D are each independently a 5 or 6-membered carbocyclic or heterocyclic ring. R³, R⁴, R^{C}, and R^{D} each independently represents none to a maximum possible number of substitutions. Each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof. Any adjacent substituents of R³, R⁴, R^{C}, and R^{D}, R^{X}, R^{Y}, and R^{Z} are optionally joined or fused into a ring. R¹ and R² are each independently selected from the group consisting of alkyl, cycloalkyl, silyl, germanyl, and substituted variants thereof as defined in claim 1. Additionally, R¹ is different from R².

An OLED comprising an anode; a cathode; and an organic layer, disposed between the anode and the cathode is also disclosed, where the organic layer comprises the compound having the formula M(L_{A})ₓ(L_{B})_{y}(L_{C})_{z}.

A consumer product comprising the OLED is also disclosed.

### BRIEF DESCRIPTION OF THE DRAWINGS

FIG. 1 shows an organic light emitting device.
FIG. 2 shows an inverted organic light emitting device that does not have a separate electron transport layer.

### DETAILED DESCRIPTION

Generally, an OLED comprises at least one organic layer disposed between and electrically connected to an anode and a cathode. When a current is applied, the anode injects holes and the cathode injects electrons into the organic layer(s). The injected holes and electrons each migrate toward the oppositely charged electrode. When an electron and hole localize on the same molecule, an "exciton," which is a localized electron-hole pair having an excited energy state, is formed. Light is emitted when the exciton relaxes via a photoemissive mechanism. In some cases, the exciton may be localized on an excimer or an exciplex. Non-radiative mechanisms, such as thermal relaxation, may also occur, but are generally considered undesirable.

The initial OLEDs used emissive molecules that emitted light from their singlet states ("fluorescence") as disclosed, for example, in U.S. Pat. No. 4,769,292. Fluorescent emission generally occurs in a time frame of less than 10 nanoseconds.

More recently, OLEDs having emissive materials that emit light from triplet states ("phosphorescence") have been demonstrated. Baldo et al., "Highly Efficient Phosphorescent Emission from Organic Electroluminescent Devices," Nature, vol. 395, 151-154, 1998; ("Baldo-I") and Baldo et al., "Very high-efficiency green organic light-emitting devices based on electrophosphorescence," Appl. Phys. Lett., vol. 75, No. 3, 4-6 (1999) ("Baldo-II"). Phosphorescence is described in more detail in US Pat. No. 7,279,704 at cols. 5-6.

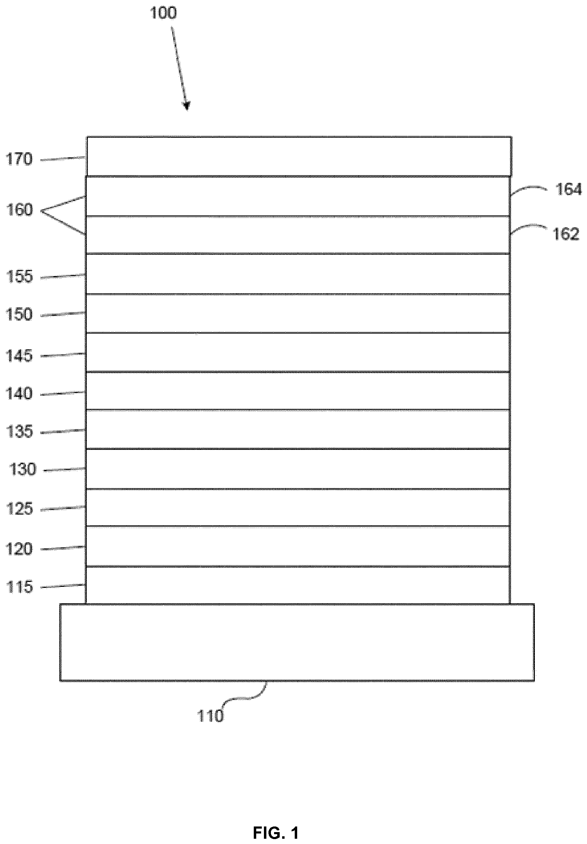

FIG. 1 shows an organic light emitting device 100. The figures are not necessarily drawn to scale. Device 100 may include a substrate 110, an anode 115, a hole injection layer 120, a hole transport layer 125, an electron blocking layer 130, an emissive layer 135, a hole blocking layer 140, an electron transport layer 145, an electron injection layer 150, a protective layer 155, a cathode 160, and a barrier layer 170. Cathode 160 is a compound cathode having a first conductive layer 162 and a second conductive layer 164. Device 100 may be fabricated by depositing the layers described, in order. The properties and functions of these various layers, as well as example materials, are described in more detail in US 7,279,704 at cols. 6-10.

More examples for each of these layers are available. For example, a flexible and transparent substrate-anode combination is disclosed in U.S. Pat. No. 5,844,363. An example of a p-doped hole transport layer is m-MTDATA doped with F₄-TCNQ at a molar ratio of 50:1, as disclosed in U.S. Patent Application Publication No. 2003/0230980. Examples of emissive and host materials are disclosed in U.S. Pat. No. 6,303,238 to Thompson et al.. An example of an n-doped electron transport layer is BPhen doped with Li at a molar ratio of 1:1, as disclosed in U.S. Patent Application Publication No. 2003/0230980. U.S. Pat. Nos. 5,703,436 and 5,707,745, disclose examples of cathodes including compound cathodes having a thin layer of metal such as Mg:Ag with an overlying transparent, electrically-conductive, sputter-deposited ITO layer. The theory and use of blocking layers is described in more detail in U.S. Pat. No. 6,097,147 and U.S. Patent Application Publication No. 2003/0230980. Examples of injection layers are provided in U.S. Patent Application Publication No. 2004/0174116. A description of protective layers may be found in U.S. Patent Application Publication No. 2004/0174116.

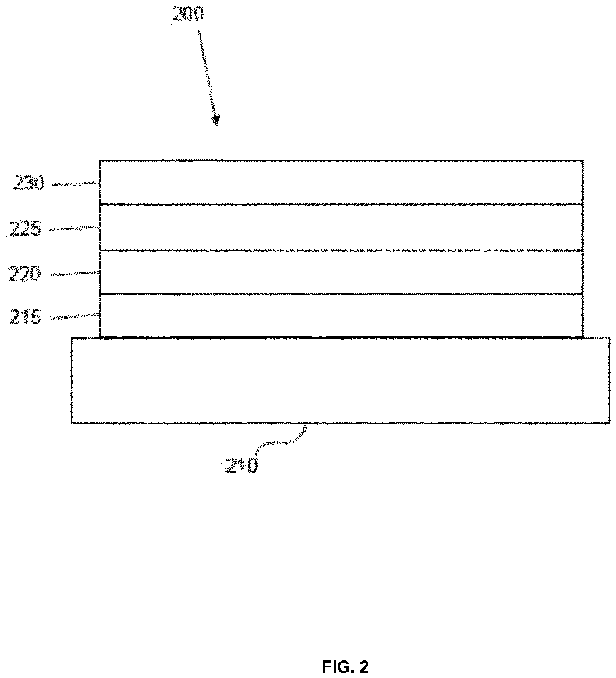

FIG. 2 shows an inverted OLED 200. The device includes a substrate 210, a cathode 215, an emissive layer 220, a hole transport layer 225, and an anode 230. Device 200 may be fabricated by depositing the layers described, in order. Because the most common OLED configuration has a cathode disposed over the anode, and device 200 has cathode 215 disposed under anode 230, device 200 may be referred to as an "inverted" OLED. Materials similar to those described with respect to device 100 may be used in the corresponding layers of device 200. FIG. 2 provides one example of how some layers may be omitted from the structure of device 100.

The simple layered structure illustrated in FIGS. 1 and 2 is provided by way of nonlimiting example, and it is understood that embodiments of the invention may be used in connection with a wide variety of other structures. The specific materials and structures described are exemplary in nature, and other materials and structures may be used. Functional OLEDs may be achieved by combining the various layers described in different ways, or layers may be omitted entirely, based on design, performance, and cost factors. Other layers not specifically described may also be included. Materials other than those specifically described may be used. Although many of the examples provided herein describe various layers as comprising a single material, it is understood that combinations of materials, such as a mixture of host and dopant, or more generally a mixture, may be used. Also, the layers may have various sublayers. The names given to the various layers herein are not intended to be strictly limiting. For example, in device 200, hole transport layer 225 transports holes and injects holes into emissive layer 220, and may be described as a hole transport layer or a hole injection layer. In one embodiment, an OLED may be described as having an "organic layer" disposed between a cathode and an anode. This organic layer may comprise a single layer, or may further comprise multiple layers of different organic materials as described, for example, with respect to FIGS. 1 and 2.

Structures and materials not specifically described may also be used, such as OLEDs comprised of polymeric materials (PLEDs) such as disclosed in U.S. Pat. No. 5,247,190 to Friend et al.. By way of further example, OLEDs having a single organic layer may be used. OLEDs may be stacked, for example as described in U.S. Pat. No. 5,707,745 to Forrest et al. The OLED structure may deviate from the simple layered structure illustrated in FIGS. 1 and 2. For example, the substrate may include an angled reflective surface to improve out-coupling, such as a mesa structure as described in U.S. Pat. No. 6,091,195 to Forrest et al., and/or a pit structure as described in U.S. Pat. No. 5,834,893 to Bulovic et al..

Unless otherwise specified, any of the layers of the various embodiments may be deposited by any suitable method. For the organic layers, preferred methods include thermal evaporation, ink-jet, such as described in U.S. Pat. Nos. 6,013,982 and 6,087,196, organic vapor phase deposition (OVPD), such as described in U.S. Pat. No. 6,337,102 to Forrest et al., and deposition by organic vapor jet printing (OVJP), such as described in U.S. Pat. No. 7,431,968. Other suitable deposition methods include spin coating and other solution based processes. Solution based processes are preferably carried out in nitrogen or an inert atmosphere. For the other layers, preferred methods include thermal evaporation. Preferred patterning methods include deposition through a mask, cold welding such as described in U.S. Pat. Nos. 6,294,398 and 6,468,819, and patterning associated with some of the deposition methods such as ink-jet and OVJD. Other methods may also be used. The materials to be deposited may be modified to make them compatible with a particular deposition method. For example, substituents such as alkyl and aryl groups, branched or unbranched, and preferably containing at least 3 carbons, may be used in small molecules to enhance their ability to undergo solution processing. Substituents having 20 carbons or more may be used, and 3-20 carbons is a preferred range. Materials with asymmetric structures may have better solution processibility than those having symmetric structures, because asymmetric materials may have a lower tendency to recrystallize. Dendrimer substituents may be used to enhance the ability of small molecules to undergo solution processing.

Devices fabricated in accordance with embodiments of the present invention may further optionally comprise a barrier layer. One purpose of the barrier layer is to protect the electrodes and organic layers from damaging exposure to harmful species in the environment including moisture, vapor and/or gases, etc. The barrier layer may be deposited over, under or next to a substrate, an electrode, or over any other parts of a device including an edge. The barrier layer may comprise a single layer, or multiple layers. The barrier layer may be formed by various known chemical vapor deposition techniques and may include compositions having a single phase as well as compositions having multiple phases. Any suitable material or combination of materials may be used for the barrier layer. The barrier layer may incorporate an inorganic or an organic compound or both. The preferred barrier layer comprises a mixture of a polymeric material and a non-polymeric material as described in U.S. Pat. No. 7,968,146, PCT Pat. Application Nos. PCT/US2007/023098 and PCT/US2009/042829. To be considered a "mixture", the aforesaid polymeric and non-polymeric materials comprising the barrier layer should be deposited under the same reaction conditions and/or at the same time. The weight ratio of polymeric to non-polymeric material may be in the range of 95:5 to 5:95. The polymeric material and the non-polymeric material may be created from the same precursor material. In one example, the mixture of a polymeric material and a non-polymeric material consists essentially of polymeric silicon and inorganic silicon.

Devices fabricated in accordance with embodiments of the invention can be incorporated into a wide variety of electronic component modules (or units) that can be incorporated into a variety of electronic products or intermediate components. Examples of such electronic products or intermediate components include display screens, lighting devices such as discrete light source devices or lighting panels, etc. that can be utilized by the end-user product manufacturers. Such electronic component modules can optionally include the driving electronics and/or power source(s). Devices fabricated in accordance with embodiments of the invention can be incorporated into a wide variety of consumer products that have one or more of the electronic component modules (or units) incorporated therein. A consumer product comprising an OLED that includes the compound of the present disclosure in the organic layer in the OLED is disclosed. Such consumer products would include any kind of products that include one or more light source(s) and/or one or more of some type of visual displays. Some examples of such consumer products include flat panel displays, computer monitors, medical monitors, televisions, billboards, lights for interior or exterior illumination and/or signaling, heads-up displays, fully or partially transparent displays, flexible displays, laser printers, telephones, mobile phones, tablets, phablets, personal digital assistants (PDAs), wearable devices, laptop computers, digital cameras, camcorders, viewfinders, micro-displays (displays that are less than (5,08 cm (2 inches) diagonal), 3-D displays, virtual reality or augmented reality displays, vehicles, video walls comprising multiple displays tiled together, theater or stadium screen, and a sign. Various control mechanisms may be used to control devices fabricated in accordance with the present invention, including passive matrix and active matrix. Many of the devices are intended for use in a temperature range comfortable to humans, such as 18 degrees C. to 30 degrees C., and more preferably at room temperature (20-25 degrees C), but could be used outside this temperature range, for example, from -40 degree C to + 80 degree C.

The materials and structures described herein may have applications in devices other than OLEDs. For example, other optoelectronic devices such as organic solar cells and organic photodetectors may employ the materials and structures. More generally, organic devices, such as organic transistors, may employ the materials and structures.

The term "halo," "halogen," or "halide" as used herein includes fluorine, chlorine, bromine, and iodine.

The term "alkyl" as used herein contemplates both straight and branched chain alkyl radicals. Preferred alkyl groups are those containing from one to fifteen carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl,and the like. Additionally, the alkyl group may be optionally substituted.

The term "cycloalkyl" as used herein contemplates cyclic alkyl radicals. Preferred cycloalkyl groups are those containing 3 to 10 ring carbon atoms and includes cyclopropyl, cyclopentyl, cyclohexyl, adamantyl, and the like. Additionally, the cycloalkyl group may be optionally substituted.

The term "alkenyl" as used herein contemplates both straight and branched chain alkene radicals. Preferred alkenyl groups are those containing two to fifteen carbon atoms. Additionally, the alkenyl group may be optionally substituted.

The term "alkynyl" as used herein contemplates both straight and branched chain alkyne radicals. Preferred alkynyl groups are those containing two to fifteen carbon atoms. Additionally, the alkynyl group may be optionally substituted.

The terms "aralkyl" or "arylalkyl" as used herein are used interchangeably and contemplate an alkyl group that has as a substituent an aromatic group. Additionally, the aralkyl group may be optionally substituted.

The term "heterocyclic group" as used herein contemplates aromatic and nonaromatic cyclic radicals. Hetero-aromatic cyclic radicals also means heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers, such as tetrahydrofuran, tetrahydropyran, and the like. Additionally, the heterocyclic group may be optionally substituted.

The term "aryl" or "aromatic group" as used herein contemplates single-ring groups and polycyclic ring systems. The polycyclic rings may have two or more rings in which two carbons are common to two adjoining rings (the rings are "fused") wherein at least one of the rings is aromatic, e.g., the other rings can be cycloalkyls, cycloalkenyls, aryl, heterocycles, and/or heteroaryls. Preferred aryl groups are those containing six to thirty carbon atoms, preferably six to twenty carbon atoms, more preferably six to twelve carbon atoms. Especially preferred is an aryl group having six carbons, ten carbons or twelve carbons. Suitable aryl groups include phenyl, biphenyl, triphenyl, triphenylene, tetraphenylene, naphthalene, anthracene, phenalene, phenanthrene, fluorene, pyrene, chrysene, perylene, and azulene, preferably phenyl, biphenyl, triphenyl, triphenylene, fluorene, and naphthalene. Additionally, the aryl group may be optionally substituted.

The term "heteroaryl" as used herein contemplates single-ring hetero-aromatic groups that may include from one to five heteroatoms. The term heteroaryl also includes polycyclic hetero-aromatic systems having two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group may be optionally substituted.

The alkyl, cycloalkyl, alkenyl, alkynyl, aralkyl, heterocyclic group, aryl, and heteroaryl may be unsubstituted or may be substituted with one or more substituents selected from the group consisting of deuterium, halogen, alkyl, cycloalkyl, heteroalkyl, arylalkyl, alkoxy, aryloxy, amino, cyclic amino, silyl, alkenyl, cycloalkenyl, heteroalkenyl, alkynyl, aryl, heteroaryl, acyl, carbonyl, carboxylic acid, ether, ester, nitrile, isonitrile, sulfanyl, sulfinyl, sulfonyl, phosphino, and combinations thereof.

As used herein, "substituted" indicates that a substituent other than H is bonded to the relevant position, such as carbon. Thus, for example, where R¹ is mono-substituted, then one R¹ must be other than H. Similarly, where R¹ is di-substituted, then two of R¹ must be other than H. Similarly, where R¹ is unsubstituted, R¹ is hydrogen for all available positions.

The "aza" designation in the fragments described herein, i.e. aza-dibenzofuran, azadibenzothiophene, etc. means that one or more of the C-H groups in the respective fragment can be replaced by a nitrogen atom, for example, and without any limitation, azatriphenylene encompasses both dibenzo[f,h]quinoxaline and dibenzo[f,h]quinoline. One of ordinary skill in the art can readily envision other nitrogen analogs of the aza-derivatives described above, and all such analogs are intended to be encompassed by the terms as set forth herein.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

Phosphorescent metal complexes containing ligands bearing two different side chains on the phenyl at the bottom of the ligand (*ortho and para* to the coordinating metal) on a cyclometallated emitter. The mentioned side chains can be either linear, branched or cyclic aliphatic chains. Partially or fully fluorinated or deuterated side chains can also be used. Having two different substituents results in the emitter with improved line shape of the emission and also increase the External Quantum Efficiency (EQE) of the emitter. Moreover, depending on the nature of the side chain, it is possible to fine-tune the color of the emission of the emitter.

A compound having a formula M(L_{A})ₓ(L_{B})_{y}(L_{C})_{z} is disclosed. In the compound, the ligand L_{A} has Formula I, 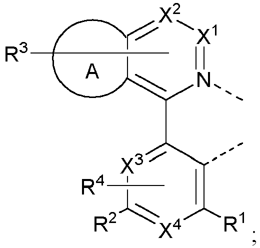 the ligand L_{B} has Formula II, 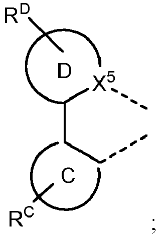 and the ligand L_{C} has Formula III, 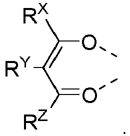 M is a metal having an atomic weight greater than 40. x is 1, 2, or 3. y is 0, 1, or 2. z is 0, 1, or 2. x+y+z is the oxidation state of the metal M. X¹ to X⁵ are each independently a carbon or nitrogen. Ring A is a 5- or 6-membered aromatic ring fused to the ring having X¹. Rings C and D are each independently a 5 or 6-membered carbocyclic or heterocyclic ring. R³, R⁴, R^{C}, and R^{D} each independently represents none to a maximum possible number of substitutions. Each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof. Any adjacent substituents of R³, R⁴, R^{C}, and R^{D}, R^{X}, R^{Y}, and R^{Z} are optionally joined or fused into a ring. R¹ and R² are each independently selected from the group consisting of alkyl, cycloalkyl, silyl, germanyl, and substituted variants thereof as defined in claim 1. Additionally, R¹ is different from R².

In some embodiments of the compound, M is selected from the group consisting of Ir, Rh, Re, Ru, Os, Pt, Au, and Cu. In some embodiments, M is Ir. In some embodiments of the compound, X¹ to X⁴ are each a carbon.

In some embodiments, the compound has a formula M(L_{A})₂(L_{C}). In some embodiments, the compound has a formula M(L_{A})(L_{B})₂.

In some embodiments of the compound, ring A is a 5-membered aromatic ring. In some embodiments, ring A is a 6-membered aromatic ring.

In some embodiments, R¹ and R² are each independently selected from the group consisting of methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, cyclopentyl, cyclohexyl, norbornyl, adamantyl, trimethylsilyl, triethylsilyl, triisopropylsilyl, trimethylgermyl, triethylgermyl, and triisopropylgermyl, partially or fully deuterated variants thereof, partially or fully fluorinated variants thereof, and combinations thereof. In some embodiments, R² has at least two more carbon atoms than R¹. In some embodiments, R² has at least three more carbon atoms than R¹. In some embodiments, R² has at least four more carbon atoms than R¹. In some embodiments, R² has at least five more carbon atoms than R¹.

In some embodiments of the compound having the formula M(L_{A})ₓ(L_{B})_{y}(L_{C})_{z}, L_{C} has Formula IIIa 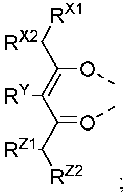 where R^{X1}, R^{X2}, R^{Z1}, and R^{Z2} are independently selected from group consisting of alkyl, cycloalkyl, aryl, and heteroaryl; and where at least one of R^{X1}, R^{X2}, R^{Z1}, and R^{Z2} has at least two carbon atoms.

In some embodiments of the compound having the formula M(L_{A})ₓ(L_{B})_{y}(L_{C})_{z}, each of R³, R⁴, R^{C}, and R^{D}, R^{X}, R^{Y}, and R^{Z} are independently selected from the group consisting of hydrogen, deuterium, alkyl, cycloalkyl, and combinations thereof. In some embodiments, R^{Y} is hydrogen.

In some embodiments of the compound, ring C is benzene, and ring D is pyridine of which X⁹ is N.

In some embodiments of the compound, the ligand L_{A} is selected from the group consisting of 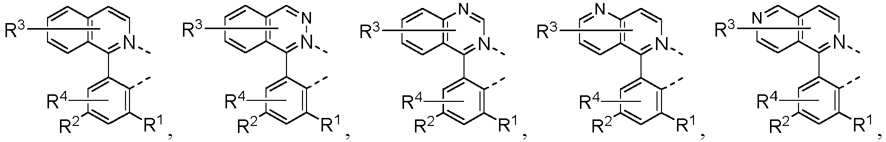 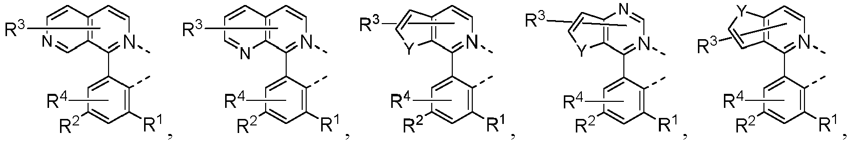 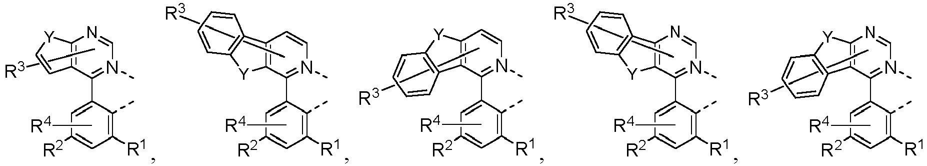 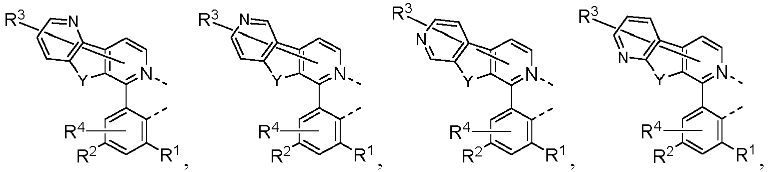 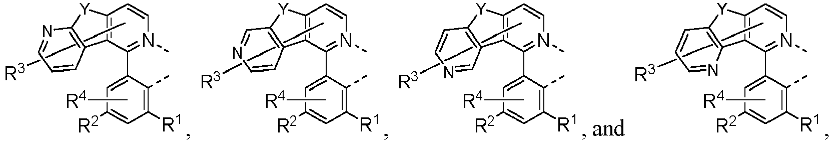 wherein Y is selected from the group consisting of S, O, Se, and N(CH₃).

In some embodiments of the compound, the ligand L_{A} is selected from the group consisting of L_{A1} through L_{A544}, that are based on a structure of Formula Ia, 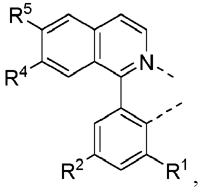 in each of which R¹, R², R⁴, and R⁵ are defined as follows:

| | **R¹** | **R²** | **R⁴** | **R⁵** | | **R¹** | **R²** | **R⁴** | **R⁵** |
|---|---|---|---|---|---|---|---|---|---|
| L_{A1} | R^{B1} | R^{B3} | H | H | L_{A273} | R^{B43} | R^{B12} | R^{B3} | H |
| L_{A2} | R^{B1} | R^{B3} | RB1 | H | L_{A274} | R^{B43} | R^{B12} | R^{B4} | H |
| L_{A3} | R^{B1} | R^{B3} | R^{B3} | H | L_{A275} | R^{B43} | R^{B12} | RB7 | H |
| L_{A4} | R^{B1} | R^{B3} | R^{B4} | H | L_{A276} | R^{B43} | R^{B12} | R^{B12} | H |
| L_{A5} | R^{B1} | R^{B3} | R^{B7} | H | L_{A277} | R^{B43} | R^{B12} | R^{B18} | H |
| L_{A6} | R^{B1} | R^{B3} | R^{B12} | H | L_{A278} | R^{B43} | R^{B12} | R^{A3} | H |
| L_{A7} | R^{B1} | R^{B3} | R^{B18} | H | L_{A279} | R^{B43} | R^{B12} | R^{A34} | H |
| L_{A8} | R^{B1} | R^{B3} | R^{A3} | H | L_{A280} | R^{B43} | R^{A34} | H | H |
| L_{A9} | R^{B1} | R^{B3} | R^{A34} | H | L_{A281} | R^{B43} | R^{A34} | R^{B1} | H |
| L_{A10} | R^{B1} | R^{B4} | H | H | L_{A282} | R^{B43} | R^{A34} | R^{B3} | H |
| L_{A11} | R^{B1} | R^{B4} | R^{B1} | H | L_{A283} | R^{B43} | R^{A34} | R^{B4} | H |
| L_{A12} | R^{B1} | R^{B4} | R^{B3} | H | L_{A284} | R^{B43} | R^{A34} | R^{B7} | H |
| L_{A13} | R^{B1} | R^{B4} | R^{B4} | H | L_{A285} | R^{B43} | R^{A34} | R^{B12} | H |
| L_{A14} | R^{B1} | R^{B4} | R^{B7} | H | L_{A286} | R^{B43} | R^{A34} | R^{B18} | H |
| L_{A15} | R^{B1} | R^{B4} | R^{B12} | H | L_{A287} | R^{B43} | R^{A34} | R^{A3} | H |
| L_{A16} | R^{B1} | R^{B4} | R^{B18} | H | L_{A288} | R^{B43} | R^{A34} | R^{A34} | H |
| L_{A17} | R^{B1} | R^{B4} | R^{A3} | H | L_{A289} | R^{B1} | R^{B3} | H | R^{B1} |
| L_{A18} | R^{B1} | R^{B4} | R^{A34} | H | L_{A290} | R^{B1} | R^{B3} | H | R^{B3} |
| L_{A19} | R^{B1} | R^{B5} | H | H | L_{A291} | R^{B1} | R^{B3} | H | R^{B4} |
| L_{A20} | R^{B1} | R^{B5} | R^{B1} | H | L_{A292} | R^{B1} | R^{B3} | H | R^{B7} |
| L_{A21} | R^{B1} | R^{B5} | R^{B3} | H | L_{A293} | R^{B1} | R^{B3} | H | R^{B12} |
| L_{A22} | R^{B1} | R^{B5} | R^{B4} | H | L_{A294} | R^{B1} | R^{B3} | H | R^{B18} |
| L_{A23} | R^{B1} | R^{B5} | R^{B7} | H | L_{A295} | R^{B1} | R^{B3} | H | R^{A3} |
| L_{A24} | R^{B1} | R^{B5} | R^{B12} | H | L_{A296} | R^{B1} | R^{B3} | H | R^{A34} |
| L_{A25} | R^{B1} | R^{B5} | R^{B18} | H | L_{A297} | R^{B1} | R^{B4} | H | R^{B1} |
| L_{A26} | R^{B1} | R^{B5} | R^{A3} | H | L_{A298} | R^{B1} | R^{B4} | H | R^{B3} |
| L_{A27} | R^{B1} | R^{B5} | R^{A34} | H | L_{A299} | R^{B1} | R^{B4} | H | R^{B4} |
| L_{A28} | R^{B1} | R^{B6} | H | H | L_{A300} | R^{B1} | R^{B4} | H | R^{B7} |
| L_{A29} | R^{B1} | R^{B6} | R^{B1} | H | L_{A301} | R^{B1} | R^{B4} | H | R^{B12} |
| L_{A30} | R^{B1} | R^{B6} | R^{B3} | H | L_{A302} | R^{B1} | R^{B4} | H | R^{B18} |
| L_{A31} | R^{B1} | R^{B6} | R^{B4} | H | L_{A303} | R^{B1} | R^{B4} | H | R^{A3} |
| L_{A32} | R^{B1} | R^{B6} | R^{B7} | H | L_{A304} | R^{B1} | R^{B4} | H | R^{A34} |
| L_{A33} | R^{B1} | R^{B6} | R^{B12} | H | L_{A305} | R^{B1} | R^{B5} | H | R^{B1} |
| L_{A34} | R^{B1} | R^{B6} | R^{B18} | H | L_{A306} | R^{B1} | R^{B5} | H | R^{B3} |
| L_{A35} | R^{B1} | R^{B6} | R^{A3} | H | L_{A307} | R^{B1} | R^{B5} | H | R^{B4} |
| L_{A36} | R^{B1} | R^{B6} | R^{A34} | H | L_{A308} | R^{B1} | R^{B5} | H | R^{B7} |
| L_{A37} | R^{B1} | R^{B7} | H | H | L_{A309} | R^{B1} | R^{B5} | H | R^{B12} |
| L_{A38} | R^{B1} | R^{B7} | R^{B1} | H | L_{A310} | R^{B1} | R^{B5} | H | R^{B18} |
| L_{A39} | R^{B1} | R^{B7} | R^{B3} | H | L_{A311} | R^{B1} | R^{B5} | H | R^{A3} |
| L_{A40} | R^{B1} | R^{B7} | R^{B4} | H | L_{A312} | R^{B1} | R^{B5} | H | R^{A34} |
| L_{A41} | R^{B1} | R^{B7} | R^{B7} | H | L_{A313} | R^{B1} | R^{B6} | H | R^{B1} |
| L_{A42} | R^{B1} | R^{B7} | R^{B12} | H | L_{A314} | R^{B1} | R^{B6} | H | R^{B3} |
| L_{A43} | R^{B1} | R^{B7} | R^{B18} | H | L_{A315} | R^{B1} | R^{B6} | H | R^{B4} |
| L_{A44} | R^{B1} | R^{B7} | R^{A3} | H | L_{A316} | R^{B1} | R^{B6} | H | R^{B7} |
| L_{A45} | R^{B1} | R^{B7} | R^{A34} | H | L_{A317} | R^{B1} | R^{B6} | H | R^{B12} |
| L_{A46} | R^{B1} | R^{B12} | H | H | L_{A318} | R^{B1} | R^{B6} | H | R^{B18} |
| L_{A47} | R^{B1} | R^{B12} | R^{B1} | H | L_{A319} | R^{B1} | R^{B6} | H | R^{A3} |
| L_{A48} | R^{B1} | R^{B12} | R^{B3} | H | L_{A320} | R^{B1} | R^{B6} | H | R^{A34} |
| L_{A49} | R^{B1} | R^{B12} | R^{B4} | H | L_{A321} | R^{B1} | R^{B7} | H | R^{B1} |
| L_{A50} | R^{B1} | R^{B12} | R^{B7} | H | L_{A322} | R^{B1} | R^{B7} | H | R^{B3} |
| L_{A51} | R^{B1} | R^{B12} | R^{B12} | H | L_{A323} | R^{B1} | R^{B7} | H | R^{B4} |
| L_{A52} | R^{B1} | R^{B12} | R^{B18} | H | L_{A324} | R^{B1} | R^{B7} | H | R^{B7} |
| L_{A53} | R^{B1} | R^{B12} | R^{A3} | H | L_{A325} | R^{B1} | R^{B7} | H | R^{B12} |
| L_{A54} | R^{B1} | R^{B12} | R^{A34} | H | L_{A326} | R^{B1} | R^{B7} | H | R^{B18} |
| L_{ASS} | R^{B1} | R^{A34} | H | H | L_{A327} | R^{B1} | R^{B7} | H | R^{A3} |
| L_{A56} | R^{B1} | R^{A34} | R^{B1} | H | L_{A328} | R^{B1} | R^{B7} | H | R^{A34} |
| L_{A57} | R^{B1} | R^{A34} | R^{B3} | H | L_{A329} | R^{B1} | R^{B12} | H | R^{B1} |
| L_{A58} | R^{B1} | R^{A34} | R^{B4} | H | L_{A330} | R^{B1} | R^{B12} | H | R^{B3} |
| L_{A59} | R^{B1} | R^{A34} | R^{B7} | H | L_{A331} | R^{B1} | R^{B12} | H | R^{B4} |
| L_{A60} | R^{B1} | R^{A34} | R^{B12} | H | L_{A332} | R^{B1} | R^{B12} | H | R^{B7} |
| L_{A61} | R^{B1} | R^{A34} | R^{B18} | H | L_{A333} | R^{B1} | R^{B12} | H | R^{B12} |
| L_{A62} | R^{B1} | R^{A34} | R^{A3} | H | L_{A334} | R^{B1} | R^{B12} | H | R^{B18} |
| L_{A63} | R^{B1} | R^{A34} | R^{A34} | H | L_{A335} | R^{B1} | R^{B12} | H | R^{A3} |
| L_{A64} | R^{B3} | R^{B4} | H | H | L_{A336} | R^{B1} | R^{B12} | H | R^{A34} |
| L_{A65} | R^{B3} | R^{B4} | R^{B1} | H | L_{A337} | R^{B1} | R^{A34} | H | R^{B1} |
| L_{A66} | R^{B3} | R^{B4} | R^{B3} | H | L_{A338} | R^{B1} | R^{A34} | H | R^{B3} |
| L_{A67} | R^{B3} | R^{B4} | R^{B4} | H | L_{A339} | R^{B1} | R^{A34} | H | R^{B4} |
| L_{A68} | R^{B3} | R^{B4} | R^{B7} | H | L_{A340} | R^{B1} | R^{A34} | H | R^{B7} |
| L_{A69} | R^{B3} | R^{B4} | R^{B12} | H | L_{A341} | R^{B1} | R^{A34} | H | R^{B12} |
| L_{A70} | R^{B3} | R^{B4} | R^{B18} | H | L_{A342} | R^{B1} | R^{A34} | H | R^{B18} |
| L_{A71} | R^{B3} | R^{B4} | R^{A3} | H | L_{A343} | R^{B1} | R^{A34} | H | R^{A3} |
| L_{A72} | R^{B3} | R^{B4} | R^{A34} | H | L_{A344} | R^{B1} | R^{A34} | H | R^{A34} |
| L_{A73} | R^{B3} | R^{B5} | H | H | L_{A345} | R^{B3} | R^{B4} | H | R^{B1} |
| L_{A74} | R^{B3} | R^{B5} | R^{B1} | H | L_{A346} | R^{B3} | R^{B4} | H | R^{B3} |
| L_{A75} | R^{B3} | R^{B5} | R^{B3} | H | L_{A347} | R^{B3} | R^{B4} | H | R^{B4} |
| L_{A76} | R^{B3} | R^{B5} | R^{B4} | H | L_{A348} | R^{B3} | R^{B4} | H | R^{B7} |
| L_{A77} | R^{B3} | R^{B5} | R^{B7} | H | L_{A349} | R^{B3} | R^{B4} | H | R^{B12} |
| L_{A78} | R^{B3} | R^{B5} | R^{B12} | H | L_{A350} | R^{B3} | R^{B4} | H | R^{B18} |
| L_{A79} | R^{B3} | R^{B5} | R^{B18} | H | L_{A351} | R^{B3} | R^{B4} | H | R^{A3} |
| L_{A80} | R^{B3} | R^{B5} | R^{A3} | H | L_{A352} | R^{B3} | R^{B4} | H | R^{A34} |
| L_{A81} | R^{B3} | R^{B5} | R^{A34} | H | L_{A353} | R^{B3} | R^{B5} | H | R^{B1} |
| L_{A82} | R^{B3} | R^{B6} | H | H | L_{A354} | R^{B3} | R^{B5} | H | R^{B3} |
| L_{A83} | R^{B3} | R^{B6} | R^{B1} | H | L_{A355} | R^{B3} | R^{B5} | H | R^{B4} |
| L_{A84} | R^{B3} | R^{B6} | R^{B3} | H | L_{A356} | R^{B3} | R^{B5} | H | R^{B7} |
| L_{A85} | R^{B3} | R^{B6} | R^{B4} | H | L_{A357} | R^{B3} | R^{B5} | H | R^{B12} |
| L_{A86} | R^{B3} | R^{B6} | R^{B7} | H | L_{A358} | R^{B3} | R^{B5} | H | R^{B18} |
| L_{A87} | R^{B3} | R^{B6} | R^{B12} | H | L_{A359} | R^{B3} | R^{B5} | H | R^{A3} |
| L_{A88} | R^{B3} | R^{B6} | R^{B18} | H | L_{A360} | R^{B3} | R^{B5} | H | R^{A34} |
| L_{A89} | R^{B3} | R^{B6} | R^{A3} | H | L_{A361} | R^{B3} | R^{B6} | H | R^{B1} |
| L_{A90} | R^{B3} | R^{B6} | R^{A34} | H | L_{A362} | R^{B3} | R^{B6} | H | R^{B3} |
| L_{A91} | R^{B3} | R^{B7} | H | H | L_{A363} | R^{B3} | R^{B6} | H | R^{B4} |
| L_{A92} | R^{B3} | R^{B7} | R^{B1} | H | L_{A364} | R^{B3} | R^{B6} | H | R^{B7} |
| L_{A93} | R^{B3} | R^{B7} | R^{B3} | H | L_{A365} | R^{B3} | R^{B6} | H | R^{B12} |
| L_{A94} | R^{B3} | R^{B7} | R^{B4} | H | L_{A366} | R^{B3} | R^{B6} | H | R^{B18} |
| L_{A95} | R^{B3} | R^{B7} | R^{B7} | H | L_{A367} | R^{B3} | R^{B6} | H | R^{A3} |
| L_{A96} | R^{B3} | R^{B7} | R^{B12} | H | L_{A368} | R^{B3} | R^{B6} | H | R^{A34} |
| L_{A97} | R^{B3} | R^{B7} | R^{B18} | H | L_{A369} | R^{B3} | R^{B7} | H | R^{B1} |
| L_{A98} | R^{B3} | R^{B7} | R^{A3} | H | L_{A370} | R^{B3} | R^{B7} | H | R^{B3} |
| L_{A99} | R^{B3} | R^{B7} | R^{A34} | H | L_{A371} | R^{B3} | R^{B7} | H | R^{B4} |
| L_{A100} | R^{B3} | R^{B12} | H | H | L_{A372} | R^{B3} | R^{B7} | H | R^{B7} |
| L_{A101} | R^{B3} | R^{B12} | R^{B1} | H | L_{A373} | R^{B3} | R^{B7} | H | R^{B12} |
| L_{A102} | R^{B3} | R^{B12} | R^{B3} | H | L_{A374} | R^{B3} | R^{B7} | H | R^{B18} |
| L_{A103} | R^{B3} | R^{B12} | R^{B4} | H | L_{A375} | R^{B3} | R^{B7} | H | R^{A3} |
| L_{A104} | R^{B3} | R^{B12} | R^{B7} | H | L_{A376} | R^{B3} | R^{B7} | H | R^{A34} |
| L_{A105} | R^{B3} | R^{B12} | R^{B12} | H | L_{A377} | R^{B3} | R^{B12} | H | R^{B1} |
| L_{A106} | R^{B3} | R^{B12} | R^{B18} | H | L_{A378} | R^{B3} | R^{B12} | H | R^{B3} |
| L_{A107} | R^{B3} | R^{B12} | R^{A3} | H | L_{A379} | R^{B3} | R^{B12} | H | R^{B4} |
| L_{A108} | R^{B3} | R^{B12} | R^{A34} | H | L_{A380} | R^{B3} | R^{B12} | H | R^{B7} |
| L_{A109} | R^{B3} | R^{A34} | H | H | L_{A381} | R^{B3} | R^{B12} | H | R^{B12} |
| L_{A110} | R^{B3} | R^{A34} | R^{B1} | H | L_{A382} | R^{B3} | R^{B12} | H | R^{B18} |
| L_{A111} | R^{B3} | R^{A34} | R^{B3} | H | L_{A383} | R^{B3} | R^{B12} | H | R^{A3} |
| L_{A112} | R^{B3} | R^{A34} | R^{B4} | H | L_{A384} | R^{B3} | R^{B12} | H | R^{A34} |
| L_{A113} | R^{B3} | R^{A34} | R^{B7} | H | L_{A385} | R^{B3} | R^{A34} | H | R^{B1} |
| L_{A114} | R^{B3} | R^{A34} | R^{B12} | H | L_{A386} | R^{B3} | R^{A34} | H | R^{B3} |
| L_{A115} | R^{B3} | R^{A34} | R^{B18} | H | L_{A387} | R^{B3} | R^{A34} | H | R^{B4} |
| L_{A116} | R^{B3} | R^{A34} | R^{A3} | H | L_{A388} | R^{B3} | R^{A34} | H | R^{B7} |
| L_{A117} | R^{B3} | R^{A34} | R^{A34} | H | L_{A389} | R^{B3} | R^{A34} | H | R^{B12} |
| L_{A118} | R^{B4} | R^{B3} | H | H | L_{A390} | R^{B3} | R^{A34} | H | R^{B18} |
| L_{A119} | R^{B4} | R^{B3} | R^{B1} | H | L_{A391} | R^{B3} | R^{A34} | H | R^{A3} |
| L_{A120} | R^{B4} | R^{B3} | R^{B3} | H | L_{A392} | R^{B3} | R^{A34} | H | R^{A34} |
| L_{A121} | R^{B4} | R^{B3} | R^{B4} | H | L_{A393} | R^{B4} | R^{B3} | H | R^{B1} |
| L_{A122} | R^{B4} | R^{B3} | R^{B7} | H | L_{A394} | R^{B4} | R^{B3} | H | R^{B3} |
| L_{A123} | R^{B4} | R^{B3} | R^{B12} | H | L_{A395} | R^{B4} | R^{B3} | H | R^{B4} |
| L_{A124} | R^{B4} | R^{B3} | R^{B18} | H | L_{A396} | R^{B4} | R^{B3} | H | R^{B7} |
| L_{A125} | R^{B4} | R^{B3} | R^{A3} | H | L_{A397} | R^{B4} | R^{B3} | H | R^{B12} |
| L_{A126} | R^{B4} | R^{B3} | R^{A34} | H | L_{A398} | R^{B4} | R^{B3} | H | R^{B18} |
| L_{A127} | R^{B4} | R^{B5} | H | H | L_{A399} | R^{B4} | R^{B3} | H | R^{A3} |
| L_{A128} | R^{B4} | R^{B5} | R^{B1} | H | L_{A400} | R^{B4} | R^{B3} | H | R^{A34} |
| L_{A129} | R^{B4} | R^{B5} | R^{B3} | H | L_{A401} | R^{B4} | R^{B5} | H | R^{B1} |
| L_{A130} | R^{B4} | R^{B5} | R^{B4} | H | L_{A402} | R^{B4} | R^{B5} | H | R^{B3} |
| L_{A131} | R^{B4} | R^{B5} | R^{B7} | H | L_{A403} | R^{B4} | R^{B5} | H | R^{B4} |
| L_{A132} | R^{B4} | R^{B5} | R^{B12} | H | L_{A404} | R^{B4} | R^{B5} | H | R^{B7} |
| L_{A133} | R^{B4} | R^{B5} | R^{B18} | H | L_{A405} | R^{B4} | R^{B5} | H | R^{B12} |
| L_{A134} | R^{B4} | R^{B5} | R^{A3} | H | L_{A406} | R^{B4} | R^{B5} | H | R^{B18} |
| L_{A135} | R^{B4} | R^{B5} | R^{A34} | H | L_{A407} | R^{B4} | R^{B5} | H | R^{A3} |
| L_{A136} | R^{B4} | R^{B6} | H | H | L_{A408} | R^{B4} | R^{B5} | H | R^{A34} |
| L_{A137} | R^{B4} | R^{B6} | R^{B1} | H | L_{A409} | R^{B4} | R^{B6} | H | R^{B1} |
| L_{A138} | R^{B4} | R^{B6} | R^{B3} | H | L_{A410} | R^{B4} | R^{B6} | H | R^{B3} |
| L_{A139} | R^{B4} | R^{B6} | R^{B4} | H | L_{A411} | R^{B4} | R^{B6} | H | R^{B4} |
| L_{A140} | R^{B4} | R^{B6} | R^{B7} | H | L_{A412} | R^{B4} | R^{B6} | H | R^{B7} |
| L_{A141} | R^{B4} | R^{B6} | R^{B12} | H | L_{A413} | R^{B4} | R^{B6} | H | R^{B12} |
| L_{A142} | R^{B4} | R^{B6} | R^{B18} | H | L_{A414} | R^{B4} | R^{B6} | H | R^{B18} |
| L_{A143} | R^{B4} | R^{B6} | R^{A3} | H | L_{A415} | R^{B4} | R^{B6} | H | R^{A3} |
| L_{A144} | R^{B4} | R^{B6} | R^{A34} | H | L_{A416} | R^{B4} | R^{B6} | H | R^{A34} |
| L_{A145} | R^{B4} | R^{B7} | H | H | L_{A417} | R^{B4} | R^{B7} | H | R^{B1} |
| L_{A146} | R^{B4} | R^{B7} | R^{B1} | H | L_{A418} | R^{B4} | R^{B7} | H | R^{B3} |
| L_{A147} | R^{B4} | R^{B7} | R^{B3} | H | L_{A419} | R^{B4} | R^{B7} | H | R^{B4} |
| L_{A148} | R^{B4} | R^{B7} | R^{B4} | H | L_{A420} | R^{B4} | R^{B7} | H | R^{B7} |
| L_{A149} | R^{B4} | R^{B7} | R^{B7} | H | L_{A421} | R^{B4} | R^{B7} | H | R^{B12} |
| L_{A150} | R^{B4} | R^{B7} | R^{B12} | H | L_{A422} | R^{B4} | R^{B7} | H | R^{B18} |
| L_{A151} | R^{B4} | R^{B7} | R^{B18} | H | L_{A423} | R^{B4} | R^{B7} | H | R^{A3} |
| L_{A152} | R^{B4} | R^{B7} | R^{A3} | H | L_{A424} | R^{B4} | R^{B7} | H | R^{A34} |
| L_{A153} | R^{B4} | R^{B7} | R^{A34} | H | L_{A425} | R^{B4} | R^{B12} | H | R^{B1} |
| L_{A154} | R^{B4} | R^{B12} | H | H | L_{A426} | R^{B4} | R^{B12} | H | R^{B3} |
| L_{A155} | R^{B4} | R^{B12} | R^{B1} | H | L_{A427} | R^{B4} | R^{B12} | H | R^{B4} |
| L_{A156} | R^{B4} | R^{B12} | R^{B3} | H | L_{A428} | R^{B4} | R^{B12} | H | R^{B7} |
| L_{A157} | R^{B4} | R^{B12} | R^{B4} | H | L_{A429} | R^{B4} | R^{B12} | H | R^{B12} |
| L_{A158} | R^{B4} | R^{B12} | R^{B7} | H | L_{A430} | R^{B4} | R^{B12} | H | R^{B18} |
| L_{A159} | R^{B4} | R^{B12} | R^{B12} | H | L_{A431} | R^{B4} | R^{B12} | H | R^{A3} |
| L_{A160} | R^{B4} | R^{B12} | R^{B18} | H | L_{A432} | R^{B4} | R^{B12} | H | R^{A34} |
| L_{A161} | R^{B4} | R^{B12} | R^{A3} | H | L_{A433} | R^{B4} | R^{A34} | H | R^{B1} |
| L_{A162} | R^{B4} | R^{B12} | R^{A34} | H | L_{A434} | R^{B4} | R^{A34} | H | R^{B3} |
| L_{A163} | R^{B4} | R^{A34} | H | H | L_{A435} | R^{B4} | R^{A34} | H | R^{B4} |
| L_{A164} | R^{B4} | R^{A34} | R^{B1} | H | L_{A436} | R^{B4} | R^{A34} | H | R^{B7} |
| L_{A165} | R^{B4} | R^{A34} | R^{B3} | H | L_{A437} | R^{B4} | R^{A34} | H | R^{B12} |
| L_{A166} | R^{B4} | R^{A34} | R^{B4} | H | L_{A438} | R^{B4} | R^{A34} | H | R^{B18} |
| L_{A167} | R^{B4} | R^{A34} | R^{B7} | H | L_{A439} | R^{B4} | R^{A34} | H | R^{A3} |
| L_{A168} | R^{B4} | R^{A34} | R^{B12} | H | L_{A440} | R^{B4} | R^{A34} | H | R^{A34} |
| L_{A169} | R^{B4} | R^{A34} | R^{B18} | H | L_{A441} | R^{B7} | R^{B3} | H | R^{B1} |
| L_{A170} | R^{B4} | R^{A34} | R^{A3} | H | L_{A442} | R^{B7} | R^{B3} | H | R^{B3} |
| L_{A171} | R^{B4} | R^{A34} | R^{A34} | H | L_{A443} | R^{B7} | R^{B3} | H | R^{B4} |
| L_{A172} | R^{B7} | R^{B3} | H | H | L_{A444} | R^{B7} | R^{B3} | H | R^{B7} |
| L_{A173} | R^{B7} | R^{B3} | R^{B1} | H | L_{A445} | R^{B7} | R^{B3} | H | R^{B12} |
| L_{A174} | R^{B7} | R^{B3} | R^{B3} | H | L_{A446} | R^{B7} | R^{B3} | H | R^{B18} |
| L_{A175} | R^{B7} | R^{B3} | R^{B4} | H | L_{A447} | R^{B7} | R^{B3} | H | R^{A3} |
| L_{A176} | R^{B7} | R^{B3} | R^{B7} | H | L_{A448} | R^{B7} | R^{B3} | H | R^{A34} |
| L_{A177} | R^{B7} | R^{B3} | R^{B12} | H | L_{A449} | R^{B7} | R^{B4} | H | R^{B1} |
| L_{A178} | R^{B7} | R^{B3} | R^{B18} | H | L_{A450} | R^{B7} | R^{B4} | H | R^{B3} |
| L_{A179} | R^{B7} | R^{B3} | R^{A3} | H | L_{A451} | R^{B7} | R^{B4} | H | R^{B4} |
| L_{A180} | R^{B7} | R^{B3} | R^{A34} | H | L_{A452} | R^{B7} | R^{B4} | H | R^{B7} |
| L_{A181} | R^{B7} | R^{B4} | H | H | L_{A453} | R^{B7} | R^{B4} | H | R^{B12} |
| L_{A182} | R^{B7} | R^{B4} | R^{B1} | H | L_{A454} | R^{B7} | R^{B4} | H | R^{B18} |
| L_{A183} | R^{B7} | R^{B4} | R^{B3} | H | L_{A455} | R^{B7} | R^{B4} | H | R^{A3} |
| L_{A184} | R^{B7} | R^{B4} | R^{B4} | H | L_{A456} | R^{B7} | R^{B4} | H | R^{A34} |
| L_{A185} | R^{B7} | R^{B4} | R^{B7} | H | L_{A457} | R^{B7} | R^{B5} | H | R^{B1} |
| L_{A186} | R^{B7} | R^{B4} | R^{B12} | H | L_{A458} | R^{B7} | R^{B5} | H | R^{B3} |
| L_{A187} | R^{B7} | R^{B4} | R^{B18} | H | L_{A459} | R^{B7} | R^{B5} | H | R^{B4} |
| L_{A188} | R^{B7} | R^{B4} | R^{A3} | H | L_{A460} | R^{B7} | R^{B5} | H | R^{B7} |
| L_{A189} | R^{B7} | R^{B4} | R^{A34} | H | L_{A461} | R^{B7} | R^{B5} | H | R^{B12} |
| L_{A190} | R^{B7} | R^{B5} | H | H | L_{A462} | R^{B7} | R^{B5} | H | R^{B18} |
| L_{A191} | R^{B7} | R^{B5} | R^{B1} | H | L_{A463} | R^{B7} | R^{B5} | H | R^{A3} |
| L_{A192} | R^{B7} | R^{B5} | R^{B3} | H | L_{A464} | R^{B7} | R^{B5} | H | R^{A34} |
| L_{A193} | R^{B7} | R^{B5} | R^{B4} | H | L_{A465} | R^{B7} | R^{B6} | H | R^{B1} |
| L_{A194} | R^{B7} | R^{B5} | R^{B7} | H | L_{A466} | R^{B7} | R^{B6} | H | R^{B3} |
| L_{A195} | R^{B7} | R^{B5} | R^{B12} | H | L_{A467} | R^{B7} | R^{B6} | H | R^{B4} |
| L_{A196} | R^{B7} | R^{B5} | R^{B18} | H | L_{A468} | R^{B7} | R^{B6} | H | R^{B7} |
| L_{A197} | R^{B7} | R^{B5} | R^{A3} | H | L_{A469} | R^{B7} | R^{B6} | H | R^{B12} |
| L_{A198} | R^{B7} | R^{B5} | R^{A34} | H | L_{A470} | R^{B7} | R^{B6} | H | R^{B18} |
| L_{A199} | R^{B7} | R^{B6} | H | H | L_{A471} | R^{B7} | R^{B6} | H | R^{A3} |
| L_{A200} | R^{B7} | R^{B6} | R^{B1} | H | L_{A472} | R^{B7} | R^{B6} | H | R^{A34} |
| L_{A201} | R^{B7} | R^{B6} | R^{B3} | H | L_{A473} | R^{B7} | R^{B12} | H | R^{B1} |
| L_{A202} | R^{B7} | R^{B6} | R^{B4} | H | L_{A474} | R^{B7} | R^{B12} | H | R^{B3} |
| L_{A203} | R^{B7} | R^{B6} | R^{B7} | H | L_{A475} | R^{B7} | R^{B12} | H | R^{B4} |
| L_{A204} | R^{B7} | R^{B6} | R^{B12} | H | L_{A476} | R^{B7} | R^{B12} | H | R^{B7} |
| L_{A205} | R^{B7} | R^{B6} | R^{B18} | H | L_{A477} | R^{B7} | R^{B12} | H | R^{B12} |
| L_{A206} | R^{B7} | R^{B6} | R^{A3} | H | L_{A478} | R^{B7} | R^{B12} | H | R^{B18} |
| L_{A207} | R^{B7} | R^{B6} | R^{A34} | H | L_{A479} | R^{B7} | R^{B12} | H | R^{A3} |
| L_{A208} | R^{B7} | R^{B12} | H | H | L_{A480} | R^{B7} | R^{B12} | H | R^{A34} |
| L_{A209} | R^{B7} | R^{B12} | R^{B1} | H | L_{A481} | R^{B7} | R^{A34} | H | R^{B1} |
| L_{A210} | R^{B7} | R^{B12} | R^{B3} | H | L_{A482} | R^{B7} | R^{A34} | H | R^{B3} |
| L_{A211} | R^{B7} | R^{B12} | R^{B4} | H | L_{A483} | R^{B7} | R^{A34} | H | R^{B4} |
| L_{A212} | R^{B7} | R^{B12} | R^{B7} | H | L_{A484} | R^{B7} | R^{A34} | H | R^{B7} |
| L_{A213} | R^{B7} | R^{B12} | R^{B12} | H | L_{A485} | R^{B7} | R^{A34} | H | R^{B12} |
| L_{A214} | R^{B7} | R^{B12} | R^{B18} | H | L_{A486} | R^{B7} | R^{A34} | H | R^{B18} |
| L_{A215} | R^{B7} | R^{B12} | R^{A3} | H | L_{A487} | R^{B7} | R^{A34} | H | R^{A3} |
| L_{A216} | R^{B7} | R^{B12} | R^{A34} | H | L_{A488} | R^{B7} | R^{A34} | H | R^{A34} |
| L_{A217} | R^{B7} | R^{A34} | H | H | L_{A489} | R^{B43} | R^{B3} | H | R^{B1} |
| L_{A218} | R^{B7} | R^{A34} | R^{B1} | H | L_{A490} | R^{B43} | R^{B3} | H | R^{B3} |
| L_{A219} | R^{B7} | R^{A34} | R^{B3} | H | L_{A491} | R^{B43} | R^{B3} | H | R^{B4} |
| L_{A220} | R^{B7} | R^{A34} | R^{B4} | H | L_{A492} | R^{B43} | R^{B3} | H | R^{B7} |
| L_{A221} | R^{B7} | R^{A34} | R^{B7} | H | L_{A493} | R^{B43} | R^{B3} | H | R^{B12} |
| L_{A222} | R^{B7} | R^{A34} | R^{B12} | H | L_{A494} | R^{B43} | R^{B3} | H | R^{B18} |
| L_{A223} | R^{B7} | R^{A34} | R^{B18} | H | L_{A495} | R^{B43} | R^{B3} | H | R^{A3} |
| L_{A224} | R^{B7} | R^{A34} | R^{A3} | H | L_{A496} | R^{B43} | R^{B3} | H | R^{A34} |
| L_{A225} | R^{B7} | R^{A34} | R^{A34} | H | L_{A497} | R^{B43} | R^{B4} | H | R^{B1} |
| L_{A226} | R^{B43} | R^{B3} | H | H | L_{A498} | R^{B43} | R^{B4} | H | R^{B3} |
| L_{A227} | R^{B43} | R^{B3} | R^{B1} | H | L_{A499} | R^{B43} | R^{B4} | H | R^{B4} |
| L_{A228} | R^{B43} | R^{B3} | R^{B3} | H | L_{A500} | R^{B43} | R^{B4} | H | R^{B7} |
| L_{A229} | R^{B43} | R^{B3} | R^{B4} | H | L_{A501} | R^{B43} | R^{B4} | H | R^{B12} |
| L_{A230} | R^{B43} | R^{B3} | R^{B7} | H | L_{AS02} | R^{B43} | R^{B4} | H | R^{B18} |
| L_{A231} | R^{B43} | R^{B3} | R^{B12} | H | L_{A503} | R^{B43} | R^{B4} | H | R^{A3} |
| L_{A232} | R^{B43} | R^{B3} | R^{B18} | H | L_{A504} | R^{B43} | R^{B4} | H | R^{A34} |
| L_{A233} | R^{B43} | R^{B3} | R^{A3} | H | L_{A505} | R^{B43} | R^{B5} | H | R^{B1} |
| L_{A234} | R^{B43} | R^{B3} | R^{A34} | H | L_{A506} | R^{B43} | R^{B5} | H | R^{B3} |
| L_{A235} | R^{B43} | R^{B4} | H | H | L_{A507} | R^{B43} | R^{B5} | H | R^{B4} |
| L_{A236} | R^{B43} | R^{B4} | R^{B1} | H | L_{A508} | R^{B43} | R^{B5} | H | R^{B7} |
| L_{A237} | R^{B43} | R^{B4} | R^{B3} | H | L_{A509} | R^{B43} | R^{B5} | H | R^{B12} |
| L_{A238} | R^{B43} | R^{B4} | R^{B4} | H | L_{A510} | R^{B43} | R^{B5} | H | R^{B18} |
| L_{A239} | R^{B43} | R^{B4} | R^{B7} | H | L_{A511} | R^{B43} | R^{B5} | H | R^{A3} |
| L_{A240} | R^{B43} | R^{B4} | R^{B12} | H | L_{A512} | R^{B43} | R^{B5} | H | R^{A34} |
| L_{A241} | R^{B43} | R^{B4} | R^{B18} | H | L_{A513} | R^{B43} | R^{B6} | H | R^{B1} |
| L_{A242} | R^{B43} | R^{B4} | R^{A3} | H | L_{A514} | R^{B43} | R^{B6} | H | R^{B3} |
| L_{A243} | R^{B43} | R^{B4} | R^{A34} | H | L_{A515} | R^{B43} | R^{B6} | H | R^{B4} |
| L_{A244} | R^{B43} | R^{B5} | H | H | L_{A516} | R^{B43} | R^{B6} | H | R^{B7} |
| L_{A245} | R^{B43} | R^{B5} | R^{B1} | H | L_{A517} | R^{B43} | R^{B6} | H | R^{B12} |
| L_{A246} | R^{B43} | R^{B5} | R^{B3} | H | L_{A518} | R^{B43} | R^{B6} | H | R^{B18} |
| L_{A247} | R^{B43} | R^{B5} | R^{B4} | H | L_{A519} | R^{B43} | R^{B6} | H | R^{A3} |
| L_{A248} | R^{B43} | R^{B5} | R^{B7} | H | L_{A520} | R^{B43} | R^{B6} | H | R^{A34} |
| L_{A249} | R^{B43} | R^{B5} | R^{B12} | H | L_{A521} | R^{B43} | R^{B7} | H | R^{B1} |
| L_{A250} | R^{B43} | R^{B5} | R^{B18} | H | L_{A522} | R^{B43} | R^{B7} | H | R^{B3} |
| L_{A251} | R^{B43} | R^{B5} | R^{A3} | H | L_{A523} | R^{B43} | R^{B7} | H | R^{B4} |
| L_{A252} | R^{B43} | R^{B5} | R^{A34} | H | L_{A524} | R^{B43} | R^{B7} | H | R^{B7} |
| L_{A253} | R^{B43} | R^{B6} | H | H | L_{A525} | R^{B43} | R^{B7} | H | R^{B12} |
| L_{A254} | R^{B43} | R^{B6} | R^{B1} | H | L_{A526} | R^{B43} | R^{B7} | H | R^{B18} |
| L_{A255} | R^{B43} | R^{B6} | R^{B3} | H | L_{A527} | R^{B43} | R^{B7} | H | R^{A3} |
| L_{A256} | R^{B43} | R^{B6} | R^{B4} | H | L_{A528} | R^{B43} | R^{B7} | H | R^{A34} |
| L_{A257} | R^{B43} | R^{B6} | R^{B7} | H | L_{A529} | R^{B43} | R^{B12} | H | R^{B1} |
| L_{A258} | R^{B43} | R^{B6} | R^{B12} | H | L_{A530} | R^{B43} | R^{B12} | H | R^{B3} |
| L_{A259} | R^{B43} | R^{B6} | R^{B18} | H | L_{A531} | R^{B43} | R^{B12} | H | R^{B4} |
| L_{A260} | R^{B43} | R^{B6} | R^{A3} | H | L_{A532} | R^{B43} | R^{B12} | H | R^{B7} |
| L_{A261} | R^{B43} | R^{B6} | R^{A34} | H | L_{A533} | R^{B43} | R^{B12} | H | R^{B12} |
| L_{A262} | R^{B43} | R^{B7} | H | H | L_{A534} | R^{B43} | R^{B12} | H | R^{B18} |
| L_{A263} | R^{B43} | R^{B7} | R^{B1} | H | L_{A535} | R^{B43} | R^{B12} | H | R^{A3} |
| L_{A264} | R^{B43} | R^{B7} | R^{B3} | H | L_{A536} | R^{B43} | R^{B12} | H | R^{A34} |
| L_{A265} | R^{B43} | R^{B7} | R^{B4} | H | L_{A537} | R^{B43} | R^{A34} | H | R^{B1} |
| L_{A266} | R^{B43} | R^{B7} | R^{B7} | H | L_{A538} | R^{B43} | R^{A34} | H | R^{B3} |
| L_{A267} | R^{B43} | R^{B7} | R^{B12} | H | L_{A539} | R^{B43} | R^{A34} | H | R^{B4} |
| L_{A268} | R^{B43} | R^{B7} | R^{B18} | H | L_{A540} | R^{B43} | R^{A34} | H | R^{B7} |
| L_{A269} | R^{B43} | R^{B7} | R^{A3} | H | L_{A541} | R^{B43} | R^{A34} | H | R^{B12} |
| L_{A270} | R^{B43} | R^{B7} | R^{A34} | H | L_{A542} | R^{B43} | R^{A34} | H | R^{B18} |
| L_{A271} | R^{B43} | R^{B12} | H | H | L_{A543} | R^{B43} | R^{A34} | H | R^{A3} |
| L_{A272} | R^{B43} | R^{B12} | R^{B1} | H | L_{A544} | R^{B43} | R^{A34} | H | R^{A34} |

L_{A545} through L_{A1088}, that are based on a structure of Formula Ib, 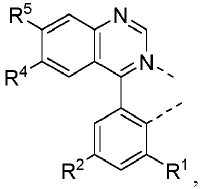 in each of which R¹, R², R⁴, and R⁵ are defined as follows:

| | **R¹** | **R²** | **R⁴** | **R⁵** | | **R¹** | **R³** | **R⁴** | **R⁵** |
|---|---|---|---|---|---|---|---|---|---|
| L_{A545} | R^{B1} | R^{B3} | H | H | L_{A817} | R^{B43} | R^{B12} | R^{B3} | H |
| L_{A546} | R^{B1} | R^{B3} | R^{B1} | H | L_{A818} | R^{B43} | R^{B12} | R^{B4} | H |
| L_{A547} | R^{B1} | R^{B3} | R^{B3} | H | L_{A819} | R^{B43} | R^{B12} | R^{B7} | H |
| L_{A548} | R^{B1} | R^{B3} | R^{B4} | H | L_{A820} | R^{B43} | R^{B12} | R^{B12} | H |
| L_{A549} | R^{B1} | R^{B3} | R^{B7} | H | L_{A821} | R^{B43} | R^{B12} | R^{B18} | H |
| L_{A550} | R^{B1} | R^{B3} | R^{B12} | H | L_{A822} | R^{B43} | R^{B12} | R^{A3} | H |
| L_{A551} | R^{B1} | R^{B3} | R^{B18} | H | L_{A823} | R^{B43} | R^{B12} | R^{A34} | H |
| L_{A552} | R^{B1} | R^{B3} | R^{A3} | H | L_{A824} | R^{B43} | R^{A34} | H | H |
| L_{A553} | R^{B1} | R^{B3} | R^{A34} | H | L_{A825} | R^{B43} | R^{A34} | R^{B1} | H |
| L_{A554} | R^{B1} | R^{B4} | H | H | L_{A826} | R^{B43} | R^{A34} | R^{B3} | H |
| L_{A555} | R^{B1} | R^{B4} | R^{B1} | H | L_{A827} | R^{B43} | R^{A34} | R^{B4} | H |
| L_{A556} | R^{B1} | R^{B4} | R^{B3} | H | L_{A828} | R^{B43} | R^{A34} | R^{B7} | H |
| L_{A557} | R^{B1} | R^{B4} | R^{B4} | H | L_{A829} | R^{B43} | R^{A34} | R^{B12} | H |
| L_{A558} | R^{B1} | R^{B4} | R^{B7} | H | L_{A830} | R^{B43} | R^{A34} | R^{B18} | H |
| L_{A559} | R^{B1} | R^{B4} | R^{B12} | H | L_{A831} | R^{B43} | R^{A34} | R^{A3} | H |
| L_{AS60} | R^{B1} | R^{B4} | R^{B18} | H | L_{A832} | R^{B43} | R^{A34} | R^{A34} | H |
| L_{A561} | R^{B1} | R^{B4} | R^{A3} | H | L_{A833} | R^{B1} | R^{B3} | H | R^{B1} |
| L_{AS62} | R^{B1} | R^{B4} | R^{A34} | H | L_{A834} | R^{B1} | R^{B3} | H | R^{B3} |
| L_{AS63} | R^{B1} | R^{B5} | H | H | L_{A835} | R^{B1} | R^{B3} | H | R^{B4} |
| L_{AS64} | R^{B1} | R^{B5} | R^{B1} | H | L_{A836} | R^{B1} | R^{B3} | H | R^{B7} |
| L_{A565} | R^{B1} | R^{B5} | R^{B3} | H | L_{A837} | R^{B1} | R^{B3} | H | R^{B12} |
| L_{A566} | R^{B1} | R^{B5} | R^{B4} | H | L_{A838} | R^{B1} | R^{B3} | H | R^{B18} |
| L_{A567} | R^{B1} | R^{B5} | R^{B7} | H | L_{A839} | R^{B1} | R^{B3} | H | R^{A3} |
| L_{AS68} | R^{B1} | R^{B5} | R^{B12} | H | L_{A840} | R^{B1} | R^{B3} | H | R^{A34} |
| L_{A569} | R^{B1} | R^{B5} | R^{B18} | H | L_{A841} | R^{B1} | R^{B4} | H | R^{B1} |
| L_{A570} | R^{B1} | R^{B5} | R^{A3} | H | L_{A842} | R^{B1} | R^{B4} | H | R^{B3} |
| L_{A571} | R^{B1} | R^{B5} | R^{A34} | H | L_{A843} | R^{B1} | R^{B4} | H | R^{B4} |
| L_{A572} | R^{B1} | R^{B6} | H | H | L_{A844} | R^{B1} | R^{B4} | H | R^{B7} |
| L_{A573} | R^{B1} | R^{B6} | R^{B1} | H | L_{A845} | R^{B1} | R^{B4} | H | R^{B12} |
| L_{A574} | R^{B1} | R^{B6} | R^{B3} | H | L_{A846} | R^{B1} | R^{B4} | H | R^{B18} |
| L_{A575} | R^{B1} | R^{B6} | R^{B4} | H | L_{A847} | R^{B1} | R^{B4} | H | R^{A3} |
| L_{A576} | R^{B1} | R^{B6} | R^{B7} | H | L_{A848} | R^{B1} | R^{B4} | H | R^{A34} |
| L_{A577} | R^{B1} | R^{B6} | R^{B12} | H | L_{A849} | R^{B1} | R^{B5} | H | R^{B1} |
| L_{A578} | R^{B1} | R^{B6} | R^{B18} | H | L_{A850} | R^{B1} | R^{B5} | H | R^{B3} |
| L_{A579} | R^{B1} | R^{B6} | R^{A3} | H | L_{A851} | R^{B1} | R^{B5} | H | R^{B4} |
| L_{A580} | R^{B1} | R^{B6} | R^{A34} | H | L_{A852} | R^{B1} | R^{B5} | H | R^{B7} |
| L_{A581} | R^{B1} | R^{B7} | H | H | L_{A853} | R^{B1} | R^{B5} | H | R^{B12} |
| L_{A582} | R^{B1} | R^{B7} | R^{B1} | H | L_{A854} | R^{B1} | R^{B5} | H | R^{B18} |
| L_{A583} | R^{B1} | R^{B7} | R^{B3} | H | L_{A855} | R^{B1} | R^{B5} | H | R^{A3} |
| L_{A584} | R^{B1} | R^{B7} | R^{B4} | H | L_{A856} | R^{B1} | R^{B5} | H | R^{A34} |
| L_{A585} | R^{B1} | R^{B7} | R^{B7} | H | L_{A857} | R^{B1} | R^{B6} | H | R^{B1} |
| L_{A586} | R^{B1} | R^{B7} | R^{B12} | H | L_{A858} | R^{B1} | R^{B6} | H | R^{B3} |
| L_{A587} | R^{B1} | R^{B7} | R^{B18} | H | L_{A859} | R^{B1} | R^{B6} | H | R^{B4} |
| L_{A588} | R^{B1} | R^{B7} | R^{A3} | H | L_{A860} | R^{B1} | R^{B6} | H | R^{B7} |
| L_{A589} | R^{B1} | R^{B7} | R^{A34} | H | L_{A861} | R^{B1} | R^{B6} | H | R^{B12} |
| L_{A590} | R^{B1} | R^{B12} | H | H | L_{A862} | R^{B1} | R^{B6} | H | R^{B18} |
| L_{A591} | R^{B1} | R^{B12} | R^{B1} | H | L_{A863} | R^{B1} | R^{B6} | H | R^{A3} |
| L_{A592} | R^{B1} | R^{B12} | R^{B3} | H | L_{A864} | R^{B1} | R^{B6} | H | R^{A34} |
| L_{A593} | R^{B1} | R^{B12} | R^{B4} | H | L_{A865} | R^{B1} | R^{B7} | H | R^{B1} |
| L_{A594} | R^{B1} | R^{B12} | R^{B7} | H | L_{A866} | R^{B1} | R^{B7} | H | R^{B3} |
| L_{A595} | R^{B1} | R^{B12} | R^{B12} | H | L_{A867} | R^{B1} | R^{B7} | H | R^{B4} |
| L_{A596} | R^{B1} | R^{B12} | R^{B18} | H | L_{A868} | R^{B1} | R^{B7} | H | R^{B7} |
| L_{A597} | R^{B1} | R^{B12} | R^{A3} | H | L_{A869} | R^{B1} | R^{B7} | H | R^{B12} |
| L_{A598} | R^{B1} | R^{B12} | R^{A34} | H | L_{A870} | R^{B1} | R^{B7} | H | R^{B18} |
| L_{A599} | R^{B1} | R^{A34} | H | H | L_{A871} | R^{B1} | R^{B7} | H | R^{A3} |
| L_{A600} | R^{B1} | R^{A34} | R^{B1} | H | L_{A872} | R^{B1} | R^{B7} | H | R^{A34} |
| L_{A601} | R^{B1} | R^{A34} | R^{B3} | H | L_{A873} | R^{B1} | R^{B12} | H | R^{B1} |
| L_{A602} | R^{B1} | R^{A34} | R^{B4} | H | L_{A874} | R^{B1} | R^{B12} | H | R^{B3} |
| L_{A603} | R^{B1} | R^{A34} | R^{B7} | H | L_{A875} | R^{B1} | R^{B12} | H | R^{B4} |
| L_{A604} | R^{B1} | R^{A34} | R^{B12} | H | L_{A876} | R^{B1} | R^{B12} | H | R^{B7} |
| L_{A605} | R^{B1} | R^{A34} | R^{B18} | H | L_{A877} | R^{B1} | R^{B12} | H | R^{B12} |
| L_{A606} | R^{B1} | R^{A34} | R^{A3} | H | L_{A878} | R^{B1} | R^{B12} | H | R^{B18} |
| L_{A607} | R^{B1} | R^{A34} | R^{A34} | H | L_{A879} | R^{B1} | R^{B12} | H | R^{A3} |
| L_{A608} | R^{B3} | R^{B4} | H | H | L_{A880} | R^{B1} | R^{B12} | H | R^{A34} |
| L_{A609} | R^{B3} | R^{B4} | R^{B1} | H | L_{A881} | R^{B1} | R^{A34} | H | R^{B1} |
| L_{A610} | R^{B3} | R^{B4} | R^{B3} | H | L_{A882} | R^{B1} | R^{A34} | H | R^{B3} |
| L_{A611} | R^{B3} | R^{B4} | R^{B4} | H | L_{A883} | R^{B1} | R^{A34} | H | R^{B4} |
| L_{A612} | R^{B3} | R^{B4} | R^{B7} | H | L_{A884} | R^{B1} | R^{A34} | H | R^{B7} |
| L_{A613} | R^{B3} | R^{B4} | R^{B12} | H | L_{A885} | R^{B1} | R^{A34} | H | R^{B12} |
| L_{A614} | R^{B3} | R^{B4} | R^{B18} | H | L_{A886} | R^{B1} | R^{A34} | H | R^{B18} |
| L_{A615} | R^{B3} | R^{B4} | R^{A3} | H | L_{A887} | R^{B1} | R^{A34} | H | R^{A3} |
| L_{A616} | R^{B3} | R^{B4} | R^{A34} | H | L_{A888} | R^{B1} | R^{A34} | H | R^{A34} |
| L_{A617} | R^{B3} | R^{B5} | H | H | L_{A889} | R^{B3} | R^{B4} | H | R^{B1} |
| L_{A618} | R^{B3} | R^{B5} | R^{B1} | H | L_{A890} | R^{B3} | R^{B4} | H | R^{B3} |
| L_{A619} | R^{B3} | R^{B5} | R^{B3} | H | L_{A891} | R^{B3} | R^{B4} | H | R^{B4} |
| L_{A620} | R^{B3} | R^{B5} | R^{B4} | H | L_{A892} | R^{B3} | R^{B4} | H | R^{B7} |
| L_{A621} | R^{B3} | R^{B5} | R^{B7} | H | L_{A893} | R^{B3} | R^{B4} | H | R^{B12} |
| L_{A622} | R^{B3} | R^{B5} | R^{B12} | H | L_{A894} | R^{B3} | R^{B4} | H | R^{B18} |
| L_{A623} | R^{B3} | R^{B5} | R^{B18} | H | L_{A895} | R^{B3} | R^{B4} | H | R^{A3} |
| L_{A624} | R^{B3} | R^{B5} | R^{A3} | H | L_{A896} | R^{B3} | R^{B4} | H | R^{A34} |
| L_{A625} | R^{B3} | R^{B5} | R^{A34} | H | L_{A897} | R^{B3} | R^{B5} | H | R^{B1} |
| L_{A626} | R^{B3} | R^{B6} | H | H | L_{A898} | R^{B3} | R^{B5} | H | R^{B3} |
| L_{A627} | R^{B3} | R^{B6} | R^{B1} | H | L_{A899} | R^{B3} | R^{B5} | H | R^{B4} |
| L_{A628} | R^{B3} | R^{B6} | R^{B3} | H | L_{A900} | R^{B3} | R^{B5} | H | R^{B7} |
| L_{A629} | R^{B3} | R^{B6} | R^{B4} | H | L_{A901} | R^{B3} | R^{B5} | H | R^{B12} |
| L_{A630} | R^{B3} | R^{B6} | R^{B7} | H | L_{A902} | R^{B3} | R^{B5} | H | R^{B18} |
| L_{A631} | R^{B3} | R^{B6} | R^{B12} | H | L_{A903} | R^{B3} | R^{B5} | H | R^{A3} |
| L_{A632} | R^{B3} | R^{B6} | R^{B18} | H | L_{A904} | R^{B3} | R^{B5} | H | R^{A34} |
| L_{A633} | R^{B3} | R^{B6} | R^{A3} | H | L_{A905} | R^{B3} | R^{B6} | H | R^{B1} |
| L_{A634} | R^{B3} | R^{B6} | R^{A34} | H | L_{A906} | R^{B3} | R^{B6} | H | R^{B3} |
| L_{A635} | R^{B3} | R^{B7} | H | H | L_{A907} | R^{B3} | R^{B6} | H | R^{B4} |
| L_{A636} | R^{B3} | R^{B7} | R^{B1} | H | L_{A908} | R^{B3} | R^{B6} | H | R^{B7} |
| L_{A637} | R^{B3} | R^{B7} | R^{B3} | H | L_{A909} | R^{B3} | R^{B6} | H | R^{B12} |
| L_{A638} | R^{B3} | R^{B7} | R^{B4} | H | L_{A910} | R^{B3} | R^{B6} | H | R^{B18} |
| L_{A639} | R^{B3} | R^{B7} | R^{B7} | H | L_{A911} | R^{B3} | R^{B6} | H | R^{A3} |
| L_{A640} | R^{B3} | R^{B7} | R^{B12} | H | L_{A912} | R^{B3} | R^{B6} | H | R^{A34} |
| L_{A641} | R^{B3} | R^{B7} | R^{B18} | H | L_{A913} | R^{B3} | R^{B7} | H | R^{B1} |
| L_{A642} | R^{B3} | R^{B7} | R^{A3} | H | L_{A914} | R^{B3} | R^{B7} | H | R^{B3} |
| L_{A643} | R^{B3} | R^{B7} | R^{A34} | H | L_{A915} | R^{B3} | R^{B7} | H | R^{B4} |
| L_{A644} | R^{B3} | R^{B12} | H | H | L_{A916} | R^{B3} | R^{B7} | H | R^{B7} |
| L_{A645} | R^{B3} | R^{B12} | R^{B1} | H | L_{A917} | R^{B3} | R^{B7} | H | R^{B12} |
| L_{A646} | R^{B3} | R^{B12} | R^{B3} | H | L_{A918} | R^{B3} | R^{B7} | H | R^{B18} |
| L_{A647} | R^{B3} | R^{B12} | R^{B4} | H | L_{A919} | R^{B3} | R^{B7} | H | R^{A3} |
| L_{A648} | R^{B3} | R^{B12} | R^{B7} | H | L_{A920} | R^{B3} | R^{B7} | H | R^{A34} |
| L_{A649} | R^{B3} | R^{B12} | R^{B12} | H | L_{A921} | R^{B3} | R^{B12} | H | R^{B1} |
| L_{A650} | R^{B3} | R^{B12} | R^{B18} | H | L_{A922} | R^{B3} | R^{B12} | H | R^{B3} |
| L_{A651} | R^{B3} | R^{B12} | R^{A3} | H | L_{A923} | R^{B3} | R^{B12} | H | R^{B4} |
| L_{A652} | R^{B3} | R^{B12} | R^{A34} | H | L_{A924} | R^{B3} | R^{B12} | H | R^{B7} |
| L_{A653} | R^{B3} | R^{A34} | H | H | L_{A925} | R^{B3} | R^{B12} | H | R^{B12} |
| L_{A654} | R^{B3} | R^{A34} | R^{B1} | H | L_{A926} | R^{B3} | R^{B12} | H | R^{B18} |
| L_{A655} | R^{B3} | R^{A34} | R^{B3} | H | L_{A927} | R^{B3} | R^{B12} | H | R^{A3} |
| L_{A656} | R^{B3} | R^{A34} | R^{B4} | H | L_{A928} | R^{B3} | R^{B12} | H | R^{A34} |
| L_{A657} | R^{B3} | R^{A34} | R^{B7} | H | L_{A929} | R^{B3} | R^{A34} | H | R^{B1} |
| L_{A658} | R^{B3} | R^{A34} | R^{B12} | H | L_{A930} | R^{B3} | R^{A34} | H | R^{B3} |
| L_{A659} | R^{B3} | R^{A34} | R^{B18} | H | L_{A931} | R^{B3} | R^{A34} | H | R^{B4} |
| L_{A660} | R^{B3} | R^{A34} | R^{A3} | H | L_{A932} | R^{B3} | R^{A34} | H | R^{B7} |
| L_{A661} | R^{B3} | R^{A34} | R^{A34} | H | L_{A933} | R^{B3} | R^{A34} | H | R^{B12} |
| L_{A662} | R^{B4} | R^{B3} | H | H | L_{A934} | R^{B3} | R^{A34} | H | R^{B18} |
| L_{A663} | R^{B4} | R^{B3} | R^{B1} | H | L_{A935} | R^{B3} | R^{A34} | H | R^{A3} |
| L_{A664} | R^{B4} | R^{B3} | R^{B3} | H | L_{A936} | R^{B3} | R^{A34} | H | R^{A34} |
| L_{A665} | R^{B4} | R^{B3} | R^{B4} | H | L_{A937} | R^{B4} | R^{B3} | H | R^{B1} |
| L_{A666} | R^{B4} | R^{B3} | R^{B7} | H | L_{A938} | R^{B4} | R^{B3} | H | R^{B3} |
| L_{A667} | R^{B4} | R^{B3} | R^{B12} | H | L_{A939} | R^{B4} | R^{B3} | H | R^{B4} |
| L_{A668} | R^{B4} | R^{B3} | R^{B18} | H | L_{A940} | R^{B4} | R^{B3} | H | R^{B7} |
| L_{A669} | R^{B4} | R^{B3} | R^{A3} | H | L_{A941} | R^{B4} | R^{B3} | H | R^{B12} |
| L_{A670} | R^{B4} | R^{B3} | R^{A34} | H | L_{A942} | R^{B4} | R^{B3} | H | R^{B18} |
| L_{A671} | R^{B4} | R^{B5} | H | H | L_{A943} | R^{B4} | R^{B3} | H | R^{A3} |
| L_{A672} | R^{B4} | R^{B5} | R^{B1} | H | L_{A944} | R^{B4} | R^{B3} | H | R^{A34} |
| L_{A673} | R^{B4} | R^{B5} | R^{B3} | H | L_{A945} | R^{B4} | R^{B5} | H | R^{B1} |
| L_{A674} | R^{B4} | R^{B5} | R^{B4} | H | L_{A946} | R^{B4} | R^{B5} | H | R^{B3} |
| L_{A675} | R^{B4} | R^{B5} | R^{B7} | H | L_{A947} | R^{B4} | R^{B5} | H | R^{B4} |
| L_{A676} | R^{B4} | R^{B5} | R^{B12} | H | L_{A948} | R^{B4} | R^{B5} | H | R^{B7} |
| L_{A677} | R^{B4} | R^{B5} | R^{B18} | H | L_{A949} | R^{B4} | R^{B5} | H | R^{B12} |
| L_{A678} | R^{B4} | R^{B5} | R^{A3} | H | L_{A950} | R^{B4} | R^{B5} | H | R^{B18} |
| L_{A679} | R^{B4} | R^{B5} | R^{A34} | H | L_{A951} | R^{B4} | R^{B5} | H | R^{A3} |
| L_{A680} | R^{B4} | R^{B6} | H | H | L_{A952} | R^{B4} | R^{B5} | H | R^{A34} |
| L_{A681} | R^{B4} | R^{B6} | R^{B1} | H | L_{A953} | R^{B4} | R^{B6} | H | R^{B1} |
| L_{A682} | R^{B4} | R^{B6} | R^{B3} | H | L_{A954} | R^{B4} | R^{B6} | H | R^{B3} |
| L_{A683} | R^{B4} | R^{B6} | R^{B4} | H | L_{A955} | R^{B4} | R^{B6} | H | R^{B4} |
| L_{A684} | R^{B4} | R^{B6} | R^{B7} | H | L_{A956} | R^{B4} | R^{B6} | H | R^{B7} |
| L_{A685} | R^{B4} | R^{B6} | R^{B12} | H | L_{A957} | R^{B4} | R^{B6} | H | R^{B12} |
| L_{A686} | R^{B4} | R^{B6} | R^{B18} | H | L_{A958} | R^{B4} | R^{B6} | H | R^{B18} |
| L_{A687} | R^{B4} | R^{B6} | R^{A3} | H | L_{A959} | R^{B4} | R^{B6} | H | R^{A3} |
| L_{A688} | R^{B4} | R^{B6} | R^{A34} | H | L_{A960} | R^{B4} | R^{B6} | H | R^{A34} |
| L_{A689} | R^{B4} | R^{B7} | H | H | L_{A961} | R^{B4} | R^{B7} | H | R^{B1} |
| L_{A690} | R^{B4} | R^{B7} | R^{B1} | H | L_{A962} | R^{B4} | R^{B7} | H | R^{B3} |
| L_{A691} | R^{B4} | R^{B7} | R^{B3} | H | L_{A963} | R^{B4} | R^{B7} | H | R^{B4} |
| L_{A692} | R^{B4} | R^{B7} | R^{B4} | H | L_{A964} | R^{B4} | R^{B7} | H | R^{B7} |
| L_{A693} | R^{B4} | R^{B7} | R^{B7} | H | L_{A965} | R^{B4} | R^{B7} | H | R^{B12} |
| L_{A694} | R^{B4} | R^{B7} | R^{B12} | H | L_{A966} | R^{B4} | R^{B7} | H | R^{B18} |
| L_{A695} | R^{B4} | R^{B7} | R^{B18} | H | L_{A967} | R^{B4} | R^{B7} | H | R^{A3} |
| L_{A696} | R^{B4} | R^{B7} | R^{A3} | H | L_{A968} | R^{B4} | R^{B7} | H | R^{A34} |
| L_{A697} | R^{B4} | R^{B7} | R^{A34} | H | L_{A969} | R^{B4} | R^{B12} | H | R^{B1} |
| L_{A698} | R^{B4} | R^{B12} | H | H | L_{A970} | R^{B4} | R^{B12} | H | R^{B3} |
| L_{A699} | R^{B4} | R^{B12} | R^{B1} | H | L_{A971} | R^{B4} | R^{B12} | H | R^{B4} |
| L_{A700} | R^{B4} | R^{B12} | R^{B3} | H | L_{A972} | R^{B4} | R^{B12} | H | R^{B7} |
| L_{A701} | R^{B4} | R^{B12} | R^{B4} | H | L_{A973} | R^{B4} | R^{B12} | H | R^{B12} |
| L_{A702} | R^{B4} | R^{B12} | R^{B7} | H | L_{A974} | R^{B4} | R^{B12} | H | R^{B18} |
| L_{A703} | R^{B4} | R^{B12} | R^{B12} | H | L_{A975} | R^{B4} | R^{B12} | H | R^{A3} |
| L_{A704} | R^{B4} | R^{B12} | R^{B18} | H | L_{A976} | R^{B4} | R^{B12} | H | R^{A34} |
| L_{A705} | R^{B4} | R^{B12} | R^{A3} | H | L_{A977} | R^{B4} | R^{A34} | H | R^{B1} |
| L_{A706} | R^{B4} | R^{B12} | R^{A34} | H | L_{A978} | R^{B4} | R^{A34} | H | R^{B3} |
| L_{A707} | R^{B4} | R^{A34} | H | H | L_{A979} | R^{B4} | R^{A34} | H | R^{B4} |
| L_{A708} | R^{B4} | R^{A34} | R^{B1} | H | L_{A980} | R^{B4} | R^{A34} | H | R^{B7} |
| L_{A709} | R^{B4} | R^{A34} | R^{B3} | H | L_{A981} | R^{B4} | R^{A34} | H | R^{B12} |
| L_{A710} | R^{B4} | R^{A34} | R^{B4} | H | L_{A982} | R^{B4} | R^{A34} | H | R^{B18} |
| L_{A711} | R^{B4} | R^{A34} | R^{B7} | H | L_{A983} | R^{B4} | R^{A34} | H | R^{A3} |
| L_{A712} | R^{B4} | R^{A34} | R^{B12} | H | L_{A984} | R^{B4} | R^{A34} | H | R^{A34} |
| L_{A713} | R^{B4} | R^{A34} | R^{B18} | H | L_{A985} | R^{B7} | R^{B3} | H | R^{B1} |
| L_{A714} | R^{B4} | R^{A34} | R^{A3} | H | L_{A986} | R^{B7} | R^{B3} | H | R^{B3} |
| L_{A715} | R^{B4} | R^{A34} | R^{A34} | H | L_{A987} | R^{B7} | R^{B3} | H | R^{B4} |
| L_{A716} | R^{B7} | R^{B3} | H | H | L_{A988} | R^{B7} | R^{B3} | H | R^{B7} |
| L_{A717} | R^{B7} | R^{B3} | R^{B1} | H | L_{A989} | R^{B7} | R^{B3} | H | R^{B12} |
| L_{A718} | R^{B7} | R^{B3} | R^{B3} | H | L_{A990} | R^{B7} | R^{B3} | H | R^{B18} |
| L_{A719} | R^{B7} | R^{B3} | R^{B4} | H | L_{A991} | R^{B7} | R^{B3} | H | R^{A3} |
| L_{A720} | R^{B7} | R^{B3} | R^{B7} | H | L_{A992} | R^{B7} | R^{B3} | H | R^{A34} |
| L_{A721} | R^{B7} | R^{B3} | R^{B12} | H | L_{A993} | R^{B7} | R^{B4} | H | R^{B1} |
| L_{A722} | R^{B7} | R^{B3} | R^{B18} | H | L_{A994} | R^{B7} | R^{B4} | H | R^{B3} |
| L_{A723} | R^{B7} | R^{B3} | R^{A3} | H | L_{A995} | R^{B7} | R^{B4} | H | R^{B4} |
| L_{A724} | R^{B7} | R^{B3} | R^{A34} | H | L_{A996} | R^{B7} | R^{B4} | H | R^{B7} |

| | **R⁵** | **R²** | **R⁴** | **R⁵** | | **R¹** | **R³** | **R⁴** | **R⁵** |
|---|---|---|---|---|---|---|---|---|---|
| L_{A725} | R^{B7} | R^{B4} | H | H | L_{A997} | R^{B7} | R^{B4} | H | R^{B12} |
| L_{A726} | R^{B7} | R^{B4} | R^{B1} | H | L_{A998} | R^{B7} | R^{B4} | H | R^{B18} |
| L_{A727} | R^{B7} | R^{B4} | R^{B3} | H | L_{A999} | R^{B7} | R^{B4} | H | R^{A3} |
| L_{A728} | R^{B7} | R^{B4} | R^{B4} | H | L_{A1000} | R^{B7} | R^{B4} | H | R^{A34} |
| L_{A729} | R^{B7} | R^{B4} | R^{B7} | H | L_{A1001} | R^{B7} | R^{B5} | H | R^{B1} |
| L_{A730} | R^{B7} | R^{B4} | R^{B12} | H | L_{A1002} | R^{B7} | R^{B5} | H | R^{B3} |
| L_{A731} | R^{B7} | R^{B4} | R^{B18} | H | L_{A1003} | R^{B7} | R^{B5} | H | R^{B4} |
| L_{A732} | R^{B7} | R^{B4} | R^{A3} | H | L_{A1004} | R^{B7} | R^{B5} | H | R^{B7} |
| L_{A733} | R^{B7} | R^{B4} | R^{A34} | H | L_{A1005} | R^{B7} | R^{B5} | H | R^{B12} |
| L_{A734} | R^{B7} | R^{B5} | H | H | L_{A1006} | R^{B7} | R^{B5} | H | R^{B18} |
| L_{A735} | R^{B7} | R^{B5} | R^{B1} | H | L_{A1007} | R^{B7} | R^{B5} | H | R^{A3} |
| L_{A736} | R^{B7} | R^{B5} | R^{B3} | H | L_{A1008} | R^{B7} | R^{B5} | H | R^{A34} |
| L_{A737} | R^{B7} | R^{B5} | R^{B4} | H | L_{A1009} | R^{B7} | R^{B6} | H | R^{B1} |
| L_{A738} | R^{B7} | R^{B5} | R^{B7} | H | L_{A1010} | R^{B7} | R^{B6} | H | R^{B3} |
| L_{A739} | R^{B7} | R^{B5} | R^{B12} | H | L_{A1011} | R^{B7} | R^{B6} | H | R^{B4} |
| L_{A740} | R^{B7} | R^{B5} | R^{B18} | H | L_{A1012} | R^{B7} | R^{B6} | H | R^{B7} |
| L_{A741} | R^{B7} | R^{B5} | R^{A3} | H | L_{A1013} | R^{B7} | R^{B6} | H | R^{B12} |
| L_{A742} | R^{B7} | R^{B5} | R^{A34} | H | L_{A1014} | R^{B7} | R^{B6} | H | R^{B18} |
| L_{A743} | R^{B7} | R^{B6} | H | H | L_{A1015} | R^{B7} | R^{B6} | H | R^{A3} |
| L_{A744} | R^{B7} | R^{B6} | R^{B1} | H | L_{A1016} | R^{B7} | R^{B6} | H | R^{A34} |
| L_{A745} | R^{B7} | R^{B6} | R^{B3} | H | L_{A1017} | R^{B7} | R^{B12} | H | R^{B1} |
| L_{A746} | R^{B7} | R^{B6} | R^{B4} | H | L_{A1018} | R^{B7} | R^{B12} | H | R^{B3} |
| L_{A747} | R^{B7} | R^{B6} | R^{B7} | H | L_{A1019} | R^{B7} | R^{B12} | H | R^{B4} |
| L_{A748} | R^{B7} | R^{B6} | R^{B12} | H | L_{A1020} | R^{B7} | R^{B12} | H | R^{B7} |
| L_{A749} | R^{B7} | R^{B6} | R^{B18} | H | L_{A1021} | R^{B7} | R^{B12} | H | R^{B12} |
| L_{A750} | R^{B7} | R^{B6} | R^{A3} | H | L_{A1022} | R^{B7} | R^{B12} | H | R^{B18} |
| L_{A751} | R^{B7} | R^{B6} | R^{A34} | H | L_{A1023} | R^{B7} | R^{B12} | H | R^{A3} |
| L_{A752} | R^{B7} | R^{B12} | H | H | L_{A1024} | R^{B7} | R^{B12} | H | R^{A34} |
| L_{A753} | R^{B7} | R^{B12} | R^{B1} | H | L_{A1025} | R^{B7} | R^{A34} | H | R^{B1} |
| L_{A754} | R^{B7} | R^{B12} | R^{B3} | H | L_{A1026} | R^{B7} | R^{A34} | H | R^{B3} |
| L_{A755} | R^{B7} | R^{B12} | R^{B4} | H | L_{A1027} | R^{B7} | R^{A34} | H | R^{B4} |

| | **R¹** | **R²** | **R⁴** | **R⁵** | | **R¹** | **R³** | **R⁴** | **R⁵** |
|---|---|---|---|---|---|---|---|---|---|
| L_{A756} | R^{B7} | R^{B12} | R^{B7} | H | L_{A1028} | R^{B7} | R^{A34} | H | R^{B1} |
| L_{A757} | R^{B7} | R^{B12} | R^{B12} | H | L_{A1029} | R^{B7} | R^{A34} | H | R^{B12} |
| L_{A758} | R^{B7} | R^{B12} | R^{B18} | H | L_{A1030} | R^{B7} | R^{A34} | H | R^{B18} |
| L_{A759} | R^{B7} | R^{B12} | R^{A3} | H | L_{A1031} | R^{B7} | R^{A34} | H | R^{A3} |
| L_{A760} | R^{B7} | R^{B12} | R^{A34} | H | L_{A1032} | R^{B7} | R^{A34} | H | R^{A34} |
| L_{A761} | R^{B7} | R^{A34} | H | H | L_{A1033} | R^{B43} | R^{B3} | H | R^{B1} |
| L_{A762} | R^{B7} | R^{A34} | R^{B1} | H | L_{A1034} | R^{B43} | R^{B3} | H | R^{B3} |
| L_{A763} | R^{B7} | R^{A34} | R^{B3} | H | L_{A1035} | R^{B43} | R^{B3} | H | R^{B4} |
| L_{A764} | R^{B7} | R^{A34} | R^{B4} | H | L_{A1036} | R^{B43} | R^{B3} | H | R^{B7} |
| L_{A765} | R^{B7} | R^{A34} | R^{B7} | H | L_{A1037} | R^{B43} | R^{B3} | H | R^{B12} |
| L_{A766} | R^{B7} | R^{A34} | R^{B12} | H | L_{A1038} | R^{B43} | R^{B3} | H | R^{B18} |
| L_{A767} | R^{B7} | R^{A34} | R^{B18} | H | L_{A1039} | R^{B43} | R^{B3} | H | R^{A3} |
| L_{A768} | R^{B7} | R^{A34} | R^{A3} | H | L_{A1040} | R^{B43} | R^{B3} | H | R^{A34} |
| L_{A769} | R^{B7} | R^{A34} | R^{A34} | H | L_{A1041} | R^{B43} | R^{B4} | H | R^{B1} |
| L_{A770} | R^{B43} | R^{B3} | H | H | L_{A1042} | R^{B43} | R^{B4} | H | R^{B3} |
| L_{A771} | R^{B43} | R^{B3} | R^{B1} | H | L_{A1043} | R^{B43} | R^{B4} | H | R^{B4} |
| L_{A772} | R^{B43} | R^{B3} | R^{B3} | H | L_{A1044} | R^{B43} | R^{B4} | H | R^{B7} |
| L_{A773} | R^{B43} | R^{B3} | R^{B4} | H | L_{A1045} | R^{B43} | R^{B4} | H | R^{B12} |
| L_{A774} | R^{B43} | R^{B3} | R^{B7} | H | L_{A1046} | R^{B43} | R^{B4} | H | R^{B18} |
| L_{A775} | R^{B43} | R^{B3} | R^{B12} | H | L_{A1047} | R^{B43} | R^{B4} | H | R^{A3} |
| L_{A776} | R^{B43} | R^{B3} | R^{B18} | H | L_{A1048} | R^{B43} | R^{B4} | H | R^{A34} |
| L_{A777} | R^{B43} | R^{B3} | R^{A3} | H | L_{A1049} | R^{B43} | R^{B5} | H | R^{B1} |
| L_{A778} | R^{B43} | R^{B3} | R^{A34} | H | L_{A1050} | R^{B43} | R^{B5} | H | R^{B3} |
| L_{A779} | R^{B43} | R^{B4} | H | H | L_{A1051} | R^{B43} | R^{B5} | H | R^{B4} |
| L_{A780} | R^{B43} | R^{B4} | R^{B1} | H | L_{A1052} | R^{B43} | R^{B5} | H | R^{B7} |
| L_{A781} | R^{B43} | R^{B4} | R^{B3} | H | L_{A1053} | R^{B43} | R^{B5} | H | R^{B12} |
| L_{A782} | R^{B43} | R^{B4} | R^{B4} | H | L_{A1054} | R^{B43} | R^{B5} | H | R^{B18} |
| L_{A783} | R^{B43} | R^{B4} | R^{B7} | H | L_{A1055} | R^{B43} | R^{B5} | H | R^{A3} |
| L_{A784} | R^{B43} | R^{B4} | R^{B12} | H | L_{A1056} | R^{B43} | R^{B5} | H | R^{A34} |
| L_{A785} | R^{B43} | R^{B4} | R^{B18} | H | L_{A1057} | R^{B43} | R^{B6} | H | R^{B1} |
| L_{A786} | R^{B43} | R^{B4} | R^{A3} | H | L_{A1058} | R^{B43} | R^{B6} | H | R^{B3} |
| L_{A787} | R^{B43} | R^{B4} | R^{A34} | H | L_{A1059} | R^{B43} | R^{B6} | H | R^{B4} |
| L_{A788} | R^{B43} | R^{B5} | H | H | L_{A1060} | R^{B43} | R^{B6} | H | R^{B7} |
| L_{A789} | R^{B43} | R^{B5} | R^{B1} | H | L_{A1061} | R^{B43} | R^{B6} | H | R^{B12} |
| L_{A790} | R^{B43} | R^{B5} | R^{B3} | H | L_{A1062} | R^{B43} | R^{B6} | H | R^{B18} |
| L_{A791} | R^{B43} | R^{B5} | R^{B4} | H | L_{A1063} | R^{B43} | R^{B6} | H | R^{A3} |
| L_{A792} | R^{B43} | R^{B5} | R^{B7} | H | L_{A1064} | R^{B43} | R^{B6} | H | R^{A34} |
| L_{A793} | R^{B43} | R^{B5} | R^{B12} | H | L_{A1065} | R^{B43} | R^{B7} | H | R^{B1} |
| L_{A794} | R^{B43} | R^{B5} | R^{B18} | H | L_{A1066} | R^{B43} | R^{B7} | H | R^{B3} |
| L_{A795} | R^{B43} | R^{B5} | R^{A3} | H | L_{A1067} | R^{B43} | R^{B7} | H | R^{B4} |
| L_{A796} | R^{B43} | R^{B5} | R^{A34} | H | L_{A1068} | R^{B43} | R^{B7} | H | R^{B7} |
| L_{A797} | R^{B43} | R^{B6} | H | H | L_{A1069} | R^{B43} | R^{B7} | H | R^{B12} |
| L_{A798} | R^{B43} | R^{B6} | R^{B1} | H | L_{A1070} | R^{B43} | R^{B7} | H | R^{B18} |
| L_{A799} | R^{B43} | R^{B6} | R^{B3} | H | L_{A1071} | R^{B43} | R^{B7} | H | R^{A3} |
| L_{A800} | R^{B43} | R^{B6} | R^{B4} | H | L_{A1072} | R^{B43} | R^{B7} | H | R^{A34} |
| L_{A801} | R^{B43} | R^{B6} | R^{B7} | H | L_{A1073} | R^{B43} | R^{B12} | H | R^{B1} |
| L_{A802} | R^{B43} | R^{B6} | R^{B12} | H | L_{A1074} | R^{B43} | R^{B12} | H | R^{B3} |
| L_{A803} | R^{B43} | R^{B6} | R^{B18} | H | L_{A1075} | R^{B43} | R^{B12} | H | R^{B4} |
| L_{A804} | R^{B43} | R^{B6} | R^{A3} | H | L_{A1076} | R^{B43} | R^{B12} | H | R^{B7} |
| L_{A805} | R^{B43} | R^{B6} | R^{A34} | H | L_{A1077} | R^{B43} | R^{B12} | H | R^{B12} |
| L_{A806} | R^{B43} | R^{B7} | H | H | L_{A1078} | R^{B43} | R^{B12} | H | R^{B18} |
| L_{A807} | R^{B43} | R^{B7} | R^{B1} | H | L_{A1079} | R^{B43} | R^{B12} | H | R^{A3} |
| L_{A808} | R^{B43} | R^{B7} | R^{B3} | H | L_{A1080} | R^{B43} | R^{B12} | H | R^{A34} |
| L_{A809} | R^{B43} | R^{B7} | R^{B4} | H | L_{A1081} | R^{B43} | R^{A34} | H | R^{B1} |
| L_{A810} | R^{B43} | R^{B7} | R^{B7} | H | L_{A1082} | R^{B43} | R^{A34} | H | R^{B3} |
| L_{A811} | R^{B43} | R^{B7} | R^{B12} | H | L_{A1083} | R^{B43} | R^{A34} | H | R^{B4} |
| L_{A812} | R^{B43} | R^{B7} | R^{B18} | H | L_{A1084} | R^{B43} | R^{A34} | H | R^{B7} |
| L_{A813} | R^{B43} | R^{B7} | R^{A3} | H | L_{A1085} | R^{B43} | R^{A34} | H | R^{B12} |
| L_{A814} | R^{B43} | R^{B7} | R^{A34} | H | L_{A1086} | R^{B43} | R^{A34} | H | R^{B18} |
| L_{A815} | R^{B43} | R^{B12} | H | H | L_{A1087} | R^{B43} | R^{A34} | H | R^{A3} |
| L_{A816} | R^{B43} | R^{B12} | R^{B1} | H | L_{A1088} | R^{B43} | R^{A34} | H | R^{A34} |

L_{A1089} through L_{A1632}, that are based on a structure of Formula Ic, 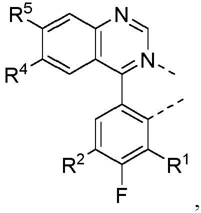 in each of which R¹, R², R⁴, and R⁵ are defined as follows:

| | **R**¹ | **R**² | **R**⁴ | **R**⁵ | | **R**¹ | **R**³ | **R**⁴ | **R⁵** |
|---|---|---|---|---|---|---|---|---|---|
| L_{A1089} | R^{B1} | R^{B3} | H | H | L_{A1361} | R^{B43} | R^{B12} | R^{B3} | H |
| L_{A1090} | R^{B1} | R^{B3} | R^{B1} | H | L_{A1362} | R^{B43} | R^{B12} | R^{B4} | H |
| L_{A1091} | R^{B1} | R^{B3} | R^{B3} | H | L_{A1363} | R^{B43} | R^{B12} | R^{B7} | H |
| L_{A1092} | R^{B1} | R^{B3} | R^{B4} | H | L_{A1364} | R^{B43} | R^{B12} | R^{B12} | H |
| L_{A1093} | R^{B1} | R^{B3} | R^{B7} | H | L_{A1365} | R^{B43} | R^{B12} | R^{B18} | H |
| L_{A1094} | R^{B1} | R^{B3} | R^{B12} | H | L_{A1366} | R^{B43} | R^{B12} | R^{A3} | H |
| L_{A1095} | R^{B1} | R^{B3} | R^{B18} | H | L_{A1367} | R^{B43} | R^{B12} | R^{A34} | H |
| L_{A1096} | R^{B1} | R^{B3} | R^{A3} | H | L_{A1368} | R^{B43} | R^{A34} | H | H |
| L_{A1097} | R^{B1} | R^{B3} | R^{A34} | H | L_{A1369} | R^{B43} | R^{A34} | R^{B1} | H |
| L_{A1098} | R^{B1} | R^{B4} | H | H | L_{A1370} | R^{B43} | R^{A34} | R^{B3} | H |
| L_{A1099} | R^{B1} | R^{B4} | R^{B1} | H | L_{A1371} | R^{B43} | R^{A34} | R^{B4} | H |
| L_{A1100} | R^{B1} | R^{B4} | R^{B3} | H | L_{A1372} | R^{B43} | R^{A34} | R^{B7} | H |
| L_{A1101} | R^{B1} | R^{B4} | R^{B4} | H | L_{A1373} | R^{B43} | R^{A34} | R^{B12} | H |
| L_{A1102} | R^{B1} | R^{B4} | R^{B7} | H | L_{A1374} | R^{B43} | R^{A34} | R^{B18} | H |
| L_{A1103} | R^{B1} | R^{B4} | R^{B12} | H | L_{A1375} | R^{B43} | R^{A34} | R^{A3} | H |
| L_{A1104} | R^{B1} | R^{B4} | R^{B18} | H | L_{A1376} | R^{B43} | R^{A34} | R^{A34} | H |
| L_{A1105} | R^{B1} | R^{B4} | R^{A3} | H | L_{A1377} | R^{B1} | R^{B3} | H | R^{B1} |
| L_{A1106} | R^{B1} | R^{B4} | R^{A34} | H | L_{A1378} | R^{B1} | R^{B3} | H | R^{B3} |
| L_{A1107} | R^{B1} | R^{B5} | H | H | L_{A1379} | R^{B1} | R^{B3} | H | R^{B4} |
| L_{A1108} | R^{B1} | R^{B5} | R^{B1} | H | L_{A1380} | R^{B1} | R^{B3} | H | R^{B7} |
| L_{A1109} | R^{B1} | R^{B5} | R^{B3} | H | L_{A1381} | R^{B1} | R^{B3} | H | R^{B12} |
| L_{A1110} | R^{B1} | R^{B5} | R^{B4} | H | L_{A1382} | R^{B1} | R^{B3} | H | R^{B18} |
| L_{A1111} | R^{B1} | R^{B5} | R^{B7} | H | L_{A1383} | R^{B1} | R^{B3} | H | R^{A3} |
| L_{A1112} | R^{B1} | R^{B5} | R^{B12} | H | L_{A1384} | R^{B1} | R^{B3} | H | R^{A34} |
| L_{A1113} | R^{B1} | R^{B5} | R^{B18} | H | L_{A1385} | R^{B1} | R^{B4} | H | R^{B1} |
| L_{A1114} | R^{B1} | R^{B5} | R^{A3} | H | L_{A1386} | R^{B1} | R^{B4} | H | R^{B3} |
| L_{A1115} | R^{B1} | R^{B5} | R^{A34} | H | L_{A1387} | R^{B1} | R^{B4} | H | R^{B4} |
| L_{A1116} | R^{B1} | R^{B6} | H | H | L_{A1388} | R^{B1} | R^{B4} | H | R^{B7} |
| L_{A1117} | R^{B1} | R^{B6} | R^{B1} | H | L_{A1389} | R^{B1} | R^{B4} | H | R^{B12} |
| L_{A1118} | R^{B1} | R^{B6} | R^{B3} | H | L_{A1390} | R^{B1} | R^{B4} | H | R^{B18} |
| L_{A1119} | R^{B1} | R^{B6} | R^{B4} | H | L_{A1391} | R^{B1} | R^{B4} | H | R^{A3} |
| L_{A1120} | R^{B1} | R^{B6} | R^{B7} | H | L_{A1392} | R^{B1} | R^{B4} | H | R^{A34} |
| L_{A1121} | R^{B1} | R^{B6} | R^{B12} | H | L_{A1393} | R^{B1} | R^{B5} | H | R^{B1} |
| L_{A1122} | R^{B1} | R^{B6} | R^{B18} | H | L_{A1394} | R^{B1} | R^{B5} | H | R^{B3} |
| L_{A1123} | R^{B1} | R^{B6} | R^{A3} | H | L_{A1395} | R^{B1} | R^{B5} | H | R^{B4} |
| L_{A1124} | R^{B1} | R^{B6} | R^{A34} | H | L_{A1396} | R^{B1} | R^{B5} | H | R^{B7} |
| L_{A1125} | R^{B1} | R^{B7} | H | H | L_{A1397} | R^{B1} | R^{B5} | H | R^{B12} |
| L_{A1126} | R^{B1} | R^{B7} | R^{B1} | H | L_{A1398} | R^{B1} | R^{B5} | H | R^{B18} |
| L_{A1127} | R^{B1} | R^{B7} | R^{B3} | H | L_{A1399} | R^{B1} | R^{B5} | H | R^{A3} |
| L_{A1128} | R^{B1} | R^{B7} | R^{B4} | H | L_{A1400} | R^{B1} | R^{B5} | H | R^{A34} |
| L_{A1129} | R^{B1} | R^{B7} | R^{B7} | H | L_{A1401} | R^{B1} | R^{B6} | H | R^{B1} |
| L_{A1130} | R^{B1} | R^{B7} | R^{B12} | H | L_{A1402} | R^{B1} | R^{B6} | H | R^{B3} |
| L_{A1131} | R^{B1} | R^{B7} | R^{B18} | H | L_{A1403} | R^{B1} | R^{B6} | H | R^{B4} |
| L_{A1132} | R^{B1} | R^{B7} | R^{A3} | H | L_{A1404} | R^{B1} | R^{B6} | H | R^{B7} |
| L_{A1133} | R^{B1} | R^{B7} | R^{A34} | H | L_{A1405} | R^{B1} | R^{B6} | H | R^{B12} |
| L_{A1134} | R^{B1} | R^{B12} | H | H | L_{A1406} | R^{B1} | R^{B6} | H | R^{B18} |
| L_{A1135} | R^{B1} | R^{B12} | R^{B1} | H | L_{A1407} | R^{B1} | R^{B6} | H | R^{A3} |
| L_{A1136} | R^{B1} | R^{B12} | R^{B3} | H | L_{A1408} | R^{B1} | R^{B6} | H | R^{A34} |
| L_{A1137} | R^{B1} | R^{B12} | R^{B4} | H | L_{A1409} | R^{B1} | R^{B7} | H | R^{B1} |
| L_{A1138} | R^{B1} | R^{B12} | R^{B7} | H | L_{A1410} | R^{B1} | R^{B7} | H | R^{B3} |
| L_{A1139} | R^{B1} | R^{B12} | R^{B12} | H | L_{A1411} | R^{B1} | R^{B7} | H | R^{B4} |
| L_{A1140} | R^{B1} | R^{B12} | R^{B18} | H | L_{A1412} | R^{B1} | R^{B7} | H | R^{B7} |
| L_{A1141} | R^{B1} | R^{B12} | R^{A3} | H | L_{A1413} | R^{B1} | R^{B7} | H | R^{B12} |
| L_{A1142} | R^{B1} | R^{B12} | R^{A34} | H | L_{A1414} | R^{B1} | R^{B7} | H | R^{B18} |
| L_{A1143} | R^{B1} | R^{A34} | H | H | L_{A1415} | R^{B1} | R^{B7} | H | R^{A3} |
| L_{A1144} | R^{B1} | R^{A34} | R^{B1} | H | L_{A1416} | R^{B1} | R^{B7} | H | R^{A34} |
| L_{A1145} | R^{B1} | R^{A34} | R^{B3} | H | L_{A1417} | R^{B1} | R^{B12} | H | R^{B1} |
| L_{A1146} | R^{B1} | R^{A34} | R^{B4} | H | L_{A1418} | R^{B1} | R^{B12} | H | R^{B3} |
| L_{A1147} | R^{B1} | R^{A34} | R^{B7} | H | L_{A1419} | R^{B1} | R^{B12} | H | R^{B4} |
| L_{A1148} | R^{B1} | R^{A34} | R^{B12} | H | L_{A1420} | R^{B1} | R^{B12} | H | R^{B7} |
| L_{A1149} | R^{B1} | R^{A34} | R^{B18} | H | L_{A1421} | R^{B1} | R^{B12} | H | R^{B12} |
| L_{A1150} | R^{B1} | R^{A34} | R^{A3} | H | L_{A1422} | R^{B1} | R^{B12} | H | R^{B18} |
| L_{A1151} | R^{B1} | R^{A34} | R^{A34} | H | L_{A1423} | R^{B1} | R^{B12} | H | R^{A3} |
| L_{A1152} | R^{B3} | R^{B4} | H | H | L_{A1424} | R^{B1} | R^{B12} | H | R^{A34} |
| L_{A1153} | R^{B3} | R^{B4} | R^{B1} | H | L_{A1425} | R^{B1} | R^{A34} | H | R^{B1} |
| L_{A1154} | R^{B3} | R^{B4} | R^{B3} | H | L_{A1426} | R^{B1} | R^{A34} | H | R^{B3} |
| L_{A1155} | R^{B3} | R^{B4} | R^{B4} | H | L_{A1427} | R^{B1} | R^{A34} | H | R^{B4} |
| L_{A1156} | R^{B3} | R^{B4} | R^{B7} | H | L_{A1428} | R^{B1} | R^{A34} | H | R^{B7} |
| L_{A1157} | R^{B3} | R^{B4} | R^{B12} | H | L_{A1429} | R^{B1} | R^{A34} | H | R^{B12} |
| L_{A1158} | R^{B3} | R^{B4} | R^{B18} | H | L_{A1430} | R^{B1} | R^{A34} | H | R^{B18} |
| L_{A1159} | R^{B3} | R^{B4} | R^{A3} | H | L_{A1431} | R^{B1} | R^{A34} | H | R^{A3} |
| L_{A1160} | R^{B3} | R^{B4} | R^{A34} | H | L_{A1432} | R^{B1} | R^{A34} | H | R^{A34} |
| L_{A1161} | R^{B3} | R^{B5} | H | H | L_{A1433} | R^{B3} | R^{B4} | H | R^{B1} |
| L_{A1162} | R^{B3} | R^{B5} | R^{B1} | H | L_{A1434} | R^{B3} | R^{B4} | H | R^{B3} |
| L_{A1163} | R^{B3} | R^{B5} | R^{B3} | H | L_{A1435} | R^{B3} | R^{B4} | H | R^{B4} |
| L_{A1164} | R^{B3} | R^{B5} | R^{B4} | H | L_{A1436} | R^{B3} | R^{B4} | H | R^{B7} |
| L_{A1165} | R^{B3} | R^{B5} | R^{B7} | H | L_{A1437} | R^{B3} | R^{B4} | H | R^{B12} |
| L_{A1166} | R^{B3} | R^{B5} | R^{B12} | H | L_{A1438} | R^{B3} | R^{B4} | H | R^{B18} |
| L_{A1167} | R^{B3} | R^{B5} | R^{B18} | H | L_{A1439} | R^{B3} | R^{B4} | H | R^{A3} |
| L_{A1168} | R^{B3} | R^{B5} | R^{A3} | H | L_{A1440} | R^{B3} | R^{B4} | H | R^{A34} |
| L_{A1169} | R^{B3} | R^{B5} | R^{A34} | H | L_{A1441} | R^{B3} | R^{B5} | H | R^{B1} |
| L_{A1170} | R^{B3} | R^{B6} | H | H | L_{A1442} | R^{B3} | R^{B5} | H | R^{B3} |
| L_{A1171} | R^{B3} | R^{B6} | R^{B1} | H | L_{A1443} | R^{B3} | R^{B5} | H | R^{B4} |
| L_{A1172} | R^{B3} | R^{B6} | R^{B3} | H | L_{A1444} | R^{B3} | R^{B5} | H | R^{B7} |
| L_{A1173} | R^{B3} | R^{B6} | R^{B4} | H | L_{A1445} | R^{B3} | R^{B5} | H | R^{B12} |
| L_{A1174} | R^{B3} | R^{B6} | R^{B7} | H | L_{A1446} | R^{B3} | R^{B5} | H | R^{B18} |
| L_{A1175} | R^{B3} | R^{B6} | R^{B12} | H | L_{A1447} | R^{B3} | R^{B5} | H | R^{A3} |
| L_{A1176} | R^{B3} | R^{B6} | R^{B18} | H | L_{A1448} | R^{B3} | R^{B5} | H | R^{A34} |
| L_{A1177} | R^{B3} | R^{B6} | R^{A3} | H | L_{A1449} | R^{B3} | R^{B6} | H | R^{B1} |
| L_{A1178} | R^{B3} | R^{B6} | R^{A34} | H | L_{A1450} | R^{B3} | R^{B6} | H | R^{B3} |
| L_{A1179} | R^{B3} | R^{B7} | H | H | L_{A1451} | R^{B3} | R^{B6} | H | R^{B4} |
| L_{A1180} | R^{B3} | R^{B7} | R^{B1} | H | L_{A1452} | R^{B3} | R^{B6} | H | R^{B7} |
| L_{A1181} | R^{B3} | R^{B7} | R^{B3} | H | L_{A1453} | R^{B3} | R^{B6} | H | R^{B12} |
| L_{A1182} | R^{B3} | R^{B7} | R^{B4} | H | L_{A1454} | R^{B3} | R^{B6} | H | R^{B18} |
| L_{A1183} | R^{B3} | R^{B7} | R^{B7} | H | L_{A1455} | R^{B3} | R^{B6} | H | R^{A3} |
| L_{A1184} | R^{B3} | R^{B7} | R^{B12} | H | L_{A1456} | R^{B3} | R^{B6} | H | R^{A34} |
| L_{A1185} | R^{B3} | R^{B7} | R^{B18} | H | L_{A1457} | R^{B3} | R^{B7} | H | R^{B1} |
| L_{A1186} | R^{B3} | R^{B7} | R^{A3} | H | L_{A1458} | R^{B3} | R^{B7} | H | R^{B3} |
| L_{A1187} | R^{B3} | R^{B7} | R^{A34} | H | L_{A1459} | R^{B3} | R^{B7} | H | R^{B4} |
| L_{A1188} | R^{B3} | R^{B12} | H | H | L_{A1460} | R^{B3} | R^{B7} | H | R^{B7} |
| L_{A1189} | R^{B3} | R^{B12} | R^{B1} | H | L_{A1461} | R^{B3} | R^{B7} | H | R^{B12} |
| L_{A1190} | R^{B3} | R^{B12} | R^{B3} | H | L_{A1462} | R^{B3} | R^{B7} | H | R^{B18} |
| L_{A1191} | R^{B3} | R^{B12} | R^{B4} | H | L_{A1463} | R^{B3} | R^{B7} | H | R^{A3} |
| L_{A1192} | R^{B3} | R^{B12} | R^{B7} | H | L_{A1464} | R^{B3} | R^{B7} | H | R^{A34} |
| L_{A1193} | R^{B3} | R^{B12} | R^{B12} | H | L_{A1465} | R^{B3} | R^{B12} | H | R^{B1} |
| L_{A1194} | R^{B3} | R^{B12} | R^{B18} | H | L_{A1466} | R^{B3} | R^{B12} | H | R^{B3} |
| L_{A1195} | R^{B3} | R^{B12} | R^{A3} | H | L_{A1467} | R^{B3} | R^{B12} | H | R^{B4} |
| L_{A1196} | R^{B3} | R^{B12} | R^{A34} | H | L_{A1468} | R^{B3} | R^{B12} | H | R^{B7} |
| L_{A1197} | R^{B3} | R^{A34} | H | H | L_{A1469} | R^{B3} | R^{B12} | H | R^{B12} |
| L_{A1198} | R^{B3} | R^{A34} | R^{B1} | H | L_{A1470} | R^{B3} | R^{B12} | H | R^{B18} |
| L_{A1199} | R^{B3} | R^{A34} | R^{B3} | H | L_{A1471} | R^{B3} | R^{B12} | H | R^{A3} |
| L_{A1200} | R^{B3} | R^{A34} | R^{B4} | H | L_{A1472} | R^{B3} | R^{B12} | H | R^{A34} |
| L_{A1201} | R^{B3} | R^{A34} | R^{B7} | H | L_{A1473} | R^{B3} | R^{A34} | H | R^{B1} |
| L_{A1202} | R^{B3} | R^{A34} | R^{B12} | H | L_{A1474} | R^{B3} | R^{A34} | H | R^{B3} |
| L_{A1203} | R^{B3} | R^{A34} | R^{B18} | H | L_{A1475} | R^{B3} | R^{A34} | H | R^{B4} |
| L_{A1204} | R^{B3} | R^{A34} | R^{A3} | H | L_{A1476} | R^{B3} | R^{A34} | H | R^{B7} |
| L_{A1205} | R^{B3} | R^{A34} | R^{A34} | H | L_{A1477} | R^{B3} | R^{A34} | H | R^{B12} |
| L_{A1206} | R^{B4} | R^{B3} | H | H | L_{A1478} | R^{B3} | R^{A34} | H | R^{B18} |
| L_{A1207} | R^{B4} | R^{B3} | R^{B1} | H | L_{A1479} | R^{B3} | R^{A34} | H | R^{A3} |
| L_{A1208} | R^{B4} | R^{B3} | R^{B3} | H | L_{A1480} | R^{B3} | R^{A34} | H | R^{A34} |
| L_{A1209} | R^{B4} | R^{B3} | R^{B4} | H | L_{A1481} | R^{B4} | R^{B3} | H | R^{B1} |
| L_{A1210} | R^{B4} | R^{B3} | R^{B7} | H | L_{A1482} | R^{B4} | R^{B3} | H | R^{B3} |
| L_{A1211} | R^{B4} | R^{B3} | R^{B12} | H | L_{A1483} | R^{B4} | R^{B3} | H | R^{B4} |
| L_{A1212} | R^{B4} | R^{B3} | R^{B18} | H | L_{A1484} | R^{B4} | R^{B3} | H | R^{B7} |
| L_{A1213} | R^{B4} | R^{B3} | R^{A3} | H | L_{A1485} | R^{B4} | R^{B3} | H | R^{B12} |
| L_{A1214} | R^{B4} | R^{B3} | R^{A34} | H | L_{A1486} | R^{B4} | R^{B3} | H | R^{B18} |
| L_{A1215} | R^{B4} | R^{B5} | H | H | L_{A1487} | R^{B4} | R^{B3} | H | R^{A3} |
| L_{A1216} | R^{B4} | R^{B5} | R^{B1} | H | L_{A1488} | R^{B4} | R^{B3} | H | R^{A34} |
| L_{A1217} | R^{B4} | R^{B5} | R^{B3} | H | L_{A1489} | R^{B4} | R^{B5} | H | R^{B1} |
| L_{A1218} | R^{B4} | R^{B5} | R^{B4} | H | L_{A1490} | R^{B4} | R^{B5} | H | R^{B3} |
| L_{A1219} | R^{B4} | R^{B5} | R^{B7} | H | L_{A1491} | R^{B4} | R^{B5} | H | R^{B4} |
| L_{A1220} | R^{B4} | R^{B5} | R^{B12} | H | L_{A1492} | R^{B4} | R^{B5} | H | R^{B7} |
| L_{A1221} | R^{B4} | R^{B5} | R^{B18} | H | L_{A1493} | R^{B4} | R^{B5} | H | R^{B12} |
| L_{A1222} | R^{B4} | R^{B5} | R^{A3} | H | L_{A1494} | R^{B4} | R^{B5} | H | R^{B18} |
| L_{A1223} | R^{B4} | R^{B5} | R^{A34} | H | L_{A1495} | R^{B4} | R^{B5} | H | R^{A3} |
| L_{A1224} | R^{B4} | R^{B6} | H | H | L_{A1496} | R^{B4} | R^{B5} | H | R^{A34} |
| L_{A1225} | R^{B4} | R^{B6} | R^{B1} | H | L_{A1497} | R^{B4} | R^{B6} | H | R^{B1} |
| L_{A1226} | R^{B4} | R^{B6} | R^{B3} | H | L_{A1498} | R^{B4} | R^{B6} | H | R^{B3} |
| L_{A1227} | R^{B4} | R^{B6} | R^{B4} | H | L_{A1499} | R^{B4} | R^{B6} | H | R^{B4} |
| L_{A1228} | R^{B4} | R^{B6} | R^{B7} | H | L_{A1500} | R^{B4} | R^{B6} | H | R^{B7} |
| L_{A1229} | R^{B4} | R^{B6} | R^{B12} | H | L_{A1501} | R^{B4} | R^{B6} | H | R^{B12} |
| L_{A1230} | R^{B4} | R^{B6} | R^{B18} | H | L_{A1502} | R^{B4} | R^{B6} | H | R^{B18} |
| L_{A1231} | R^{B4} | R^{B6} | R^{A3} | H | L_{A1503} | R^{B4} | R^{B6} | H | R^{A3} |
| L_{A1232} | R^{B4} | R^{B6} | R^{A34} | H | L_{A1504} | R^{B4} | R^{B6} | H | R^{A34} |
| L_{A1233} | R^{B4} | R^{B7} | H | H | L_{A1505} | R^{B4} | R^{B7} | H | R^{B1} |
| L_{A1234} | R^{B4} | R^{B7} | R^{B1} | H | L_{A1506} | R^{B4} | R^{B7} | H | R^{B3} |
| L_{A1235} | R^{B4} | R^{B7} | R^{B3} | H | L_{A1507} | R^{B4} | R^{B7} | H | R^{B4} |
| L_{A1236} | R^{B4} | R^{B7} | R^{B4} | H | L_{A1508} | R^{B4} | R^{B7} | H | R^{B7} |
| L_{A1237} | R^{B4} | R^{B7} | R^{B7} | H | L_{A1509} | R^{B4} | R^{B7} | H | R^{B12} |
| L_{A1238} | R^{B4} | R^{B7} | R^{B12} | H | L_{A1510} | R^{B4} | R^{B7} | H | R^{B18} |
| L_{A1239} | R^{B4} | R^{B7} | R^{B18} | H | L_{A1511} | R^{B4} | R^{B7} | H | R^{A3} |
| L_{A1240} | R^{B4} | R^{B7} | R^{A3} | H | L_{A1512} | R^{B4} | R^{B7} | H | R^{A34} |
| L_{A1241} | R^{B4} | R^{B7} | R^{A34} | H | L_{A1513} | R^{B4} | R^{B12} | H | R^{B1} |
| L_{A1242} | R^{B4} | R^{B12} | H | H | L_{A1514} | R^{B4} | R^{B12} | H | R^{B3} |
| L_{A1243} | R^{B4} | R^{B12} | R^{B1} | H | L_{A1515} | R^{B4} | R^{B12} | H | R^{B4} |
| L_{A1244} | R^{B4} | R^{B12} | R^{B3} | H | L_{A1516} | R^{B4} | R^{B12} | H | R^{B7} |
| L_{A1245} | R^{B4} | R^{B12} | R^{B4} | H | L_{A1517} | R^{B4} | R^{B12} | H | R^{B12} |
| L_{A1246} | R^{B4} | R^{B12} | R^{B7} | H | L_{A1518} | R^{B4} | R^{B12} | H | R^{B18} |
| L_{A1247} | R^{B4} | R^{B12} | R^{B12} | H | L_{A1519} | R^{B4} | R^{B12} | H | R^{A3} |
| L_{A1248} | R^{B4} | R^{B12} | R^{B18} | H | L_{A1520} | R^{B4} | R^{B12} | H | R^{A34} |
| L_{A1249} | R^{B4} | R^{B12} | R^{A3} | H | L_{A1521} | R^{B4} | R^{A34} | H | R^{B1} |
| L_{A1250} | R^{B4} | R^{B12} | R^{A34} | H | L_{A1522} | R^{B4} | R^{A34} | H | R^{B3} |
| L_{A1251} | R^{B4} | R^{A34} | H | H | L_{A1523} | R^{B4} | R^{A34} | H | R^{B4} |
| L_{A1252} | R^{B4} | R^{A34} | R^{B1} | H | L_{A1524} | R^{B4} | R^{A34} | H | R^{B7} |
| L_{A1253} | R^{B4} | R^{A34} | R^{B3} | H | L_{A1525} | R^{B4} | R^{A34} | H | R^{B12} |
| L_{A1254} | R^{B4} | R^{A34} | R^{B4} | H | L_{A1526} | R^{B4} | R^{A34} | H | R^{B18} |
| L_{A1255} | R^{B4} | R^{A34} | R^{B7} | H | L_{A1527} | R^{B4} | R^{A34} | H | R^{A3} |
| L_{A1256} | R^{B4} | R^{A34} | R^{B12} | H | L_{A1528} | R^{B4} | R^{A34} | H | R^{A34} |
| L_{A1257} | R^{B4} | R^{A34} | R^{B18} | H | L_{A1529} | R^{B7} | R^{B3} | H | RB1 |
| L_{A1258} | R^{B4} | R^{A34} | R^{A3} | H | L_{A1530} | R^{B7} | R^{B3} | H | R^{B3} |
| L_{A1259} | R^{B4} | R^{A34} | R^{A34} | H | L_{A1531} | R^{B7} | R^{B3} | H | R^{B4} |
| L_{A1260} | R^{B7} | R^{B3} | H | H | L_{A1532} | R^{B7} | R^{B3} | H | RB7 |
| L_{A1261} | R^{B7} | R^{B3} | R^{B1} | H | L_{A1533} | R^{B7} | R^{B3} | H | R^{B12} |
| L_{A1262} | R^{B7} | RB3 | R^{B3} | H | L_{A1534} | R^{B7} | RB3 | H | R^{B18} |
| L_{A1263} | R^{B7} | R^{B3} | R^{B4} | H | L_{A1535} | R^{B7} | R^{B3} | H | R^{A3} |
| L_{A1264} | R^{B7} | R^{B3} | R^{B7} | H | L_{A1536} | R^{B7} | R^{B3} | H | R^{A34} |
| L_{A1265} | R^{B7} | R^{B3} | R^{B12} | H | L_{A1537} | R^{B7} | R^{B4} | H | R^{B1} |
| L_{A1266} | R^{B7} | R^{B3} | R^{B18} | H | L_{A1538} | R^{B7} | R^{B4} | H | R^{B3} |
| L_{A1267} | R^{B7} | R^{B3} | R^{A3} | H | L_{A1539} | R^{B7} | R^{B4} | H | R^{B4} |
| L_{A1268} | R^{B7} | R^{B3} | R^{A34} | H | L_{A1540} | R^{B7} | R^{B4} | H | R^{B7} |
| L_{A1269} | R^{B7} | R^{B4} | H | H | L_{A1541} | R^{B7} | R^{B4} | H | R^{B12} |
| L_{A1270} | R^{B7} | R^{B4} | R^{B1} | H | L_{A1542} | R^{B7} | R^{B4} | H | R^{B18} |
| L_{A1271} | R^{B7} | R^{B4} | R^{B3} | H | L_{A1543} | R^{B7} | R^{B4} | H | R^{A3} |
| L_{A1272} | R^{B7} | R^{B4} | R^{B4} | H | L_{A1544} | R^{B7} | R^{B4} | H | R^{A34} |
| L_{A1273} | R^{B7} | R^{B4} | R^{B7} | H | L_{A1545} | R^{B7} | R^{B5} | H | R^{B1} |
| L_{A1274} | R^{B7} | R^{B4} | R^{B12} | H | L_{A1546} | R^{B7} | R^{B5} | H | R^{B3} |
| L_{A1275} | R^{B7} | R^{B4} | R^{B18} | H | L_{A1547} | R^{B7} | R^{B5} | H | R^{B4} |
| L_{A1276} | R^{B7} | R^{B4} | R^{A3} | H | L_{A1548} | R^{B7} | R^{B5} | H | R^{B7} |
| L_{A1277} | R^{B7} | R^{B4} | R^{A34} | H | L_{A1549} | R^{B7} | R^{B5} | H | R^{B12} |
| L_{A1278} | R^{B7} | R^{B5} | H | H | L_{A1550} | R^{B7} | R^{B5} | H | R^{B18} |
| L_{A1279} | R^{B7} | R^{B5} | R^{B1} | H | L_{A1551} | R^{B7} | R^{B5} | H | R^{A3} |
| L_{A1280} | R^{B7} | R^{B5} | R^{B3} | H | L_{A1552} | R^{B7} | R^{B5} | H | R^{A34} |
| L_{A1281} | R^{B7} | R^{B5} | R^{B4} | H | L_{A1553} | R^{B7} | R^{B6} | H | R^{B1} |
| L_{A1282} | R^{B7} | R^{B5} | R^{B7} | H | L_{A1554} | R^{B7} | R^{B6} | H | R^{B3} |
| L_{A1283} | R^{B7} | R^{B5} | R^{B12} | H | L_{A1555} | R^{B7} | R^{B6} | H | R^{B4} |
| L_{A1284} | R^{B7} | R^{B5} | R^{B18} | H | L_{A1556} | R^{B7} | R^{B6} | H | R^{B7} |
| L_{A1285} | R^{B7} | R^{B5} | R^{A3} | H | L_{A1557} | R^{B7} | R^{B6} | H | R^{B12} |
| L_{A1286} | R^{B7} | R^{B5} | R^{A34} | H | L_{A1558} | R^{B7} | R^{B6} | H | R^{B18} |
| L_{A1287} | R^{B7} | R^{B6} | H | H | L_{A1559} | R^{B7} | R^{B6} | H | R^{A3} |
| L_{A1288} | R^{B7} | R^{B6} | R^{B1} | H | L_{A1560} | R^{B7} | R^{B6} | H | R^{A34} |
| L_{A1289} | R^{B7} | R^{B6} | R^{B3} | H | L_{A1561} | R^{B7} | R^{B12} | H | R^{B1} |
| L_{A1290} | R^{B7} | R^{B6} | R^{B4} | H | L_{A1562} | R^{B7} | R^{B12} | H | R^{B3} |
| L_{A1291} | R^{B7} | R^{B6} | R^{B7} | H | L_{A1563} | R^{B7} | R^{B12} | H | R^{B4} |
| L_{A1292} | R^{B7} | R^{B6} | R^{B12} | H | L_{A1564} | R^{B7} | R^{B12} | H | R^{B7} |
| L_{A1293} | R^{B7} | R^{B6} | R^{B18} | H | L_{A1565} | R^{B7} | R^{B12} | H | R^{B12} |
| L_{A1294} | R^{B7} | R^{B6} | R^{A3} | H | L_{A1566} | R^{B7} | R^{B12} | H | R^{B18} |
| L_{A1295} | R^{B7} | R^{B6} | R^{A34} | H | L_{A1567} | R^{B7} | R^{B12} | H | R^{A3} |
| L_{A1296} | R^{B7} | R^{B12} | H | H | L_{A1568} | R^{B7} | R^{B12} | H | R^{A34} |
| L_{A1297} | R^{B7} | R^{B12} | R^{B1} | H | L_{A1569} | R^{B7} | R^{A34} | H | R^{B1} |
| L_{A1298} | R^{B7} | R^{B12} | R^{B3} | H | L_{A1570} | R^{B7} | R^{A34} | H | R^{B3} |
| L_{A1299} | R^{B7} | R^{B12} | R^{B4} | H | L_{A1571} | R^{B7} | R^{A34} | H | R^{B4} |
| L_{A1300} | R^{B7} | R^{B12} | R^{B7} | H | L_{A1572} | R^{B7} | R^{A34} | H | R^{B7} |
| L_{A1301} | R^{B7} | R^{B12} | R^{B12} | H | L_{A1573} | R^{B7} | R^{A34} | H | R^{B12} |
| L_{A1302} | R^{B7} | R^{B12} | R^{B18} | H | L_{A1574} | R^{B7} | R^{A34} | H | R^{B18} |
| L_{A1303} | R^{B7} | R^{B12} | R^{A3} | H | L_{A1575} | R^{B7} | R^{A34} | H | R^{A3} |
| L_{A1304} | R^{B7} | R^{B12} | R^{A34} | H | L_{A1576} | R^{B7} | R^{A34} | H | R^{A34} |
| L_{A1305} | R^{B7} | R^{A34} | H | H | L_{A1577} | R^{B43} | R^{B3} | H | R^{B1} |
| L_{A1306} | R^{B7} | R^{A34} | R^{B1} | H | L_{A1578} | R^{B43} | R^{B3} | H | R^{B3} |
| L_{A1307} | R^{B7} | R^{A34} | R^{B3} | H | L_{A1579} | R^{B43} | R^{B3} | H | R^{B4} |
| L_{A1308} | R^{B7} | R^{A34} | R^{B4} | H | L_{A1580} | R^{B43} | R^{B3} | H | R^{B7} |
| L_{A1309} | R^{B7} | R^{A34} | R^{B7} | H | L_{A1581} | R^{B43} | R^{B3} | H | R^{B12} |
| L_{A1310} | R^{B7} | R^{A34} | R^{B12} | H | L_{A1582} | R^{B43} | R^{B3} | H | R^{B18} |
| L_{A1311} | R^{B7} | R^{A34} | R^{B18} | H | L_{A1583} | R^{B43} | R^{B3} | H | R^{A3} |
| L_{A1312} | R^{B7} | R^{A34} | R^{A3} | H | L_{A1584} | R^{B43} | R^{B3} | H | R^{A34} |
| L_{A1313} | R^{B7} | R^{A34} | R^{A34} | H | L_{A1585} | R^{B43} | R^{B4} | H | R^{B1} |
| L_{A1314} | R^{B43} | R^{B3} | H | H | L_{A1586} | R^{B43} | R^{B4} | H | R^{B3} |
| L_{A1315} | R^{B43} | R^{B3} | R^{B1} | H | L_{A1587} | R^{B43} | R^{B4} | H | R^{B4} |
| L_{A1316} | R^{B43} | R^{B3} | R^{B3} | H | L_{A1588} | R^{B43} | R^{B4} | H | R^{B7} |
| L_{A1317} | R^{B43} | R^{B3} | R^{B4} | H | L_{A1589} | R^{B43} | R^{B4} | H | R^{B12} |
| L_{A1318} | R^{B43} | R^{B3} | R^{B7} | H | L_{A1590} | R^{B43} | R^{B4} | H | R^{B18} |
| L_{A1319} | R^{B43} | R^{B3} | R^{B12} | H | L_{A1591} | R^{B43} | R^{B4} | H | R^{A3} |
| L_{A1320} | R^{B43} | R^{B3} | R^{B18} | H | L_{A1592} | R^{B43} | R^{B4} | H | R^{A34} |
| L_{A1321} | R^{B43} | R^{B3} | R^{A3} | H | L_{A1593} | R^{B43} | R^{B5} | H | R^{B1} |
| L_{A1322} | R^{B43} | R^{B3} | R^{A34} | H | L_{A1594} | R^{B43} | R^{B5} | H | R^{B3} |
| L_{A1323} | R^{B43} | R^{B4} | H | H | L_{A1595} | R^{B43} | R^{B5} | H | R^{B4} |
| L_{A1324} | R^{B43} | R^{B4} | R^{B1} | H | L_{A1596} | R^{B43} | R^{B5} | H | R^{B7} |
| L_{A1325} | R^{B43} | R^{B4} | R^{B3} | H | L_{A1597} | R^{B43} | R^{B5} | H | R^{B12} |
| L_{A1326} | R^{B43} | R^{B4} | R^{B4} | H | L_{A1598} | R^{B43} | R^{B5} | H | R^{B18} |
| L_{A1327} | R^{B43} | R^{B4} | R^{B7} | H | L_{A1599} | R^{B43} | R^{B5} | H | R^{A3} |
| L_{A1328} | R^{B43} | R^{B4} | R^{B12} | H | L_{A1600} | R^{B43} | R^{B5} | H | R^{A34} |
| L_{A1329} | R^{B43} | R^{B4} | R^{B18} | H | L_{A1601} | R^{B43} | R^{B6} | H | R^{B1} |
| L_{A1330} | R^{B43} | R^{B4} | R^{A3} | H | L_{A1602} | R^{B43} | R^{B6} | H | R^{B3} |
| L_{A1331} | R^{B43} | R^{B4} | R^{A34} | H | L_{A1603} | R^{B43} | R^{B6} | H | R^{B4} |
| L_{A1332} | R^{B43} | R^{B5} | H | H | L_{A1604} | R^{B43} | R^{B6} | H | R^{B7} |
| L_{A1333} | R^{B43} | R^{B5} | R^{B1} | H | L_{A1605} | R^{B43} | R^{B6} | H | R^{B12} |
| L_{A1334} | R^{B43} | R^{B5} | R^{B3} | H | L_{A1606} | R^{B43} | R^{B6} | H | R^{B18} |
| L_{A1335} | R^{B43} | R^{B5} | R^{B4} | H | L_{A1607} | R^{B43} | R^{B6} | H | R^{A3} |
| L_{A1336} | R^{B43} | R^{B5} | R^{B7} | H | L_{A1608} | R^{B43} | R^{B6} | H | R^{A34} |
| L_{A1337} | R^{B43} | R^{B5} | R^{B12} | H | L_{A1609} | R^{B43} | R^{B7} | H | R^{B1} |
| L_{A1338} | R^{B43} | R^{B5} | R^{B18} | H | L_{A1610} | R^{B43} | R^{B7} | H | R^{B3} |
| L_{A1339} | R^{B43} | R^{B5} | R^{A3} | H | L_{A1611} | R^{B43} | R^{B7} | H | R^{B4} |
| L_{A1340} | R^{B43} | R^{B5} | R^{A34} | H | L_{A1612} | R^{B43} | R^{B7} | H | R^{B7} |
| L_{A1341} | R^{B43} | R^{B6} | H | H | L_{A1613} | R^{B43} | R^{B7} | H | R^{B12} |
| L_{A1342} | R^{B43} | R^{B6} | R^{B1} | H | L_{A1614} | R^{B43} | R^{B7} | H | R^{B18} |
| L_{A1343} | R^{B43} | R^{B6} | R^{B3} | H | L_{A1615} | R^{B43} | R^{B7} | H | R^{A3} |
| L_{A1344} | R^{B43} | R^{B6} | R^{B4} | H | L_{A1616} | R^{B43} | R^{B7} | H | R^{A34} |
| L_{A1345} | R^{B43} | R^{B6} | R^{B7} | H | L_{A1617} | R^{B43} | R^{B12} | H | R^{B1} |
| L_{A1346} | R^{B43} | R^{B6} | R^{B12} | H | L_{A1618} | R^{B43} | R^{B12} | H | R^{B3} |
| L_{A1347} | R^{B43} | R^{B6} | R^{B18} | H | L_{A1619} | R^{B43} | R^{B12} | H | R^{B4} |
| L_{A1348} | R^{B43} | R^{B6} | R^{A3} | H | L_{A1620} | R^{B43} | R^{B12} | H | R^{B7} |
| L_{A1349} | R^{B43} | R^{B6} | R^{A34} | H | L_{A1621} | R^{B43} | R^{B12} | H | R^{B12} |
| L_{A1350} | R^{B43} | R^{B7} | H | H | L_{A1622} | R^{B43} | R^{B12} | H | R^{B18} |
| L_{A1351} | R^{B43} | R^{B7} | R^{B1} | H | L_{A1623} | R^{B43} | R^{B12} | H | R^{A3} |
| L_{A1352} | R^{B43} | R^{B7} | R^{B3} | H | L_{A1624} | R^{B43} | R^{B12} | H | R^{A34} |
| L_{A1353} | R^{B43} | R^{B7} | R^{B4} | H | L_{A1625} | R^{B43} | R^{A34} | H | R^{B1} |
| L_{A1354} | R^{B43} | R^{B7} | R^{B7} | H | L_{A1626} | R^{B43} | R^{A34} | H | R^{B3} |
| L_{A1355} | R^{B43} | R^{B7} | R^{B12} | H | L_{A1627} | R^{B43} | R^{A34} | H | R^{B4} |
| L_{A1356} | R^{B43} | R^{B7} | R^{B18} | H | L_{A1628} | R^{B43} | R^{A34} | H | R^{B7} |
| L_{A1357} | R^{B43} | R^{B7} | R^{A3} | H | L_{A1629} | R^{B43} | R^{A34} | H | R^{B12} |
| L_{A1358} | R^{B43} | R^{B7} | R^{A34} | H | L_{A1630} | R^{B43} | R^{A34} | H | R^{B18} |
| L_{A1359} | R^{B43} | R^{B12} | H | H | L_{A1631} | R^{B43} | R^{A34} | H | R^{A3} |
| L_{A1360} | R^{B43} | R^{B12} | R^{B1} | H | L_{A1632} | R^{B43} | R^{A34} | H | R^{A34} |

L_{A1633} through L_{A2176}, that are based on a structure of Formula Id, 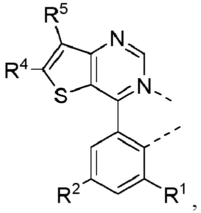 in each of which R¹, R², R⁴, and R⁵ are defined as follows:

| | **R¹** | **R²** | **R⁴** | **R⁵** | | **R¹** | **R³** | **R⁴** | **R⁵** |
|---|---|---|---|---|---|---|---|---|---|
| L_{A1633} | R^{B1} | R^{B3} | H | H | L_{A1905} | R^{B43} | R^{B12} | R^{B3} | H |
| L_{A1634} | R^{B1} | R^{B3} | R^{B1} | H | L_{A1906} | R^{B43} | R^{B12} | R^{B4} | H |
| L_{A1635} | R^{B1} | R^{B3} | R^{B3} | H | L_{A1907} | R^{B43} | R^{B12} | R^{B7} | H |
| L_{A1636} | R^{B1} | R^{B3} | R^{B4} | H | L_{A1908} | R^{B43} | R^{B12} | R^{B12} | H |
| L_{A1637} | R^{B1} | R^{B3} | R^{B7} | H | L_{A1909} | R^{B43} | R^{B12} | R^{B18} | H |
| L_{A1638} | R^{B1} | R^{B3} | R^{B12} | H | L_{A1910} | R^{B43} | R^{B12} | R^{A3} | H |
| L_{A1639} | R^{B1} | R^{B3} | R^{B18} | H | L_{A1911} | R^{B43} | R^{B12} | R^{A34} | H |
| L_{A1640} | R^{B1} | R^{B3} | R^{A3} | H | L_{A1912} | R^{B43} | R^{A34} | H | H |
| L_{A1641} | R^{B1} | R^{B3} | R^{A34} | H | L_{A1913} | R^{B43} | R^{A34} | R^{B1} | H |
| L_{A1642} | R^{B1} | R^{B4} | H | H | L_{A1914} | R^{B43} | R^{A34} | R^{B3} | H |
| L_{A1643} | R^{B1} | R^{B4} | R^{B1} | H | L_{A1915} | R^{B43} | R^{A34} | R^{B4} | H |
| L_{A1644} | R^{B1} | R^{B4} | R^{B3} | H | L_{A1916} | R^{B43} | R^{A34} | R^{B7} | H |
| L_{A1645} | R^{B1} | R^{B4} | R^{B4} | H | L_{A1917} | R^{B43} | R^{A34} | R^{B12} | H |
| L_{A1646} | R^{B1} | R^{B4} | R^{B7} | H | L_{A1918} | R^{B43} | R^{A34} | R^{B18} | H |
| L_{A1647} | R^{B1} | R^{B4} | R^{B12} | H | L_{A1919} | R^{B43} | R^{A34} | R^{A3} | H |
| L_{A1648} | R^{B1} | R^{B4} | R^{B18} | H | L_{A1920} | R^{B43} | R^{A34} | R^{A34} | H |
| L_{A1649} | R^{B1} | R^{B4} | R^{A3} | H | L_{A1921} | R^{B1} | R^{B3} | H | R^{B1} |
| L_{A1650} | R^{B1} | R^{B4} | R^{A34} | H | L_{A1922} | R^{B1} | R^{B3} | H | R^{B3} |
| L_{A1651} | R^{B1} | R^{B5} | H | H | L_{A1923} | R^{B1} | R^{B3} | H | R^{B4} |
| L_{A1652} | R^{B1} | R^{B5} | R^{B1} | H | L_{A1924} | R^{B1} | R^{B3} | H | R^{B7} |
| L_{A1653} | R^{B1} | R^{B5} | R^{B3} | H | L_{A1925} | R^{B1} | R^{B3} | H | R^{B12} |
| L_{A1654} | R^{B1} | R^{B5} | R^{B4} | H | L_{A1926} | R^{B1} | R^{B3} | H | R^{B18} |
| L_{A1655} | R^{B1} | R^{B5} | R^{B7} | H | L_{A1927} | R^{B1} | R^{B3} | H | R^{A3} |
| L_{A1656} | R^{B1} | R^{B5} | R^{B12} | H | L_{A1928} | R^{B1} | R^{B3} | H | R^{A34} |
| L_{A1657} | R^{B1} | R^{B5} | R^{B18} | H | L_{A1929} | R^{B1} | R^{B4} | H | R^{B1} |
| L_{A1658} | R^{B1} | R^{B5} | R^{A3} | H | L_{A1930} | R^{B7} | R^{B4} | H | R^{B3} |
| L_{A1659} | R^{B1} | R^{B5} | R^{A34} | H | L_{A1931} | R^{B1} | R^{B4} | H | R^{B4} |
| L_{A1660} | R^{B1} | R^{B6} | H | H | L_{A1932} | R^{B1} | R^{B4} | H | R^{B7} |
| L_{A1661} | R^{B1} | R^{B6} | R^{B1} | H | L_{A1933} | R^{B1} | R^{B4} | H | R^{B12} |
| L_{A1662} | R^{B1} | R^{B6} | R^{B3} | H | L_{A1934} | R^{B1} | R^{B4} | H | R^{B18} |
| L_{A1663} | R^{B1} | R^{B6} | R^{B4} | H | L_{A1935} | R^{B1} | R^{B4} | H | R^{A3} |
| L_{A1664} | R^{B1} | R^{B6} | R^{B7} | H | L_{A1936} | R^{B1} | R^{B4} | H | R^{A34} |
| L_{A1665} | R^{B1} | R^{B6} | R^{B12} | H | L_{A1937} | R^{B1} | R^{B5} | H | R^{B1} |
| L_{A1666} | R^{B1} | R^{B6} | R^{B18} | H | L_{A1938} | R^{B1} | R^{B5} | H | R^{B3} |
| L_{A1667} | R^{B1} | R^{B6} | R^{A3} | H | L_{A1939} | R^{B1} | R^{B5} | H | R^{B4} |
| L_{A1668} | R^{B1} | R^{B6} | R^{A34} | H | L_{A1940} | R^{B1} | R^{B5} | H | R^{B7} |
| L_{A1669} | R^{B1} | R^{B7} | H | H | L_{A1941} | R^{B1} | R^{B5} | H | R^{B12} |
| L_{A1670} | R^{B1} | R^{B7} | R^{B1} | H | L_{A1942} | R^{B1} | R^{B5} | H | R^{B18} |
| L_{A1671} | R^{B1} | R^{B7} | R^{B3} | H | L_{A1943} | R^{B1} | R^{B5} | H | R^{A3} |
| L_{A1672} | R^{B1} | R^{B7} | R^{B4} | H | L_{A1944} | R^{B1} | R^{B5} | H | R^{A34} |
| L_{A1673} | R^{B1} | R^{B7} | R^{B7} | H | L_{A1945} | R^{B1} | R^{B6} | H | R^{B1} |
| L_{A1674} | R^{B1} | R^{B7} | R^{B12} | H | L_{A1946} | R^{B1} | R^{B6} | H | R^{B3} |
| L_{A1675} | R^{B1} | R^{B7} | R^{B18} | H | L_{A1947} | R^{B1} | R^{B6} | H | R^{B4} |
| L_{A1676} | R^{B1} | R^{B7} | R^{A3} | H | L_{A1948} | R^{B1} | R^{B6} | H | R^{B7} |
| L_{A1677} | R^{B1} | R^{B7} | R^{A34} | H | L_{A1949} | R^{B1} | R^{B6} | H | R^{B12} |
| L_{A1678} | R^{B1} | R^{B12} | H | H | L_{A1950} | R^{B1} | R^{B6} | H | R^{B18} |
| L_{A1679} | R^{B1} | R^{B12} | R^{B1} | H | L_{A1951} | R^{B1} | R^{B6} | H | R^{A3} |
| L_{A1680} | R^{B1} | R^{B12} | R^{B3} | H | L_{A1952} | R^{B1} | R^{B6} | H | R^{A34} |
| L_{A1681} | R^{B1} | R^{B12} | R^{B4} | H | L_{A1953} | R^{B1} | R^{B7} | H | R^{B1} |
| L_{A1682} | R^{B1} | R^{B12} | R^{B7} | H | L_{A1954} | R^{B7} | R^{B7} | H | R^{B3} |
| L_{A1683} | R^{B1} | R^{B12} | R^{B12} | H | L_{A1955} | R^{B1} | R^{B7} | H | R^{B4} |
| L_{A1684} | R^{B1} | R^{B12} | R^{B18} | H | L_{A1956} | R^{B1} | R^{B7} | H | R^{B7} |
| L_{A1685} | R^{B1} | R^{B12} | R^{A3} | H | L_{A1957} | R^{B1} | R^{B7} | H | R^{B12} |
| L_{A1686} | R^{B1} | R^{B12} | R^{A34} | H | L_{A1958} | R^{B1} | R^{B7} | H | R^{B18} |
| L_{A1687} | R^{B1} | R^{A34} | H | H | L_{A1959} | R^{B1} | R^{B7} | H | R^{A3} |
| L_{A1688} | R^{B1} | R^{A34} | R^{B1} | H | L_{A1960} | R^{B1} | R^{B7} | H | R^{A34} |
| L_{A1689} | R^{B1} | R^{A34} | R^{B3} | H | L_{A1961} | R^{B1} | R^{B12} | H | R^{B1} |
| L_{A1690} | R^{B1} | R^{A34} | R^{B4} | H | L_{A1962} | R^{B1} | R^{B12} | H | R^{B3} |
| L_{A1691} | R^{B1} | R^{A34} | R^{B7} | H | L_{A1963} | R^{B1} | R^{B12} | H | R^{B4} |
| L_{A1692} | R^{B1} | R^{A34} | R^{B12} | H | L_{A1964} | R^{B1} | R^{B12} | H | R^{B7} |
| L_{A1693} | R^{B1} | R^{A34} | R^{B18} | H | L_{A1965} | R^{B1} | R^{B12} | H | R^{B12} |
| L_{A1694} | R^{B1} | R^{A34} | R^{A3} | H | L_{A1966} | R^{B1} | R^{B12} | H | R^{B18} |
| L_{A1695} | R^{B1} | R^{A34} | R^{A34} | H | L_{A1967} | R^{B1} | R^{B12} | H | R^{A3} |
| L_{A1696} | R^{B3} | R^{B4} | H | H | L_{A1968} | R^{B1} | R^{B12} | H | R^{A34} |
| L_{A1697} | R^{B3} | R^{B4} | R^{B1} | H | L_{A1969} | R^{B1} | R^{A34} | H | R^{B1} |
| L_{A1698} | R^{B3} | R^{B4} | R^{B3} | H | L_{A1970} | R^{B1} | R^{A34} | H | R^{B3} |
| L_{A1699} | R^{B3} | R^{B4} | R^{B4} | H | L_{A1971} | R^{B1} | R^{A34} | H | R^{B4} |
| L_{A1700} | R^{B3} | R^{B4} | R^{B7} | H | L_{A1972} | R^{B1} | R^{A34} | H | R^{B7} |
| L_{A1701} | R^{B3} | R^{B4} | R^{B12} | H | L_{A1973} | R^{B1} | R^{A34} | H | R^{B12} |
| L_{A1702} | R^{B3} | R^{B4} | R^{B18} | H | L_{A1974} | R^{B1} | R^{A34} | H | R^{B18} |
| L_{A1703} | R^{B3} | R^{B4} | R^{A3} | H | L_{A1975} | R^{B1} | R^{A34} | H | R^{A3} |
| L_{A1704} | R^{B3} | R^{B4} | R^{A34} | H | L_{A1976} | R^{B1} | R^{A34} | H | R^{A34} |
| L_{A1705} | R^{B3} | R^{B5} | H | H | L_{A1977} | R^{B3} | R^{B4} | H | R^{B1} |
| L_{A1706} | R^{B3} | R^{B5} | R^{B1} | H | L_{A1978} | R^{B3} | R^{B4} | H | R^{B3} |
| L_{A1707} | R^{B3} | R^{B5} | R^{B3} | H | L_{A1979} | R^{B3} | R^{B4} | H | R^{B4} |
| L_{A1708} | R^{B3} | R^{B5} | R^{B4} | H | L_{A1980} | R^{B3} | R^{B4} | H | R^{B7} |
| L_{A1709} | R^{B3} | R^{B5} | R^{B7} | H | L_{A1981} | R^{B3} | R^{B4} | H | R^{B12} |
| L_{A1710} | R^{B3} | R^{B5} | R^{B12} | H | L_{A1982} | R^{B3} | R^{B4} | H | R^{B18} |
| L_{A1711} | R^{B3} | R^{B5} | R^{B18} | H | L_{A1983} | R^{B3} | R^{B4} | H | R^{A3} |
| L_{A1712} | R^{B3} | R^{B5} | R^{A3} | H | L_{A1984} | R^{B3} | R^{B4} | H | R^{A34} |
| L_{A1713} | R^{B3} | R^{B5} | R^{A34} | H | L_{A1985} | R^{B3} | R^{B5} | H | R^{B1} |
| L_{A1714} | R^{B3} | R^{B6} | H | H | L_{A1986} | R^{B3} | R^{B5} | H | R^{B3} |
| L_{A1715} | R^{B3} | R^{B6} | R^{B1} | H | L_{A1987} | R^{B3} | R^{B5} | H | R^{B4} |
| L_{A1716} | R^{B3} | R^{B6} | R^{B3} | H | L_{A1988} | R^{B3} | R^{B5} | H | R^{B7} |
| L_{A1717} | R^{B3} | R^{B6} | R^{B4} | H | L_{A1989} | R^{B3} | R^{B5} | H | R^{B12} |
| L_{A1718} | R^{B3} | R^{B6} | R^{B7} | H | L_{A1990} | R^{B3} | R^{B5} | H | R^{B18} |
| L_{A1719} | R^{B3} | R^{B6} | R^{B12} | H | L_{A1991} | R^{B3} | R^{B5} | H | R^{A3} |
| L_{A1720} | R^{B3} | R^{B6} | R^{B18} | H | L_{A1992} | R^{B3} | R^{B5} | H | R^{A34} |
| L_{A1721} | R^{B3} | R^{B6} | R^{A3} | H | L_{A1993} | R^{B3} | R^{B6} | H | R^{B1} |
| L_{A1722} | R^{B3} | R^{B6} | R^{A34} | H | L_{A1994} | R^{B3} | R^{B6} | H | R^{B3} |
| L_{A1723} | R^{B3} | R^{B7} | H | H | L_{A1995} | R^{B3} | R^{B6} | H | R^{B4} |
| L_{A1724} | R^{B3} | R^{B7} | R^{B1} | H | L_{A1996} | R^{B3} | R^{B6} | H | R^{B7} |
| L_{A1725} | R^{B3} | R^{B7} | R^{B3} | H | L_{A1997} | R^{B3} | R^{B6} | H | R^{B12} |
| L_{A1726} | R^{B3} | R^{B7} | R^{B4} | H | L_{A1998} | R^{B3} | R^{B6} | H | R^{B18} |
| L_{A1727} | R^{B3} | R^{B7} | R^{B7} | H | L_{A1999} | R^{B3} | R^{B6} | H | R^{A3} |
| L_{A1728} | R^{B3} | R^{B7} | R^{B12} | H | L_{A2000} | R^{B3} | R^{B6} | H | R^{A34} |
| L_{A1729} | R^{B3} | R^{B7} | R^{B18} | H | L_{A2001} | R^{B3} | R^{B7} | H | R^{B1} |
| L_{A1730} | R^{B3} | R^{B7} | R^{A3} | H | L_{A2002} | R^{B3} | R^{B7} | H | R^{B3} |
| L_{A1731} | R^{B3} | R^{B7} | R^{A34} | H | L_{A2003} | R^{B3} | R^{B7} | H | R^{B4} |
| L_{A1732} | R^{B3} | R^{B12} | H | H | L_{A2004} | R^{B3} | R^{B7} | H | R^{B7} |
| L_{A1733} | R^{B3} | R^{B12} | R^{B1} | H | L_{A2005} | R^{B3} | R^{B7} | H | R^{B12} |
| L_{A1734} | R^{B3} | R^{B12} | R^{B3} | H | L_{A2006} | R^{B3} | R^{B7} | H | R^{B18} |
| L_{A1735} | R^{B3} | R^{B12} | R^{B4} | H | L_{A2007} | R^{B3} | R^{B7} | H | R^{A3} |
| L_{A1736} | R^{B3} | R^{B12} | R^{B7} | H | L_{A2008} | R^{B3} | R^{B7} | H | R^{A34} |
| L_{A1737} | R^{B3} | R^{B12} | R^{B12} | H | L_{A2009} | R^{B3} | R^{B12} | H | R^{B1} |
| L_{A1738} | R^{B3} | R^{B12} | R^{B18} | H | L_{A2010} | R^{B3} | R^{B12} | H | R^{B3} |
| L_{A1739} | R^{B3} | R^{B12} | R^{A3} | H | L_{A2011} | R^{B3} | R^{B12} | H | R^{B4} |
| L_{A1740} | R^{B3} | R^{B12} | R^{A34} | H | L_{A2012} | R^{B3} | R^{B12} | H | R^{B7} |
| L_{A1741} | R^{B3} | R^{A34} | H | H | L_{A2013} | R^{B3} | R^{B12} | H | R^{B12} |
| L_{A1742} | R^{B3} | R^{A34} | R^{B1} | H | L_{A2014} | R^{B3} | R^{B12} | H | R^{B18} |
| L_{A1743} | R^{B3} | R^{A34} | R^{B3} | H | L_{A2015} | R^{B3} | R^{B12} | H | R^{A3} |
| L_{A1744} | R^{B3} | R^{A34} | R^{B4} | H | L_{A2016} | R^{B3} | R^{B12} | H | R^{A34} |
| L_{A1745} | R^{B3} | R^{A34} | R^{B7} | H | L_{A2017} | R^{B3} | R^{A34} | H | R^{B1} |
| L_{A1746} | R^{B3} | R^{A34} | R^{B12} | H | L_{A2018} | R^{B3} | R^{A34} | H | R^{B3} |
| L_{A1747} | R^{B3} | R^{A34} | R^{B18} | H | L_{A2019} | R^{B3} | R^{A34} | H | R^{B4} |
| L_{A1748} | R^{B3} | R^{A34} | R^{A3} | H | L_{A2020} | R^{B3} | R^{A34} | H | R^{B7} |
| L_{A1749} | R^{B3} | R^{A34} | R^{A34} | H | L_{A2021} | R^{B3} | R^{A34} | H | R^{B12} |
| L_{A1750} | R^{B4} | R^{B3} | H | H | L_{A2022} | R^{B3} | R^{A34} | H | R^{B18} |
| L_{A1751} | R^{B4} | R^{B3} | R^{B1} | H | L_{A2023} | R^{B3} | R^{A34} | H | R^{A3} |
| L_{A1752} | R^{B4} | R^{B3} | R^{B3} | H | L_{A2024} | R^{B3} | R^{A34} | H | R^{A34} |
| L_{A1753} | R^{B4} | R^{B3} | R^{B4} | H | L_{A2025} | R^{B4} | R^{B3} | H | R^{B1} |
| L_{A1754} | R^{B4} | R^{B3} | R^{B7} | H | L_{A2026} | R^{B4} | R^{B3} | H | R^{B3} |
| L_{A1755} | R^{B4} | R^{B3} | R^{B12} | H | L_{A2027} | R^{B4} | R^{B3} | H | R^{B4} |
| L_{A1756} | R^{B4} | R^{B3} | R^{B18} | H | L_{A2028} | R^{B4} | R^{B3} | H | R^{B7} |
| L_{A1757} | R^{B4} | R^{B3} | R^{A3} | H | L_{A2029} | R^{B4} | R^{B3} | H | R^{B12} |
| L_{A1758} | R^{B4} | R^{B3} | R^{A34} | H | L_{A2030} | R^{B4} | R^{B3} | H | R^{B18} |
| L_{A1759} | R^{B4} | R^{B5} | H | H | L_{A2031} | R^{B4} | R^{B3} | H | R^{A3} |
| L_{A1760} | R^{B4} | R^{B5} | R^{B1} | H | L_{A2032} | R^{B4} | R^{B3} | H | R^{A34} |
| L_{A1761} | R^{B4} | R^{B5} | R^{B3} | H | L_{A2033} | R^{B4} | R^{B5} | H | R^{B1} |
| L_{A1762} | R^{B4} | R^{B5} | R^{B4} | H | L_{A2034} | R^{B4} | R^{B5} | H | R^{B3} |
| L_{A1763} | R^{B4} | R^{B5} | R^{B7} | H | L_{A2035} | R^{B4} | R^{B5} | H | R^{B4} |
| L_{A1764} | R^{B4} | R^{B5} | R^{B12} | H | L_{A2036} | R^{B4} | R^{B5} | H | R^{B7} |
| L_{A1765} | R^{B4} | R^{B5} | R^{B18} | H | L_{A2037} | R^{B4} | R^{B5} | H | R^{B12} |
| L_{A1766} | R^{B4} | R^{B5} | R^{A3} | H | L_{A2038} | R^{B4} | R^{B5} | H | R^{B18} |
| L_{A1767} | R^{B4} | R^{B5} | R^{A34} | H | L_{A2039} | R^{B4} | R^{B5} | H | R^{A3} |
| L_{A1768} | R^{B4} | R^{B6} | H | H | L_{A2040} | R^{B4} | R^{B5} | H | R^{A34} |
| L_{A1769} | R^{B4} | R^{B6} | R^{B1} | H | L_{A2041} | R^{B4} | R^{B6} | H | R^{B1} |
| L_{A1770} | R^{B4} | R^{B6} | R^{B3} | H | L_{A2042} | R^{B4} | R^{B6} | H | R^{B3} |
| L_{A1771} | R^{B4} | R^{B6} | R^{B4} | H | L_{A2043} | R^{B4} | R^{B6} | H | R^{B4} |
| L_{A1772} | R^{B4} | R^{B6} | R^{B7} | H | L_{A2044} | R^{B4} | R^{B6} | H | R^{B7} |
| L_{A1773} | R^{B4} | R^{B6} | R^{B12} | H | L_{A2045} | R^{B4} | R^{B6} | H | R^{B12} |
| L_{A1774} | R^{B4} | R^{B6} | R^{B18} | H | L_{A2046} | R^{B4} | R^{B6} | H | R^{B18} |
| L_{A1775} | R^{B4} | R^{B6} | R^{A3} | H | L_{A2047} | R^{B4} | R^{B6} | H | R^{A3} |
| L_{A1776} | R^{B4} | R^{B6} | R^{A34} | H | L_{A2048} | R^{B4} | R^{B6} | H | R^{A34} |
| L_{A1777} | R^{B4} | R^{B7} | H | H | L_{A2049} | R^{B4} | R^{B7} | H | R^{B1} |
| L_{A1778} | R^{B4} | R^{B7} | R^{B1} | H | L_{A2050} | R^{B4} | R^{B7} | H | R^{B3} |
| L_{A1779} | R^{B4} | R^{B7} | R^{B3} | H | L_{A2051} | R^{B4} | R^{B7} | H | R^{B4} |
| L_{A1780} | R^{B4} | R^{B7} | R^{B4} | H | L_{A2052} | R^{B4} | R^{B7} | H | R^{B7} |
| L_{A1781} | R^{B4} | R^{B7} | R^{B7} | H | L_{A2053} | R^{B4} | R^{B7} | H | R^{B12} |
| L_{A1782} | R^{B4} | R^{B7} | R^{B12} | H | L_{A2054} | R^{B4} | R^{B7} | H | R^{B18} |
| L_{A1783} | R^{B4} | R^{B7} | R^{B18} | H | L_{A2055} | R^{B4} | R^{B7} | H | R^{A3} |
| L_{A1784} | R^{B4} | R^{B7} | R^{A3} | H | L_{A2056} | R^{B4} | R^{B7} | H | R^{A34} |
| L_{A1785} | R^{B4} | R^{B7} | R^{A34} | H | L_{A2057} | R^{B4} | R^{B12} | H | R^{B1} |
| L_{A1786} | R^{B4} | R^{B12} | H | H | L_{A2058} | R^{B4} | R^{B12} | H | R^{B3} |
| L_{A1787} | R^{B4} | R^{B12} | R^{B1} | H | L_{A2059} | R^{B4} | R^{B12} | H | R^{B4} |
| L_{A1788} | R^{B4} | R^{B12} | R^{B3} | H | L_{A2060} | R^{B4} | R^{B12} | H | R^{B7} |
| L_{A1789} | R^{B4} | R^{B12} | R^{B4} | H | L_{A2061} | R^{B4} | R^{B12} | H | R^{B12} |
| L_{A1790} | R^{B4} | R^{B12} | R^{B7} | H | L_{A2062} | R^{B4} | R^{B12} | H | R^{B18} |
| L_{A1791} | R^{B4} | R^{B12} | R^{B12} | H | L_{A2063} | R^{B4} | R^{B12} | H | R^{A3} |
| L_{A1792} | R^{B4} | R^{B12} | R^{B18} | H | L_{A2064} | R^{B4} | R^{B12} | H | R^{A34} |
| L_{A1793} | R^{B4} | R^{B12} | R^{A3} | H | L_{A2065} | R^{B4} | R^{A34} | H | R^{B1} |
| L_{A1794} | R^{B4} | R^{B12} | R^{A34} | H | L_{A2066} | R^{B4} | R^{A34} | H | R^{B3} |
| L_{A1795} | R^{B4} | R^{A34} | H | H | L_{A2067} | R^{B4} | R^{A34} | H | R^{B4} |
| L_{A1796} | R^{B4} | R^{A34} | R^{B1} | H | L_{A2068} | R^{B4} | R^{A34} | H | R^{B7} |
| L_{A1797} | R^{B4} | R^{A34} | R^{B3} | H | L_{A2069} | R^{B4} | R^{A34} | H | R^{B12} |
| L_{A1798} | R^{B4} | R^{A34} | R^{B4} | H | L_{A2070} | R^{B4} | R^{A34} | H | R^{B18} |
| L_{A1799} | R^{B4} | R^{A34} | R^{B7} | H | L_{A2071} | R^{B4} | R^{A34} | H | R^{A3} |
| L_{A1800} | R^{B4} | R^{A34} | R^{B12} | H | L_{A2072} | R^{B4} | R^{A34} | H | R^{A34} |
| L_{A1801} | R^{B4} | R^{A34} | R^{B18} | H | L_{A2073} | R^{B7} | R^{B3} | H | R^{B1} |
| L_{A1802} | R^{B4} | R^{A34} | R^{A3} | H | L_{A2074} | R^{B7} | R^{B3} | H | R^{B3} |
| L_{A1803} | R^{B4} | R^{A34} | R^{A34} | H | L_{A2075} | R^{B7} | R^{B3} | H | R^{B4} |
| L_{A1804} | R^{B7} | R^{B3} | H | H | L_{A2076} | R^{B7} | R^{B3} | H | R^{B7} |
| L_{A1805} | R^{B7} | R^{B3} | R^{B1} | H | L_{A2077} | R^{B7} | R^{B3} | H | R^{B12} |
| L_{A1806} | R^{B7} | R^{B3} | R^{B3} | H | L_{A2078} | R^{B7} | R^{B3} | H | R^{B18} |
| L_{A1807} | R^{B7} | R^{B3} | R^{B4} | H | L_{A2079} | R^{B7} | R^{B3} | H | R^{A3} |
| L_{A1808} | R^{B7} | R^{B3} | R^{B7} | H | L_{A2080} | R^{B7} | R^{B3} | H | R^{A34} |
| L_{A1809} | R^{B7} | R^{B3} | R^{B12} | H | L_{A2081} | R^{B7} | R^{B4} | H | R^{B1} |
| L_{A1810} | R^{B7} | R^{B3} | R^{B18} | H | L_{A2082} | R^{B7} | R^{B4} | H | R^{B3} |
| L_{A1811} | R^{B7} | R^{B3} | R^{A3} | H | L_{A2083} | R^{B7} | R^{B4} | H | R^{B4} |
| L_{A1812} | R^{B7} | R^{B3} | R^{A34} | H | L_{A2084} | R^{B7} | R^{B4} | H | R^{B7} |
| L_{A1813} | R^{B7} | R^{B4} | H | H | L_{A2085} | R^{B7} | R^{B4} | H | R^{B12} |
| L_{A1814} | R^{B7} | R^{B4} | R^{B1} | H | L_{A2086} | R^{B7} | R^{B4} | H | R^{B18} |
| L_{A1815} | R^{B7} | R^{B4} | R^{B3} | H | L_{A2087} | R^{B7} | R^{B4} | H | R^{A3} |
| L_{A1816} | R^{B7} | R^{B4} | R^{B4} | H | L_{A2088} | R^{B7} | R^{B4} | H | R^{A34} |
| L_{A1817} | R^{B7} | R^{B4} | R^{B7} | H | L_{A2089} | R^{B7} | R^{B5} | H | R^{B1} |
| L_{A1818} | R^{B7} | R^{B4} | R^{B12} | H | L_{A2090} | R^{B7} | R^{B5} | H | R^{B3} |
| L_{A1819} | R^{B7} | R^{B4} | R^{B18} | H | L_{A2091} | R^{B7} | R^{B5} | H | R^{B4} |
| L_{A1820} | R^{B7} | R^{B4} | R^{A3} | H | L_{A2092} | R^{B7} | R^{B5} | H | R^{B7} |
| L_{A1821} | R^{B7} | R^{B4} | R^{A34} | H | L_{A2093} | R^{B7} | R^{B5} | H | R^{B12} |
| L_{A1822} | R^{B7} | R^{B5} | H | H | L_{A2094} | R^{B7} | R^{B5} | H | R^{B18} |
| L_{A1823} | R^{B7} | R^{B5} | R^{B1} | H | L_{A2095} | R^{B7} | R^{B5} | H | R^{A3} |
| L_{A1824} | R^{B7} | R^{B5} | R^{B3} | H | L_{A2096} | R^{B7} | R^{B5} | H | R^{A34} |
| L_{A1825} | R^{B7} | R^{B5} | R^{B4} | H | L_{A2097} | R^{B7} | R^{B6} | H | R^{B1} |
| L_{A1826} | R^{B7} | R^{B5} | R^{B7} | H | L_{A2098} | R^{B7} | R^{B6} | H | R^{B3} |
| L_{A1827} | R^{B7} | R^{B5} | R^{B12} | H | L_{A2099} | R^{B7} | R^{B6} | H | R^{B4} |
| L_{A1828} | R^{B7} | R^{B5} | R^{B18} | H | L_{A2100} | R^{B7} | R^{B6} | H | R^{B7} |
| L_{A1829} | R^{B7} | R^{B5} | R^{A3} | H | L_{A2101} | R^{B7} | R^{B6} | H | R^{B12} |
| L_{A1830} | R^{B7} | R^{B5} | R^{A34} | H | L_{A2102} | R^{B7} | R^{B6} | H | R^{B18} |
| L_{A1831} | R^{B7} | R^{B6} | H | H | L_{A2103} | R^{B7} | R^{B6} | H | R^{A3} |
| L_{A1832} | R^{B7} | R^{B6} | R^{B1} | H | L_{A2104} | R^{B7} | R^{B6} | H | R^{A34} |
| L_{A1833} | R^{B7} | R^{B6} | R^{B3} | H | L_{A2105} | R^{B7} | R^{B12} | H | R^{B1} |
| L_{A1834} | R^{B7} | R^{B6} | R^{B4} | H | L_{A2106} | R^{B7} | R^{B12} | H | R^{B3} |
| L_{A1835} | R^{B7} | R^{B6} | R^{B7} | H | L_{A2107} | R^{B7} | R^{B12} | H | R^{B4} |
| L_{A1836} | R^{B7} | R^{B6} | R^{B12} | H | L_{A2108} | R^{B7} | R^{B12} | H | R^{B7} |
| L_{A1837} | R^{B7} | R^{B6} | R^{B18} | H | L_{A2109} | R^{B7} | R^{B12} | H | R^{B12} |
| L_{A1838} | R^{B7} | R^{B6} | R^{A3} | H | L_{A2110} | R^{B7} | R^{B12} | H | R^{B18} |
| L_{A1839} | R^{B7} | R^{B6} | R^{A34} | H | L_{A2111} | R^{B7} | R^{B12} | H | R^{A3} |
| L_{A1840} | R^{B7} | R^{B12} | H | H | L_{A2112} | R^{B7} | R^{B12} | H | R^{A34} |
| L_{A1841} | R^{B7} | R^{B12} | R^{B1} | H | L_{A2113} | R^{B7} | R^{A34} | H | R^{B1} |
| L_{A1842} | R^{B7} | R^{B12} | R^{B3} | H | L_{A2114} | R^{B7} | R^{A34} | H | R^{B3} |
| L_{A1843} | R^{B7} | R^{B12} | R^{B4} | H | L_{A2115} | R^{B7} | R^{A34} | H | R^{B4} |
| L_{A1844} | R^{B7} | R^{B12} | R^{B7} | H | L_{A2116} | R^{B7} | R^{A34} | H | R^{B7} |
| L_{A1845} | R^{B7} | R^{B12} | R^{B12} | H | L_{A2117} | R^{B7} | R^{A34} | H | R^{B12} |
| L_{A1846} | R^{B7} | R^{B12} | R^{B18} | H | L_{A2118} | R^{B7} | R^{A34} | H | R^{B18} |
| L_{A1847} | R^{B7} | R^{B12} | R^{A3} | H | L_{A2119} | R^{B7} | R^{A34} | H | R^{A3} |
| L_{A1848} | R^{B7} | R^{B12} | R^{A34} | H | L_{A2120} | R^{B7} | R^{A34} | H | R^{A34} |
| L_{A1849} | R^{B7} | R^{A34} | H | H | L_{A2121} | R^{B43} | R^{B3} | H | R^{B1} |
| L_{A1850} | R^{B7} | R^{A34} | R^{B1} | H | L_{A2122} | R^{B43} | R^{B3} | H | R^{B3} |
| L_{A1851} | R^{B7} | R^{A34} | R^{B3} | H | L_{A2123} | R^{B43} | R^{B3} | H | R^{B4} |
| L_{A1852} | R^{B7} | R^{A34} | R^{B4} | H | L_{A2124} | R^{B43} | R^{B3} | H | R^{B7} |
| L_{A1853} | R^{B7} | R^{A34} | R^{B7} | H | L_{A2125} | R^{B43} | R^{B3} | H | R^{B12} |
| L_{A1854} | R^{B7} | R^{A34} | R^{B12} | H | L_{A2126} | R^{B43} | R^{B3} | H | R^{B18} |
| L_{A1855} | R^{B7} | R^{A34} | R^{B18} | H | L_{A2127} | R^{B43} | R^{B3} | H | R^{A3} |
| L_{A1856} | R^{B7} | R^{A34} | R^{A3} | H | L_{A2128} | R^{B43} | R^{B3} | H | R^{A34} |
| L_{A1857} | R^{B7} | R^{A34} | R^{A34} | H | L_{A2129} | R^{B43} | R^{B4} | H | R^{B1} |
| L_{A1858} | R^{B43} | R^{B3} | H | H | L_{A2130} | R^{B43} | R^{B4} | H | R^{B3} |
| L_{A1859} | R^{B43} | R^{B3} | R^{B1} | H | L_{A2131} | R^{B43} | R^{B4} | H | R^{B4} |
| L_{A1860} | R^{B43} | R^{B3} | R^{B3} | H | L_{A2132} | R^{B43} | R^{B4} | H | R^{B7} |
| L_{A1861} | R^{B43} | R^{B3} | R^{B4} | H | L_{A2133} | R^{B43} | R^{B4} | H | R^{B12} |
| L_{A1862} | R^{B43} | R^{B3} | R^{B7} | H | L_{A2134} | R^{B43} | R^{B4} | H | R^{B18} |
| L_{A1863} | R^{B43} | R^{B3} | R^{B12} | H | L_{A2135} | R^{B43} | R^{B4} | H | R^{A3} |
| L_{A1864} | R^{B43} | R^{B3} | R^{B18} | H | L_{A2136} | R^{B43} | R^{B4} | H | R^{A34} |
| L_{A1865} | R^{B43} | R^{B3} | R^{A3} | H | L_{A2137} | R^{B43} | R^{B5} | H | R^{B1} |
| L_{A1866} | R^{B43} | R^{B3} | R^{A34} | H | L_{A2138} | R^{B43} | R^{B5} | H | R^{B3} |
| L_{A1867} | R^{B43} | R^{B4} | H | H | L_{A2139} | R^{B43} | R^{B5} | H | R^{B4} |
| L_{A1868} | R^{B43} | R^{B4} | R^{B1} | H | L_{A2140} | R^{B43} | R^{B5} | H | R^{B7} |
| L_{A1869} | R^{B43} | R^{B4} | R^{B3} | H | L_{A2141} | R^{B43} | R^{B5} | H | R^{B12} |
| L_{A1870} | R^{B43} | R^{B4} | R^{B4} | H | L_{A2142} | R^{B43} | R^{B5} | H | R^{B18} |
| L_{A1871} | R^{B43} | R^{B4} | R^{B7} | H | L_{A2143} | R^{B43} | R^{B5} | H | R^{A3} |
| L_{A1872} | R^{B43} | R^{B4} | R^{B12} | H | L_{A2144} | R^{B43} | R^{B5} | H | R^{A34} |
| L_{A1873} | R^{B43} | R^{B4} | R^{B18} | H | L_{A2145} | R^{B43} | R^{B6} | H | R^{B1} |
| L_{A1874} | R^{B43} | R^{B4} | R^{A3} | H | L_{A2146} | R^{B43} | R^{B6} | H | R^{B3} |
| L_{A1875} | R^{B43} | R^{B4} | R^{A34} | H | L_{A2147} | R^{B43} | R^{B6} | H | R^{B4} |
| L_{A1876} | R^{B43} | R^{B5} | H | H | L_{A2148} | R^{B43} | R^{B6} | H | R^{B7} |
| L_{A1877} | R^{B43} | R^{B5} | R^{B1} | H | L_{A2149} | R^{B43} | R^{B6} | H | R^{B12} |
| L_{A1878} | R^{B43} | R^{B5} | R^{B3} | H | L_{A2150} | R^{B43} | R^{B6} | H | R^{B18} |
| L_{A1879} | R^{B43} | R^{B5} | R^{B4} | H | L_{A2151} | R^{B43} | R^{B6} | H | R^{A3} |
| L_{A1880} | R^{B43} | R^{B5} | R^{B7} | H | L_{A2152} | R^{B43} | R^{B6} | H | R^{A34} |
| L_{A1881} | R^{B43} | R^{B5} | R^{B12} | H | L_{A2153} | R^{B43} | R^{B7} | H | R^{B1} |
| L_{A1882} | R^{B43} | R^{B5} | R^{B18} | H | L_{A2154} | R^{B43} | R^{B7} | H | R^{B3} |
| L_{A1883} | R^{B43} | R^{B5} | R^{A3} | H | L_{A2155} | R^{B43} | R^{B7} | H | R^{B4} |
| L_{A1884} | R^{B43} | R^{B5} | R^{A34} | H | L_{A2156} | R^{B43} | R^{B7} | H | R^{B7} |
| L_{A1885} | R^{B43} | R^{B6} | H | H | L_{A2157} | R^{B43} | R^{B7} | H | R^{B12} |
| L_{A1886} | R^{B43} | R^{B6} | R^{B1} | H | L_{A2158} | R^{B43} | R^{B7} | H | R^{B18} |
| L_{A1887} | R^{B43} | R^{B6} | R^{B3} | H | L_{A2159} | R^{B43} | R^{B7} | H | R^{A3} |
| L_{A1888} | R^{B43} | R^{B6} | R^{B4} | H | L_{A2160} | R^{B43} | R^{B7} | H | R^{A34} |
| L_{A1889} | R^{B43} | R^{B6} | R^{B7} | H | L_{A2161} | R^{B43} | R^{B12} | H | R^{B1} |
| L_{A1890} | R^{B43} | R^{B6} | R^{B12} | H | L_{A2162} | R^{B43} | R^{B12} | H | R^{B3} |
| L_{A1891} | R^{B43} | R^{B6} | R^{B18} | H | L_{A2163} | R^{B43} | R^{B12} | H | R^{B4} |
| L_{A1892} | R^{B43} | R^{B6} | R^{A3} | H | L_{A2164} | R^{B43} | R^{B12} | H | R^{B7} |
| L_{A1893} | R^{B43} | R^{B6} | R^{A34} | H | L_{A2165} | R^{B43} | R^{B12} | H | R^{B12} |
| L_{A1894} | R^{B43} | R^{B7} | H | H | L_{A2166} | R^{B43} | R^{B12} | H | R^{B18} |
| L_{A1895} | R^{B43} | R^{B7} | R^{B1} | H | L_{A2167} | R^{B43} | R^{B12} | H | R^{A3} |
| L_{A1896} | R^{B43} | R^{B7} | R^{B3} | H | L_{A2168} | R^{B43} | R^{B12} | H | R^{A34} |
| L_{A1897} | R^{B43} | R^{B7} | R^{B4} | H | L_{A2169} | R^{B43} | R^{A34} | H | R^{B1} |
| L_{A1898} | R^{B43} | R^{B7} | R^{B7} | H | L_{A2170} | R^{B43} | R^{A34} | H | R^{B3} |
| L_{A1899} | R^{B43} | R^{B7} | R^{B12} | H | L_{A2171} | R^{B43} | R^{A34} | H | R^{B4} |
| L_{A1900} | R^{B43} | R^{B7} | R^{B18} | H | L_{A2172} | R^{B43} | R^{A34} | H | R^{B7} |
| L_{A1901} | R^{B43} | R^{B7} | R^{A3} | H | L_{A2173} | R^{B43} | R^{A34} | H | R^{B12} |
| L_{A1902} | R^{B43} | R^{B7} | R^{A34} | H | L_{A2174} | R^{B43} | R^{A34} | H | R^{B18} |
| L_{A1903} | R^{B43} | R^{B12} | H | H | L_{A2175} | R^{B43} | R^{A34} | H | R^{A3} |
| L_{A1904} | R^{B43} | R^{B12} | R^{B1} | H | L_{A2176} | R^{B43} | R^{A34} | H | R^{A34} |

L_{A2177} through L_{A2719}, and L_{A2720}, that are based on a structure of Formula Ie, 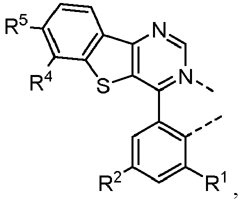 in each of which R¹, R², R⁴, and R⁵ are defined as follows:

| | **R¹** | **R²** | **R⁴** | **R⁵** | | **R¹** | **R³** | **R⁴** | **R⁵** |
|---|---|---|---|---|---|---|---|---|---|
| L_{A2177} | R^{B1} | R^{B3} | H | H | L_{A2449} | R^{B43} | R^{B12} | R^{B3} | H |
| L_{A2178} | R^{B1} | R^{B3} | R^{B1} | H | L_{A2450} | R^{B43} | R^{B12} | R^{B4} | H |
| L_{A2179} | R^{B1} | R^{B3} | R^{B3} | H | L_{A2451} | R^{B43} | R^{B12} | R^{B7} | H |
| L_{A2180} | R^{B1} | R^{B3} | R^{B4} | H | L_{A2452} | R^{B43} | R^{B12} | R^{B12} | H |
| L_{A2181} | R^{B1} | R^{B3} | R^{B7} | H | L_{A2453} | R^{B43} | R^{B12} | R^{B18} | H |
| L_{A2182} | R^{B1} | R^{B3} | R^{B12} | H | L_{A2454} | R^{B43} | R^{B12} | R^{A3} | H |
| L_{A2183} | R^{B1} | R^{B3} | R^{B18} | H | L_{A2455} | R^{B43} | R^{B12} | R^{A34} | H |
| L_{A2184} | R^{B1} | R^{B3} | R^{A3} | H | L_{A2456} | R^{B43} | R^{A34} | H | H |
| L_{A2185} | R^{B1} | R^{B3} | R^{A34} | H | L_{A2457} | R^{B43} | R^{A34} | R^{B1} | H |
| L_{A2186} | R^{B1} | R^{B4} | H | H | L_{A2458} | R^{B43} | R^{A34} | R^{B3} | H |
| L_{A2187} | R^{B1} | R^{B4} | R^{B1} | H | L_{A2459} | R^{B43} | R^{A34} | R^{B4} | H |
| L_{A2188} | R^{B1} | R^{B4} | R^{B3} | H | L_{A2460} | R^{B43} | R^{A34} | R^{B7} | H |
| L_{A2189} | R^{B1} | R^{B4} | R^{B4} | H | L_{A2461} | R^{B43} | R^{A34} | R^{B12} | H |
| L_{A2190} | R^{B1} | R^{B4} | R^{B7} | H | L_{A2462} | R^{B43} | R^{A34} | R^{B18} | H |
| L_{A2191} | R^{B1} | R^{B4} | R^{B12} | H | L_{A2463} | R^{B43} | R^{A34} | R^{A3} | H |
| L_{A2192} | R^{B1} | R^{B4} | R^{B18} | H | L_{A2464} | R^{B43} | R^{A34} | R^{A34} | H |
| L_{A2193} | R^{B1} | R^{B4} | R^{A3} | H | L_{A2465} | R^{B1} | R^{B3} | H | R^{B1} |
| L_{A2194} | R^{B1} | R^{B4} | R^{A34} | H | L_{A2466} | R^{B1} | R^{B3} | H | R^{B3} |
| L_{A2195} | R^{B1} | R^{B5} | H | H | L_{A2467} | R^{B1} | R^{B3} | H | R^{B4} |
| L_{A2196} | R^{B1} | R^{B5} | R^{B1} | H | L_{A2468} | R^{B1} | R^{B3} | H | R^{B7} |
| L_{A2197} | R^{B1} | R^{B5} | R^{B3} | H | L_{A2469} | R^{B1} | R^{B3} | H | R^{B12} |
| L_{A2198} | R^{B1} | R^{B5} | R^{B4} | H | L_{A2470} | R^{B1} | R^{B3} | H | R^{B18} |
| L_{A2199} | R^{B1} | R^{B5} | R^{B7} | H | L_{A2471} | R^{B1} | R^{B3} | H | R^{A3} |
| L_{A2200} | R^{B1} | R^{B5} | R^{B12} | H | L_{A2472} | R^{B1} | R^{B3} | H | R^{A34} |
| L_{A2201} | R^{B1} | R^{B5} | R^{B18} | H | L_{A2473} | R^{B1} | R^{B4} | H | R^{B1} |
| L_{A2202} | R^{B1} | R^{B5} | R^{A3} | H | L_{A2474} | R^{B1} | R^{B4} | H | R^{B3} |
| L_{A2203} | R^{B1} | R^{B5} | R^{A34} | H | L_{A2475} | R^{B1} | R^{B4} | H | R^{B4} |
| L_{A2204} | R^{B1} | R^{B6} | H | H | L_{A2476} | R^{B1} | R^{B4} | H | R^{B7} |
| L_{A2205} | R^{B1} | R^{B6} | R^{B1} | H | L_{A2477} | R^{B1} | R^{B4} | H | R^{B12} |
| L_{A2206} | R^{B1} | R^{B6} | R^{B3} | H | L_{A2478} | R^{B1} | R^{B4} | H | R^{B18} |
| L_{A2207} | R^{B1} | R^{B6} | R^{B4} | H | L_{A2479} | R^{B1} | R^{B4} | H | R^{A3} |
| L_{A2208} | R^{B1} | R^{B6} | R^{B7} | H | L_{A2480} | R^{B1} | R^{B4} | H | R^{A34} |
| L_{A2209} | R^{B1} | R^{B6} | R^{B12} | H | L_{A2481} | R^{B1} | R^{B5} | H | R^{B1} |
| L_{A2210} | R^{B1} | R^{B6} | R^{B18} | H | L_{A2482} | R^{B1} | R^{B5} | H | R^{B3} |
| L_{A2211} | R^{B1} | R^{B6} | R^{A3} | H | L_{A2483} | R^{B1} | R^{B5} | H | R^{B4} |
| L_{A2212} | R^{B1} | R^{B6} | R^{A34} | H | L_{A2484} | R^{B1} | R^{B5} | H | R^{B7} |
| L_{A2213} | R^{B1} | R^{B7} | H | H | L_{A2485} | R^{B1} | R^{B5} | H | R^{B12} |
| L_{A2214} | R^{B1} | R^{B7} | R^{B1} | H | L_{A2486} | R^{B1} | R^{B5} | H | R^{B18} |
| L_{A2215} | R^{B1} | R^{B7} | R^{B3} | H | L_{A2487} | R^{B1} | R^{B5} | H | R^{A3} |
| L_{A2216} | R^{B1} | R^{B7} | R^{B4} | H | L_{A2488} | R^{B1} | R^{B5} | H | R^{A34} |
| L_{A2217} | R^{B1} | R^{B7} | R^{B7} | H | L_{A2489} | R^{B1} | R^{B6} | H | R^{B1} |
| L_{A2218} | R^{B1} | R^{B7} | R^{B12} | H | L_{A2490} | R^{B1} | R^{B6} | H | R^{B3} |
| L_{A2219} | R^{B1} | R^{B7} | R^{B18} | H | L_{A2491} | R^{B1} | R^{B6} | H | R^{B4} |
| L_{A2220} | R^{B1} | R^{B7} | R^{A3} | H | L_{A2492} | R^{B1} | R^{B6} | H | R^{B7} |
| L_{A2221} | R^{B1} | R^{B7} | R^{A34} | H | L_{A2493} | R^{B1} | R^{B6} | H | R^{B12} |
| L_{A2222} | R^{B1} | R^{B12} | H | H | L_{A2494} | R^{B1} | R^{B6} | H | R^{B18} |
| L_{A2223} | R^{B1} | R^{B12} | R^{B1} | H | L_{A2495} | R^{B1} | R^{B6} | H | R^{A3} |
| L_{A2224} | R^{B1} | R^{B12} | R^{B3} | H | L_{A2496} | R^{B1} | R^{B6} | H | R^{A34} |
| L_{A2225} | R^{B1} | R^{B12} | R^{B4} | H | L_{A2497} | R^{B1} | R^{B7} | H | R^{B1} |
| L_{A2226} | R^{B1} | R^{B12} | R^{B7} | H | L_{A2498} | R^{B1} | R^{B7} | H | R^{B3} |
| L_{A2227} | R^{B1} | R^{B12} | R^{B12} | H | L_{A2499} | R^{B1} | R^{B7} | H | R^{B4} |
| L_{A2228} | R^{B1} | R^{B12} | R^{B18} | H | L_{A2500} | R^{B1} | R^{B7} | H | R^{B7} |
| L_{A2229} | R^{B1} | R^{B12} | R^{A3} | H | L_{A2501} | R^{B1} | R^{B7} | H | R^{B12} |
| L_{A2230} | R^{B1} | R^{B12} | R^{A34} | H | L_{A2502} | R^{B1} | R^{B7} | H | R^{B18} |
| L_{A2231} | R^{B1} | R^{A34} | H | H | L_{A2503} | R^{B1} | R^{B7} | H | R^{A3} |
| L_{A2232} | R^{B1} | R^{A34} | R^{B1} | H | L_{A2504} | R^{B1} | R^{B7} | H | R^{A34} |
| L_{A2233} | R^{B1} | R^{A34} | R^{B3} | H | L_{A2505} | R^{B1} | R^{B12} | H | R^{B1} |
| L_{A2234} | R^{B1} | R^{A34} | R^{B4} | H | L_{A2506} | R^{B1} | R^{B12} | H | R^{B3} |
| L_{A2235} | R^{B1} | R^{A34} | R^{B7} | H | L_{A2507} | R^{B1} | R^{B12} | H | R^{B4} |
| L_{A2236} | R^{B1} | R^{A34} | R^{B12} | H | L_{A2508} | R^{B1} | R^{B12} | H | R^{B7} |
| L_{A2237} | R^{B1} | R^{A34} | R^{B18} | H | L_{A2509} | R^{B1} | R^{B12} | H | R^{B12} |
| L_{A2238} | R^{B1} | R^{A34} | R^{A3} | H | L_{A2510} | R^{B1} | R^{B12} | H | R^{B18} |
| L_{A2239} | R^{B1} | R^{A34} | R^{A34} | H | L_{A2511} | R^{B1} | R^{B12} | H | R^{A3} |
| L_{A2240} | R^{B3} | R^{B4} | H | H | L_{A2512} | R^{B1} | R^{B12} | H | R^{A34} |
| L_{A2241} | R^{B3} | R^{B4} | R^{B1} | H | L_{A2513} | R^{B1} | R^{A34} | H | R^{B1} |
| L_{A2242} | R^{B3} | R^{B4} | R^{B3} | H | L_{A2514} | R^{B1} | R^{A34} | H | R^{B3} |
| L_{A2243} | R^{B3} | R^{B4} | R^{B4} | H | L_{A2515} | R^{B1} | R^{A34} | H | R^{B4} |
| L_{A2244} | R^{B3} | R^{B4} | R^{B7} | H | L_{A2516} | R^{B1} | R^{A34} | H | R^{B7} |
| L_{A2245} | R^{B3} | R^{B4} | R^{B12} | H | L_{A2517} | R^{B1} | R^{A34} | H | R^{B12} |
| L_{A2246} | R^{B3} | R^{B4} | R^{B18} | H | L_{A2518} | R^{B1} | R^{A34} | H | R^{B18} |
| L_{A2247} | R^{B3} | R^{B4} | R^{A3} | H | L_{A2519} | R^{B1} | R^{A34} | H | R^{A3} |
| L_{A2248} | R^{B3} | R^{B4} | R^{A34} | H | L_{A2520} | R^{B1} | R^{A34} | H | R^{A34} |
| L_{A2249} | R^{B3} | R^{B5} | H | H | L_{A2521} | R^{B3} | R^{B4} | H | R^{B1} |
| L_{A2250} | R^{B3} | R^{B5} | R^{B1} | H | L_{A2522} | R^{B3} | R^{B4} | H | R^{B3} |
| L_{A2251} | R^{B3} | R^{B5} | R^{B3} | H | L_{A2523} | R^{B3} | R^{B4} | H | R^{B4} |
| L_{A2252} | R^{B3} | R^{B5} | R^{B4} | H | L_{A2524} | R^{B3} | R^{B4} | H | R^{B7} |
| L_{A2253} | R^{B3} | R^{B5} | R^{B7} | H | L_{A2525} | R^{B3} | R^{B4} | H | R^{B12} |
| L_{A2254} | R^{B3} | R^{B5} | R^{B12} | H | L_{A2526} | R^{B3} | R^{B4} | H | R^{B18} |
| L_{A2255} | R^{B3} | R^{B5} | R^{B18} | H | L_{A2527} | R^{B3} | R^{B4} | H | R^{A3} |
| L_{A2256} | R^{B3} | R^{B5} | R^{A3} | H | L_{A2528} | R^{B3} | R^{B4} | H | R^{A34} |
| L_{A2257} | R^{B3} | R^{B5} | R^{A34} | H | L_{A2529} | R^{B3} | R^{B5} | H | R^{B1} |
| L_{A2258} | R^{B3} | R^{B6} | H | H | L_{A2530} | R^{B3} | R^{B5} | H | R^{B3} |
| L_{A2259} | R^{B3} | R^{B6} | R^{B1} | H | L_{A2531} | R^{B3} | R^{B5} | H | R^{B4} |
| L_{A2260} | R^{B3} | R^{B6} | R^{B3} | H | L_{A2532} | R^{B3} | R^{B5} | H | R^{B7} |
| L_{A2261} | R^{B3} | R^{B6} | R^{B4} | H | L_{A2533} | R^{B3} | R^{B5} | H | R^{B12} |
| L_{A2262} | R^{B3} | R^{B6} | R^{B7} | H | L_{A2534} | R^{B3} | R^{B5} | H | R^{B18} |
| L_{A2263} | R^{B3} | R^{B6} | R^{B12} | H | L_{A2535} | R^{B3} | R^{B5} | H | R^{A3} |
| L_{A2264} | R^{B3} | R^{B6} | R^{B18} | H | L_{A2536} | R^{B3} | R^{B5} | H | R^{A34} |
| L_{A2265} | R^{B3} | R^{B6} | R^{A3} | H | L_{A2537} | R^{B3} | R^{B6} | H | R^{B1} |
| L_{A2266} | R^{B3} | R^{B6} | R^{A34} | H | L_{A2538} | R^{B3} | R^{B6} | H | R^{B3} |
| L_{A2267} | R^{B3} | R^{B7} | H | H | L_{A2539} | R^{B3} | R^{B6} | H | R^{B4} |
| L_{A2268} | R^{B3} | R^{B7} | R^{B1} | H | L_{A2540} | R^{B3} | R^{B6} | H | R^{B7} |
| L_{A2269} | R^{B3} | R^{B7} | R^{B3} | H | L_{A2541} | R^{B3} | R^{B6} | H | R^{B12} |
| L_{A2270} | R^{B3} | R^{B7} | R^{B4} | H | L_{A2542} | R^{B3} | R^{B6} | H | R^{B18} |
| L_{A2271} | R^{B3} | R^{B7} | R^{B7} | H | L_{A2543} | R^{B3} | R^{B6} | H | R^{A3} |
| L_{A2272} | R^{B3} | R^{B7} | R^{B12} | H | L_{A2544} | R^{B3} | R^{B6} | H | R^{A34} |
| L_{A2273} | R^{B3} | R^{B7} | R^{B18} | H | L_{A2545} | R^{B3} | R^{B7} | H | R^{B1} |
| L_{A2274} | R^{B3} | R^{B7} | R^{A3} | H | L_{A2546} | R^{B3} | R^{B7} | H | R^{B3} |
| L_{A2275} | R^{B3} | R^{B7} | R^{A34} | H | L_{A2547} | R^{B3} | R^{B7} | H | R^{B4} |
| L_{A2276} | R^{B3} | R^{B12} | H | H | L_{A2548} | R^{B3} | R^{B7} | H | R^{B7} |
| L_{A2277} | R^{B3} | R^{B12} | R^{B1} | H | L_{A2549} | R^{B3} | R^{B7} | H | R^{B12} |
| L_{A2278} | R^{B3} | R^{B12} | R^{B3} | H | L_{A2550} | R^{B3} | R^{B7} | H | R^{B18} |
| L_{A2279} | R^{B3} | R^{B12} | R^{B4} | H | L_{A2551} | R^{B3} | R^{B7} | H | R^{A3} |
| L_{A2280} | R^{B3} | R^{B12} | R^{B7} | H | L_{A2552} | R^{B3} | R^{B7} | H | R^{A34} |
| L_{A2281} | R^{B3} | R^{B12} | R^{B12} | H | L_{A2553} | R^{B3} | R^{B12} | H | R^{B1} |
| L_{A2282} | R^{B3} | R^{B12} | R^{B18} | H | L_{A2554} | R^{B3} | R^{B12} | H | R^{B3} |
| L_{A2283} | R^{B3} | R^{B12} | R^{A3} | H | L_{A2555} | R^{B3} | R^{B12} | H | R^{B4} |
| L_{A2284} | R^{B3} | R^{B12} | R^{A34} | H | L_{A2556} | R^{B3} | R^{B12} | H | R^{B7} |
| L_{A2285} | R^{B3} | R^{A34} | H | H | L_{A2557} | R^{B3} | R^{B12} | H | R^{B12} |
| L_{A2286} | R^{B3} | R^{A34} | R^{B1} | H | L_{A2558} | R^{B3} | R^{B12} | H | R^{B18} |
| L_{A2287} | R^{B3} | R^{A34} | R^{B3} | H | L_{A2559} | R^{B3} | R^{B12} | H | R^{A3} |
| L_{A2288} | R^{B3} | R^{A34} | R^{B4} | H | L_{A2560} | R^{B3} | R^{B12} | H | R^{A34} |
| L_{A2289} | R^{B3} | R^{A34} | R^{B7} | H | L_{A2561} | R^{B3} | R^{A34} | H | R^{B1} |
| L_{A2290} | R^{B3} | R^{A34} | R^{B12} | H | L_{A2562} | R^{B3} | R^{A34} | H | R^{B3} |
| L_{A2291} | R^{B3} | R^{A34} | R^{B18} | H | L_{A2563} | R^{B3} | R^{A34} | H | R^{B4} |
| L_{A2292} | R^{B3} | R^{A34} | R^{A3} | H | L_{A2564} | R^{B3} | R^{A34} | H | R^{B7} |
| L_{A2293} | R^{B3} | R^{A34} | R^{A34} | H | L_{A2565} | R^{B3} | R^{A34} | H | R^{B12} |
| L_{A2294} | R^{B4} | R^{B3} | H | H | L_{A2566} | R^{B3} | R^{A34} | H | R^{B18} |
| L_{A2295} | R^{B4} | R^{B3} | R^{B1} | H | L_{A2567} | R^{B3} | R^{A34} | H | R^{A3} |
| L_{A2296} | R^{B4} | R^{B3} | R^{B3} | H | L_{A2568} | R^{B3} | R^{A34} | H | R^{A34} |
| L_{A2297} | R^{B4} | R^{B3} | R^{B4} | H | L_{A2569} | R^{B4} | R^{B3} | H | R^{B1} |
| L_{A2298} | R^{B4} | R^{B3} | R^{B7} | H | L_{A2570} | R^{B4} | R^{B3} | H | R^{B3} |
| L_{A2299} | R^{B4} | R^{B3} | R^{B12} | H | L_{A2571} | R^{B4} | R^{B3} | H | R^{B4} |
| L_{A2300} | R^{B4} | R^{B3} | R^{B18} | H | L_{A2572} | R^{B4} | R^{B3} | H | R^{B7} |
| L_{A2301} | R^{B4} | R^{B3} | R^{A3} | H | L_{A2573} | R^{B4} | R^{B3} | H | R^{B12} |
| L_{A2302} | R^{B4} | R^{B3} | R^{A34} | H | L_{A2574} | R^{B4} | R^{B3} | H | R^{B18} |
| L_{A2303} | R^{B4} | R^{B5} | H | H | L_{A2575} | R^{B4} | R^{B3} | H | R^{A3} |
| L_{A2304} | R^{B4} | R^{B5} | R^{B1} | H | L_{A2576} | R^{B4} | R^{B3} | H | R^{A34} |
| L_{A2305} | R^{B4} | R^{B5} | R^{B3} | H | L_{A2577} | R^{B4} | R^{B5} | H | R^{B1} |
| L_{A2306} | R^{B4} | R^{B5} | R^{B4} | H | L_{A2578} | R^{B4} | R^{B5} | H | R^{B3} |
| L_{A2307} | R^{B4} | R^{B5} | R^{B7} | H | L_{A2579} | R^{B4} | R^{B5} | H | R^{B4} |
| L_{A2308} | R^{B4} | R^{B5} | R^{B12} | H | L_{A2580} | R^{B4} | R^{B5} | H | R^{B7} |
| L_{A2309} | R^{B4} | R^{B5} | R^{B18} | H | L_{A2581} | R^{B4} | R^{B5} | H | R^{B12} |
| L_{A2310} | R^{B4} | R^{B5} | R^{A3} | H | L_{A2582} | R^{B4} | R^{B5} | H | R^{B18} |
| L_{A2311} | R^{B4} | R^{B5} | R^{A34} | H | L_{A2583} | R^{B4} | R^{B5} | H | R^{A3} |
| L_{A2312} | R^{B4} | R^{B6} | H | H | L_{A2584} | R^{B4} | R^{B5} | H | R^{A34} |
| L_{A2313} | R^{B4} | R^{B6} | R^{B1} | H | L_{A2585} | R^{B4} | R^{B6} | H | R^{B1} |
| L_{A2314} | R^{B4} | R^{B6} | R^{B3} | H | L_{A2586} | R^{B4} | R^{B6} | H | R^{B3} |
| L_{A2315} | R^{B4} | R^{B6} | R^{B4} | H | L_{A2587} | R^{B4} | R^{B6} | H | R^{B4} |
| L_{A2316} | R^{B4} | R^{B6} | R^{B7} | H | L_{A2588} | R^{B4} | R^{B6} | H | R^{B7} |
| L_{A2317} | R^{B4} | R^{B6} | R^{B12} | H | L_{A2589} | R^{B4} | R^{B6} | H | R^{B12} |
| L_{A2318} | R^{B4} | R^{B6} | R^{B18} | H | L_{A2590} | R^{B4} | R^{B6} | H | R^{B18} |
| L_{A2319} | R^{B4} | R^{B6} | RA3 | H | L_{A2591} | R^{B4} | R^{B6} | H | R^{A3} |
| L_{A2320} | R^{B4} | R^{B6} | R^{A34} | H | L_{A2592} | R^{B4} | R^{B6} | H | R^{A34} |
| L_{A2321} | R^{B4} | R^{B7} | H | H | L_{A2593} | R^{B4} | R^{B7} | H | R^{B1} |
| L_{A2322} | R^{B4} | R^{B7} | R^{B1} | H | L_{A2594} | R^{B4} | R^{B7} | H | R^{B3} |
| L_{A2323} | R^{B4} | R^{B7} | R^{B3} | H | L_{A2595} | R^{B4} | R^{B7} | H | R^{B4} |
| L_{A2324} | R^{B4} | R^{B7} | R^{B4} | H | L_{A2596} | R^{B4} | R^{B7} | H | R^{B7} |
| L_{A2325} | R^{B4} | R^{B7} | R^{B7} | H | L_{A2597} | R^{B4} | R^{B7} | H | R^{B12} |
| L_{A2326} | R^{B4} | R^{B7} | R^{B12} | H | L_{A2598} | R^{B4} | R^{B7} | H | R^{B18} |
| L_{A2327} | R^{B4} | R^{B7} | R^{B18} | H | L_{A2599} | R^{B4} | R^{B7} | H | R^{A3} |
| L_{A2328} | R^{B4} | R^{B7} | R^{A3} | H | L_{A2600} | R^{B4} | R^{B7} | H | R^{A34} |
| L_{A2329} | R^{B4} | R^{B7} | R^{A34} | H | L_{A2601} | R^{B4} | R^{B12} | H | R^{B1} |
| L_{A2330} | R^{B4} | R^{B12} | H | H | L_{A2602} | R^{B4} | R^{B12} | H | R^{B3} |
| L_{A2331} | R^{B4} | R^{B12} | R^{B1} | H | L_{A2603} | R^{B4} | R^{B12} | H | R^{B4} |
| L_{A2332} | R^{B4} | R^{B12} | R^{B3} | H | L_{A2604} | R^{B4} | R^{B12} | H | R^{B7} |
| L_{A2333} | R^{B4} | R^{B12} | R^{B4} | H | L_{A2605} | R^{B4} | R^{B12} | H | R^{B12} |
| L_{A2334} | R^{B4} | R^{B12} | R^{B7} | H | L_{A2606} | R^{B4} | R^{B12} | H | R^{B18} |
| L_{A2335} | R^{B4} | R^{B12} | R^{B12} | H | L_{A2607} | R^{B4} | R^{B12} | H | R^{A3} |
| L_{A2336} | R^{B4} | R^{B12} | R^{B18} | H | L_{A2608} | R^{B4} | R^{B12} | H | R^{A34} |
| L_{A2337} | R^{B4} | R^{B12} | R^{A3} | H | L_{A2609} | R^{B4} | R^{A34} | H | R^{B1} |
| L_{A2338} | R^{B4} | R^{B12} | R^{A34} | H | L_{A2610} | R^{B4} | R^{A34} | H | R^{B3} |
| L_{A2339} | R^{B4} | R^{A34} | H | H | L_{A2611} | R^{B4} | R^{A34} | H | R^{B4} |
| L_{A2340} | R^{B4} | R^{A34} | R^{B1} | H | L_{A2612} | R^{B4} | R^{A34} | H | R^{B7} |
| L_{A2341} | R^{B4} | R^{A34} | R^{B3} | H | L_{A2613} | R^{B4} | R^{A34} | H | R^{B12} |
| L_{A2342} | R^{B4} | R^{A34} | R^{B4} | H | L_{A2614} | R^{B4} | R^{A34} | H | R^{B18} |
| L_{A2343} | R^{B4} | R^{A34} | R^{B7} | H | L_{A2615} | R^{B4} | R^{A34} | H | R^{A3} |
| L_{A2344} | R^{B4} | R^{A34} | R^{B12} | H | L_{A2616} | R^{B4} | R^{A34} | H | R^{A34} |
| L_{A2345} | R^{B4} | R^{A34} | R^{B18} | H | L_{A2617} | R^{B7} | R^{B3} | H | R^{B1} |
| L_{A2346} | R^{B4} | R^{A34} | R^{A3} | H | L_{A2618} | R^{B7} | R^{B3} | H | R^{B3} |
| L_{A2347} | R^{B4} | R^{A34} | R^{A34} | H | L_{A2619} | R^{B7} | R^{B3} | H | R^{B4} |
| L_{A2348} | R^{B7} | R^{B3} | H | H | L_{A2620} | R^{B7} | R^{B3} | H | R^{B7} |
| L_{A2349} | R^{B7} | R^{B3} | R^{B1} | H | L_{A2621} | R^{B7} | R^{B3} | H | R^{B12} |
| L_{A2350} | R^{B7} | R^{B3} | R^{B3} | H | L_{A2622} | R^{B7} | R^{B3} | H | R^{B18} |
| L_{A2351} | R^{B7} | R^{B3} | R^{B4} | H | L_{A2623} | R^{B7} | R^{B3} | H | R^{A3} |
| L_{A2352} | R^{B7} | R^{B3} | R^{B7} | H | L_{A2624} | R^{B7} | R^{B3} | H | R^{A34} |
| L_{A2353} | R^{B7} | R^{B3} | R^{B12} | H | L_{A2625} | R^{B7} | R^{B4} | H | R^{B1} |
| L_{A2354} | R^{B7} | R^{B3} | R^{B18} | H | L_{A2626} | R^{B7} | R^{B4} | H | R^{B3} |
| L_{A2355} | R^{B7} | R^{B3} | R^{A3} | H | L_{A2627} | R^{B7} | R^{B4} | H | R^{B4} |
| L_{A2356} | R^{B7} | R^{B3} | R^{A34} | H | L_{A2628} | R^{B7} | R^{B4} | H | R^{B7} |
| L_{A2357} | R^{B7} | R^{B4} | H | H | L_{A2629} | R^{B7} | R^{B4} | H | R^{B12} |
| L_{A2358} | R^{B7} | R^{B4} | R^{B1} | H | L_{A2630} | R^{B7} | R^{B4} | H | R^{B18} |
| L_{A2359} | R^{B7} | R^{B4} | R^{B3} | H | L_{A2631} | R^{B7} | R^{B4} | H | R^{A3} |
| L_{A2360} | R^{B7} | R^{B4} | R^{B4} | H | L_{A2632} | R^{B7} | R^{B4} | H | R^{A34} |
| L_{A2361} | R^{B7} | R^{B4} | R^{B7} | H | L_{A2633} | R^{B7} | R^{B5} | H | R^{B1} |
| L_{A2362} | R^{B7} | R^{B4} | R^{B12} | H | L_{A2634} | R^{B7} | R^{B5} | H | R^{B3} |
| L_{A2363} | R^{B7} | R^{B4} | R^{B18} | H | L_{A2635} | R^{B7} | R^{B5} | H | R^{B4} |
| L_{A2364} | R^{B7} | R^{B4} | R^{A3} | H | L_{A2636} | R^{B7} | R^{B5} | H | R^{B7} |
| L_{A2365} | R^{B7} | R^{B4} | R^{A34} | H | L_{A2637} | R^{B7} | R^{B5} | H | R^{B12} |
| L_{A2366} | R^{B7} | R^{B5} | H | H | L_{A2638} | R^{B7} | R^{B5} | H | R^{B18} |
| L_{A2367} | R^{B7} | R^{B5} | R^{B1} | H | L_{A2639} | R^{B7} | R^{B5} | H | R^{A3} |
| L_{A2368} | R^{B7} | R^{B5} | R^{B3} | H | L_{A2640} | R^{B7} | R^{B5} | H | R^{A34} |
| L_{A2369} | R^{B7} | R^{B5} | R^{B4} | H | L_{A2641} | R^{B7} | R^{B6} | H | R^{B1} |
| L_{A2370} | R^{B7} | R^{B5} | R^{B7} | H | L_{A2642} | R^{B7} | R^{B6} | H | R^{B3} |
| L_{A2371} | R^{B7} | R^{B5} | R^{B12} | H | L_{A2643} | R^{B7} | R^{B6} | H | R^{B4} |
| L_{A2372} | R^{B7} | R^{B5} | R^{B18} | H | L_{A2644} | R^{B7} | R^{B6} | H | R^{B7} |
| L_{A2373} | R^{B7} | R^{B5} | R^{A3} | H | L_{A2645} | R^{B7} | R^{B6} | H | R^{B12} |
| L_{A2374} | R^{B7} | R^{B5} | R^{A34} | H | L_{A2646} | R^{B7} | R^{B6} | H | R^{B18} |
| L_{A2375} | R^{B7} | R^{B6} | H | H | L_{A2647} | R^{B7} | R^{B6} | H | R^{A3} |
| L_{A2376} | R^{B7} | R^{B6} | R^{B1} | H | L_{A2648} | R^{B7} | R^{B6} | H | R^{A34} |
| L_{A2377} | R^{B7} | R^{B6} | R^{B3} | H | L_{A2649} | R^{B7} | R^{B12} | H | R^{B1} |
| L_{A2378} | R^{B7} | R^{B6} | R^{B4} | H | L_{A2650} | R^{B7} | R^{B12} | H | R^{B3} |
| L_{A2379} | R^{B7} | R^{B6} | R^{B7} | H | L_{A2651} | R^{B7} | R^{B12} | H | R^{B4} |
| L_{A2380} | R^{B7} | R^{B6} | R^{B12} | H | L_{A2652} | R^{B7} | R^{B12} | H | R^{B7} |
| L_{A2381} | R^{B7} | R^{B6} | R^{B18} | H | L_{A2653} | R^{B7} | R^{B12} | H | R^{B12} |
| L_{A2382} | R^{B7} | R^{B6} | R^{A3} | H | L_{A2654} | R^{B7} | R^{B12} | H | R^{B18} |
| L_{A2383} | R^{B7} | R^{B6} | R^{A34} | H | L_{A2655} | R^{B7} | R^{B12} | H | R^{A3} |
| L_{A2384} | R^{B7} | R^{B12} | H | H | L_{A2656} | R^{B7} | R^{B12} | H | R^{A34} |
| L_{A2385} | R^{B7} | R^{B12} | R^{B1} | H | L_{A2657} | R^{B7} | R^{A34} | H | R^{B1} |
| L_{A2386} | R^{B7} | R^{B12} | R^{B3} | H | L_{A2658} | R^{B7} | R^{A34} | H | R^{B3} |
| L_{A2387} | R^{B7} | R^{B12} | R^{B4} | H | L_{A2659} | R^{B7} | R^{A34} | H | R^{B4} |
| L_{A2388} | R^{B7} | R^{B12} | R^{B7} | H | L_{A2660} | R^{B7} | R^{A34} | H | R^{B7} |
| L_{A2389} | R^{B7} | R^{B12} | R^{B12} | H | L_{A2661} | R^{B7} | R^{A34} | H | R^{B12} |
| L_{A2390} | R^{B7} | R^{B12} | R^{B18} | H | L_{A2662} | R^{B7} | R^{A34} | H | R^{B18} |
| L_{A2391} | R^{B7} | R^{B12} | R^{A3} | H | L_{A2663} | R^{B7} | R^{A34} | H | R^{A3} |
| L_{A2392} | R^{B7} | R^{B12} | R^{A34} | H | L_{A2664} | R^{B7} | R^{A34} | H | R^{A34} |
| L_{A2393} | R^{B7} | R^{A34} | H | H | L_{A2665} | R^{B43} | R^{B3} | H | R^{B1} |
| L_{A2394} | R^{B7} | R^{A34} | R^{B1} | H | L_{A2666} | R^{B43} | R^{B3} | H | R^{B3} |
| L_{A2395} | R^{B7} | R^{A34} | R^{B3} | H | L_{A2667} | R^{B43} | R^{B3} | H | R^{B4} |
| L_{A2396} | R^{B7} | R^{A34} | R^{B4} | H | L_{A2668} | R^{B43} | R^{B3} | H | R^{B7} |
| L_{A2397} | R^{B7} | R^{A34} | R^{B7} | H | L_{A2669} | R^{B43} | R^{B3} | H | R^{B12} |
| L_{A2398} | R^{B7} | R^{A34} | R^{B12} | H | L_{A2670} | R^{B43} | R^{B3} | H | R^{B18} |
| L_{A2399} | R^{B7} | R^{A34} | R^{B18} | H | L_{A2671} | R^{B43} | R^{B3} | H | R^{A3} |
| L_{A2400} | R^{B7} | R^{A34} | R^{A3} | H | L_{A2672} | R^{B43} | R^{B3} | H | R^{A34} |
| L_{A2401} | R^{B7} | R^{A34} | R^{A34} | H | L_{A2673} | R^{B43} | R^{B4} | H | R^{B1} |
| L_{A2402} | R^{B43} | R^{B3} | H | H | L_{A2674} | R^{B43} | R^{B4} | H | R^{B3} |
| L_{A2403} | R^{B43} | R^{B3} | R^{B1} | H | L_{A2675} | R^{B43} | R^{B4} | H | R^{B4} |
| L_{A2404} | R^{B43} | R^{B3} | R^{B3} | H | L_{A2676} | R^{B43} | R^{B4} | H | R^{B7} |
| L_{A2405} | R^{B43} | R^{B3} | R^{B4} | H | L_{A2677} | R^{B43} | R^{B4} | H | R^{B12} |
| L_{A2406} | R^{B43} | R^{B3} | R^{B7} | H | L_{A2678} | R^{B43} | R^{B4} | H | R^{B18} |
| L_{A2407} | R^{B43} | R^{B3} | R^{B12} | H | L_{A2679} | R^{B43} | R^{B4} | H | R^{A3} |
| L_{A2408} | R^{B43} | R^{B3} | R^{B18} | H | L_{A2680} | R^{B43} | R^{B4} | H | R^{A34} |
| L_{A2409} | R^{B43} | R^{B3} | R^{A3} | H | L_{A2681} | R^{B43} | R^{B5} | H | R^{B1} |
| L_{A2410} | R^{B43} | R^{B3} | R^{A34} | H | L_{A2682} | R^{B43} | R^{B5} | H | R^{B3} |
| L_{A2411} | R^{B43} | R^{B4} | H | H | L_{A2683} | R^{B43} | R^{B5} | H | R^{B4} |
| L_{A2412} | R^{B43} | R^{B4} | R^{B1} | H | L_{A2684} | R^{B43} | R^{B5} | H | R^{B7} |
| L_{A2413} | R^{B43} | R^{B4} | R^{B3} | H | L_{A2685} | R^{B43} | R^{B5} | H | R^{B12} |
| L_{A2414} | R^{B43} | R^{B4} | R^{B4} | H | L_{A2686} | R^{B43} | R^{B5} | H | R^{B18} |
| L_{A2415} | R^{B43} | R^{B4} | R^{B7} | H | L_{A2687} | R^{B43} | R^{B5} | H | R^{A3} |
| L_{A2416} | R^{B43} | R^{B4} | R^{B12} | H | L_{A2688} | R^{B43} | R^{B5} | H | R^{A34} |
| L_{A2417} | R^{B43} | R^{B4} | R^{B18} | H | L_{A2689} | R^{B43} | R^{B6} | H | R^{B1} |
| L_{A2418} | R^{B43} | R^{B4} | R^{A3} | H | L_{A2690} | R^{B43} | R^{B6} | H | R^{B3} |
| L_{A2419} | R^{B43} | R^{B4} | R^{A34} | H | L_{A2691} | R^{B43} | R^{B6} | H | R^{B4} |
| L_{A2420} | R^{B43} | R^{B5} | H | H | L_{A2692} | R^{B43} | R^{B6} | H | R^{B7} |
| L_{A2421} | R^{B43} | R^{B5} | R^{B1} | H | L_{A2693} | R^{B43} | R^{B6} | H | R^{B12} |
| L_{A2422} | R^{B43} | R^{B5} | R^{B3} | H | L_{A2694} | R^{B43} | R^{B6} | H | R^{B18} |
| L_{A2423} | R^{B43} | R^{B5} | R^{B4} | H | L_{A2695} | R^{B43} | R^{B6} | H | R^{A3} |
| L_{A2424} | R^{B43} | R^{B5} | R^{B7} | H | L_{A2696} | R^{B43} | R^{B6} | H | R^{A34} |
| L_{A2425} | R^{B43} | R^{B5} | R^{B12} | H | L_{A2697} | R^{B43} | R^{B7} | H | R^{B1} |
| L_{A2426} | R^{B43} | R^{B5} | R^{B18} | H | L_{A2698} | R^{B43} | R^{B7} | H | R^{B3} |
| L_{A2427} | R^{B43} | R^{B5} | R^{A3} | H | L_{A2699} | R^{B43} | R^{B7} | H | R^{B4} |
| L_{A2428} | R^{B43} | R^{B5} | R^{A34} | H | L_{A2700} | R^{B43} | R^{B7} | H | R^{B7} |
| L_{A2429} | R^{B43} | R^{B6} | H | H | L_{A2701} | R^{B43} | R^{B7} | H | R^{B12} |
| L_{A2430} | R^{B43} | R^{B6} | R^{B1} | H | L_{A2702} | R^{B43} | R^{B7} | H | R^{B18} |
| L_{A2431} | R^{B43} | R^{B6} | R^{B3} | H | L_{A2703} | R^{B43} | R^{B7} | H | R^{A3} |
| L_{A2432} | R^{B43} | R^{B6} | R^{B4} | H | L_{A2704} | R^{B43} | R^{B7} | H | R^{A34} |
| L_{A2433} | R^{B43} | R^{B6} | R^{B7} | H | L_{A2705} | R^{B43} | R^{B12} | H | R^{B1} |
| L_{A2434} | R^{B43} | R^{B6} | R^{B12} | H | L_{A2706} | R^{B43} | R^{B12} | H | R^{B3} |
| L_{A2435} | R^{B43} | R^{B6} | R^{B18} | H | L_{A2707} | R^{B43} | R^{B12} | H | R^{B4} |
| L_{A2436} | R^{B43} | R^{B6} | R^{A3} | H | L_{A2708} | R^{B43} | R^{B12} | H | R^{B7} |
| L_{A2437} | R^{B43} | R^{B6} | R^{A34} | H | L_{A2709} | R^{B43} | R^{B12} | H | R^{B12} |
| L_{A2438} | R^{B43} | R^{B7} | H | H | L_{A2710} | R^{B43} | R^{B12} | H | R^{B18} |
| L_{A2439} | R^{B43} | R^{B7} | R^{B1} | H | L_{A2711} | R^{B43} | R^{B12} | H | R^{A3} |
| L_{A2440} | R^{B43} | R^{B7} | R^{B3} | H | L_{A2712} | R^{B43} | R^{B12} | H | R^{A34} |
| L_{A2441} | R^{B43} | R^{B7} | R^{B4} | H | L_{A2713} | R^{B43} | R^{A34} | H | R^{B1} |
| L_{A2442} | R^{B43} | R^{B7} | R^{B7} | H | L_{A2714} | R^{B43} | R^{A34} | H | R^{B3} |
| L_{A2443} | R^{B43} | R^{B7} | R^{B12} | H | L_{A2715} | R^{B43} | R^{A34} | H | R^{B4} |
| L_{A2444} | R^{B43} | R^{B7} | R^{B18} | H | L_{A2716} | R^{B43} | R^{A34} | H | R^{B7} |
| L_{A2445} | R^{B43} | R^{B7} | R^{A3} | H | L_{A2717} | R^{B43} | R^{A34} | H | R^{B12} |
| L_{A2446} | R^{B43} | R^{B7} | R^{A34} | H | L_{A2718} | R^{B43} | R^{A34} | H | R^{B18} |
| L_{A2447} | R^{B43} | R^{B12} | H | H | L_{A2719} | R^{B43} | R^{A34} | H | R^{A3} |
| L_{A2448} | R^{B43} | R^{B12} | R^{B1} | H | L_{A2720} | R^{B43} | R^{A34} | H | R^{A34} |

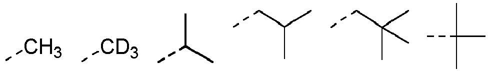

where R^{B1} to R^{B44} have the following structures: R^{B1} , R^{B2} , R^{B3} , R^{B4} , R^{B5} , R^{B6} 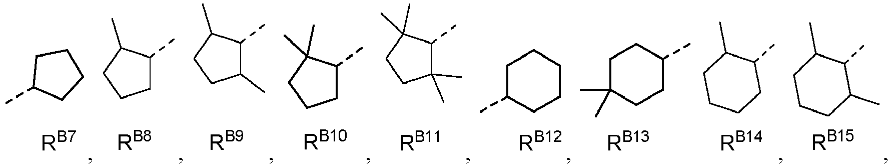 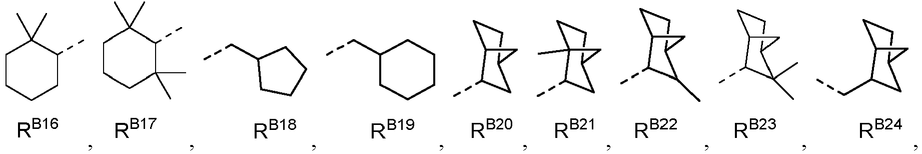 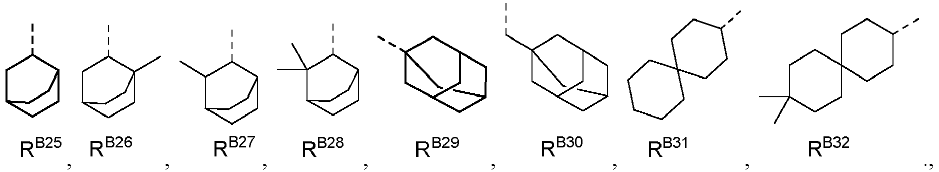 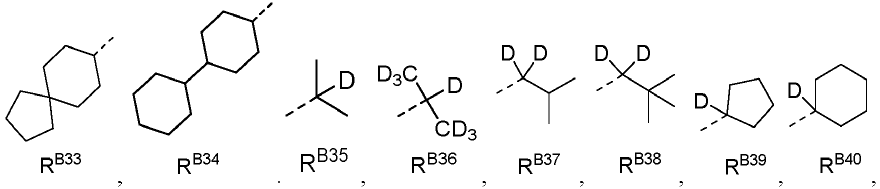 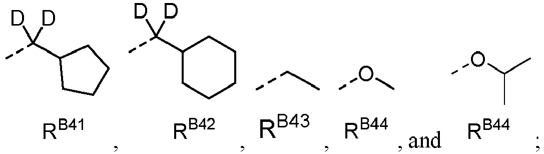 where R^{A1} to R^{A51} have the following structures: 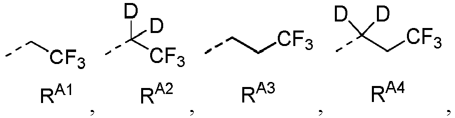 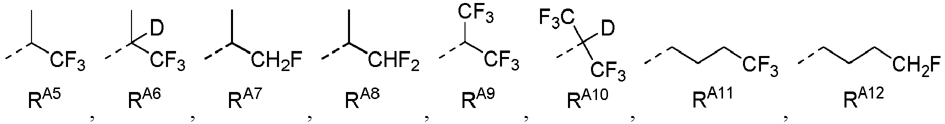 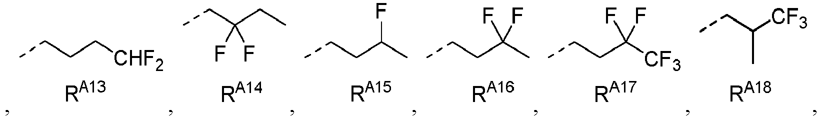 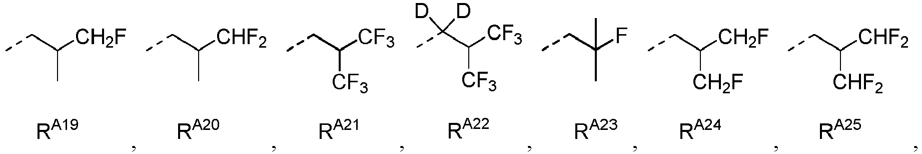 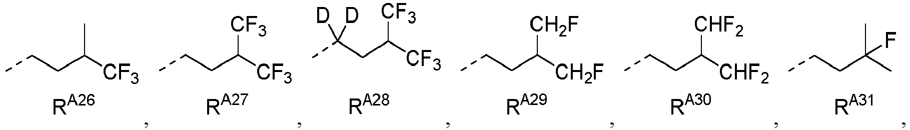 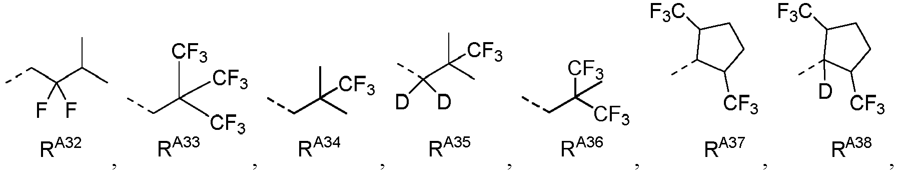 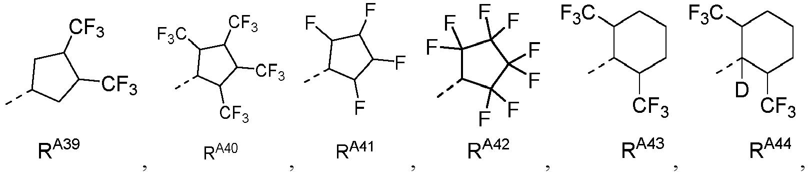 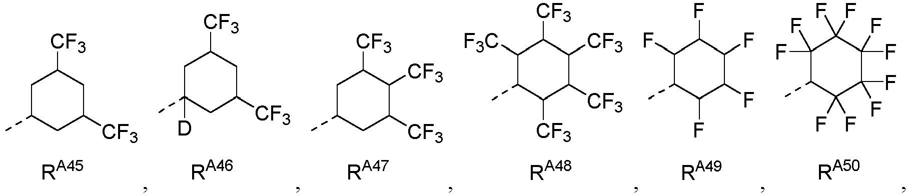 and

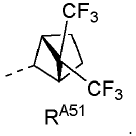

In some embodiments of the compound, the ligand L_{B} is selected from the group consisting of 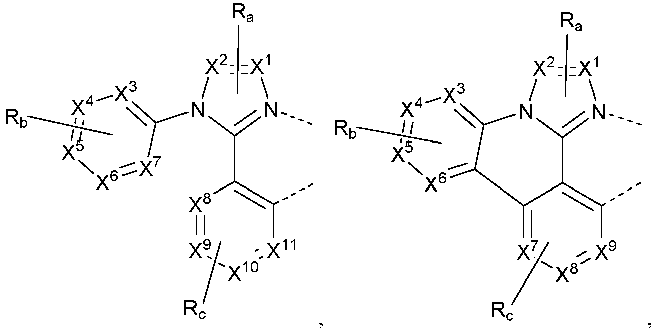 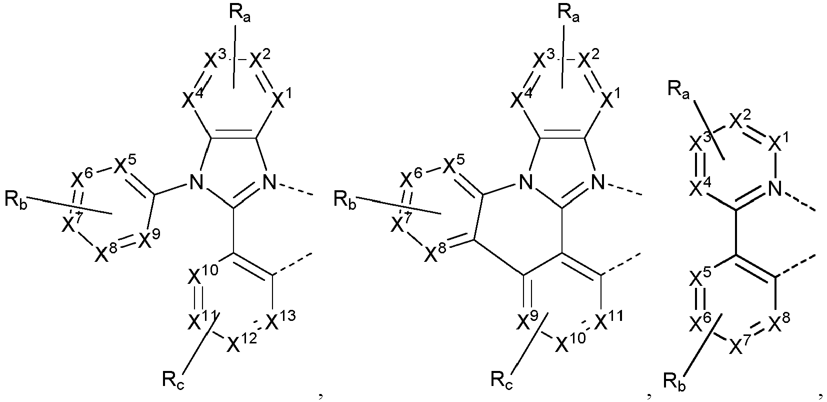 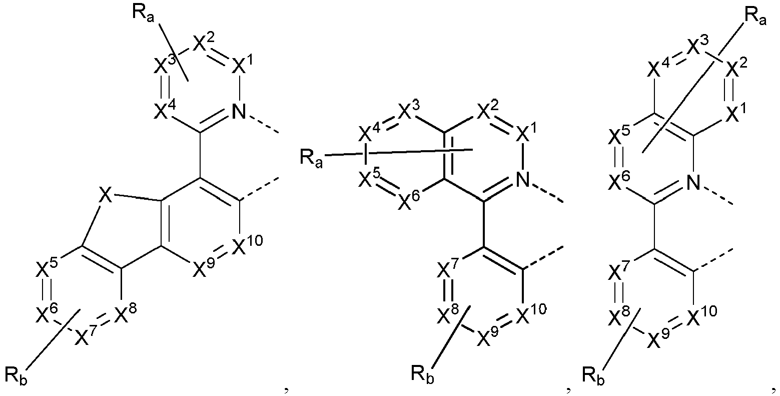 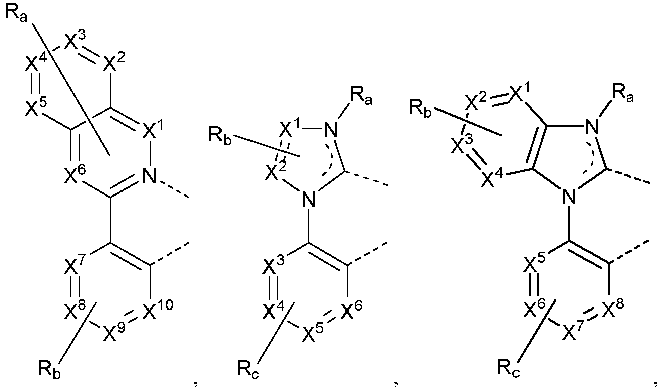 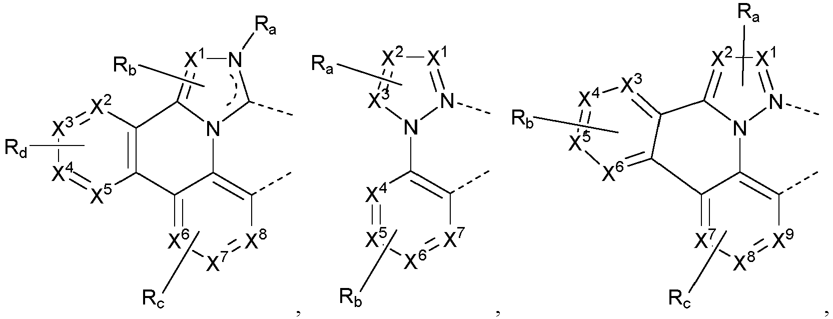 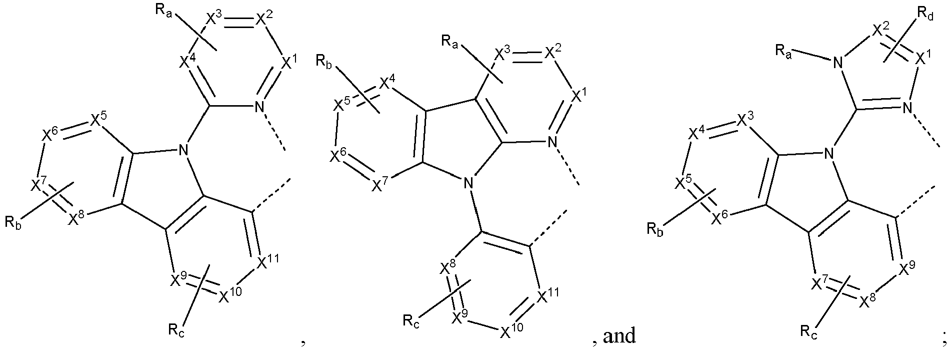 where each X¹ to X¹³ are independently selected from the group consisting of carbon and nitrogen; where X is selected from the group consisting of BR', NR', PR', O, S, Se, C=O, S=O, SO₂, CR'R", SiR'R", and GeR'R"; where R' and R" are optionally fused or joined to form a ring; where each Rₐ, R_{b}, R_{c}, and R_{d} may represent from mono substitution to the possible maximum number of substitution, or no substitution; where R', R", Rₐ, R_{b}, R_{c}, and Rₐ are each independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof; and where any two adjacent substituents of Rₐ, R_{b}, R_{c}, and R_{d} are optionally fused or joined to form a ring or form a multidentate ligand. In some embodiments, the ligand L_{B} is selected from the group consisting of 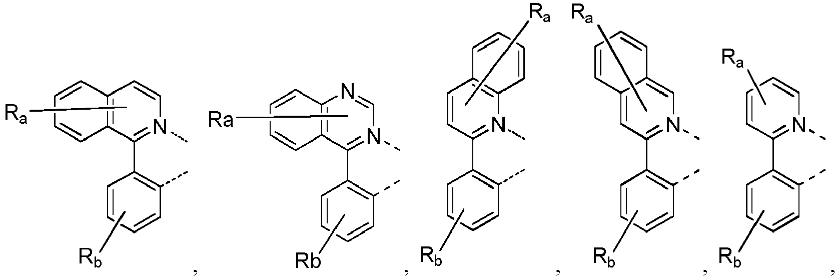 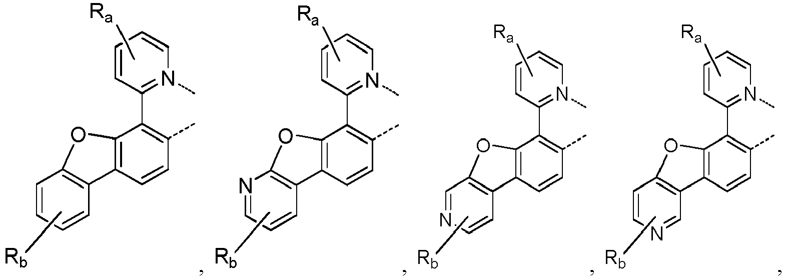 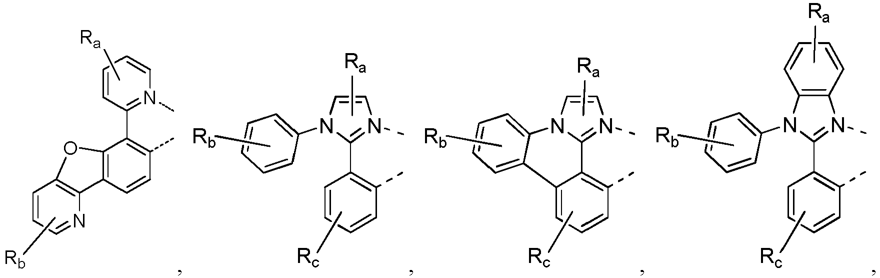 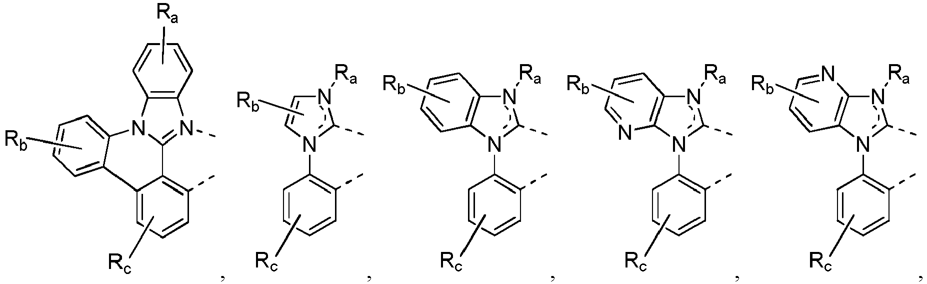 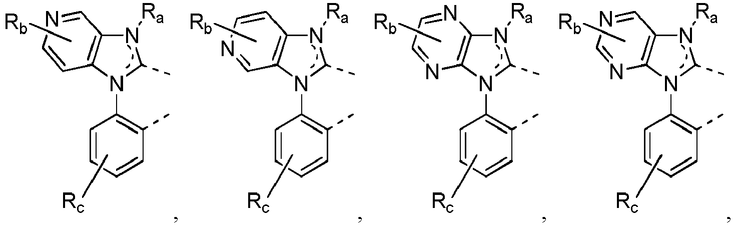 and

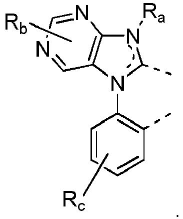

In some embodiments of the compound, the ligand L_{C} is selected from the group consisting of

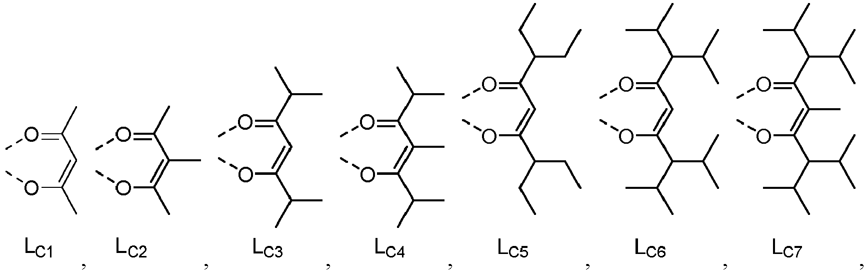

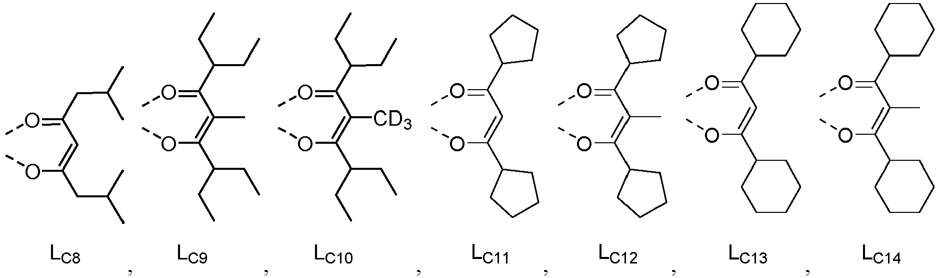

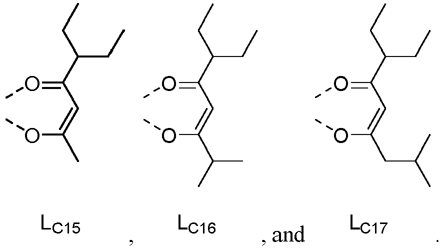

In some embodiments of the compound, where the ligand L_{A} is selected from the group consisting of L_{A1} through L_{A2720}, the compound is the Compound *x* having the formula Ir(L_{A*i*}))₂(L_{C*j*}); where *x* = 2720*i*+*j*-2720; *i* is an integer from 1 to 17, and *j* is an integer from 1 to 2720; and L_{C} is selected from the group consisting of:

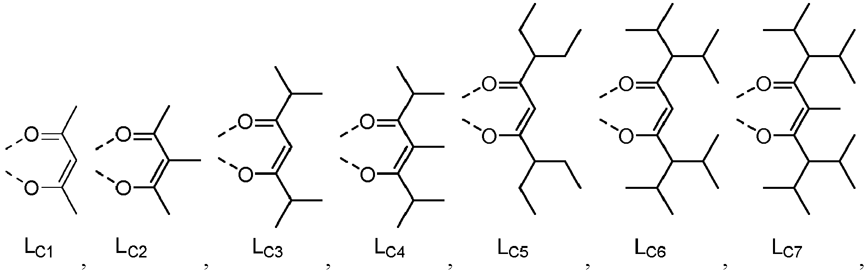

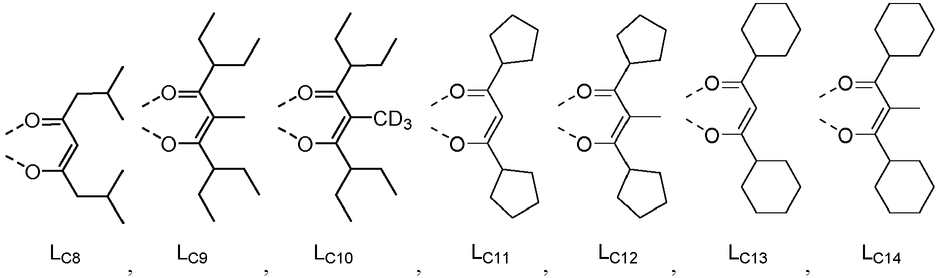

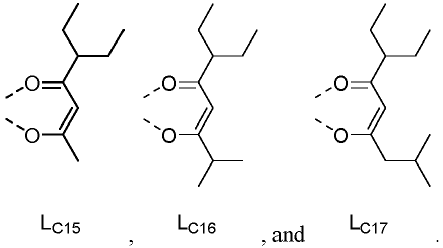

An organic light emitting device (OLED) is also disclosed. The OLED comprises an anode; a cathode; and an organic layer, disposed between the anode and the cathode. The organic layer comprises a compound having a formula M(L_{A})ₓ(L_{B})_{y}(L_{C})_{z}:
where the ligand L_{A} has Formula I, 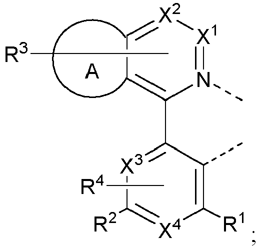 the ligand L_{B} has Formula II, 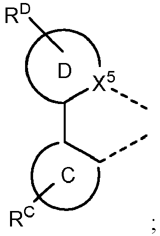 and the ligand L_{C} has Formula III, 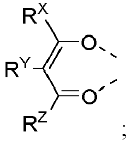 where M is a metal having an atomic weight greater than 40; x is 1, 2, or 3; y is 0, 1, or 2; z is 0, 1, or 2; x+y+z is the oxidation state of the metal M; X¹ to X⁵ are each independently a carbon or nitrogen; ring A is a 5- or 6-membered aromatic ring fused to the ring having X¹; rings C and D are each independently a 5 or 6-membered carbocyclic or heterocyclic ring; R³, R⁴, R^{C}, and R^{D} each independently represent none to possible maximum number of substitution;
where each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof; where any adjacent substituents of R³, R⁴, R^{C}, and R^{D}, R^{X}, R^{Y}, and R^{Z} are optionally joined or fused into a ring;
where R¹ and R² are each independently selected from the group consisting of alkyl, cycloalkyl, silyl, germanyl as defined in claim 1, and substituted variants thereof; and
where R¹ is different from R².

In some embodiments of the OLED, the organic layer is an emissive layer and the compound is an emissive dopant or a non-emissive dopant.

In some embodiments of the OLED, the organic layer further comprises a host, where the host comprises a triphenylene containing benzo-fused thiophene or benzo-fused furan;
where any substituent in the host is an unfused substituent independently selected from the group consisting of CₙH₂ₙ₊₁, OCₙH₂ₙ₊₁, OAr₁, N(CₙH₂ₙ₊₁)₂, N(Ar₁)(Ar₂), CH=CH-CₙH₂ₙ₊₁, C≡CCₙH₂ₙ₊₁, Ar₁, Ar₁-Ar₂, and CₙH₂ₙ-Ar₁, or the host has no substitution;
where n is from 1 to 10; and
where Ar₁ and Ar₂ are independently selected from the group consisting of benzene, biphenyl, naphthalene, triphenylene, carbazole, and heteroaromatic analogs thereof.

In some embodiments of the OLED, the organic layer further comprises a host, where host comprises at least one chemical group selected from the group consisting of triphenylene, carbazole, dibenzothiphene, dibenzofuran, dibenzoselenophene, azatriphenylene, azacarbazole, aza-dibenzothiophene, aza-dibenzofuran, and aza-dibenzoselenophene.

In some embodiments of the OLED, the organic layer further comprises a host, wherein the host is selected from the group consisting of 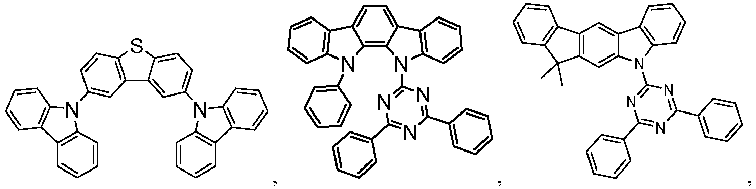 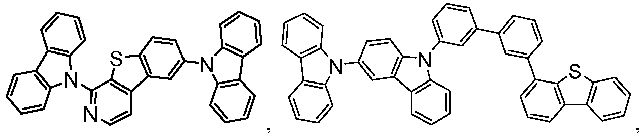 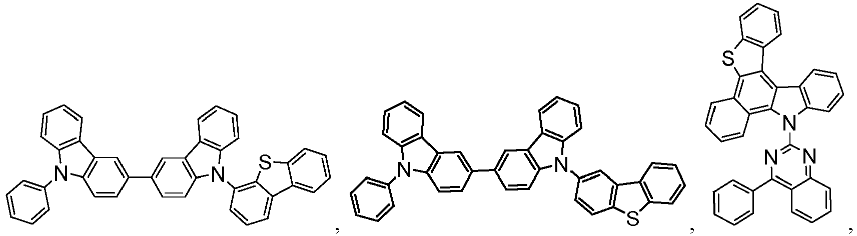 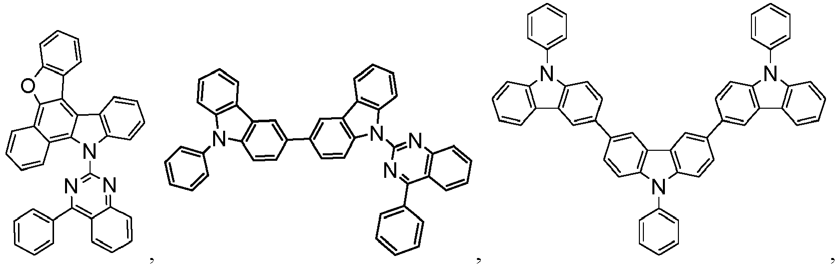 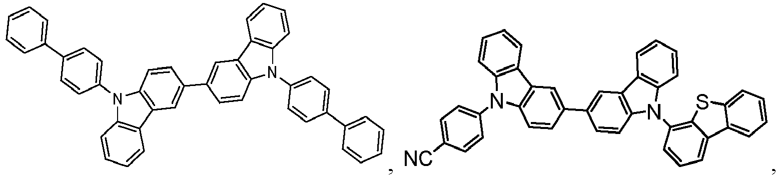 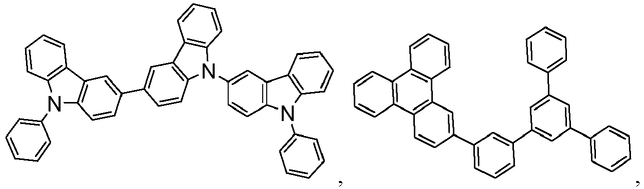 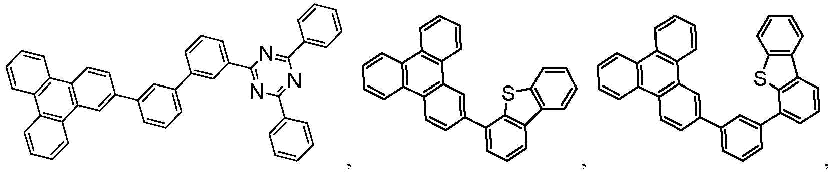 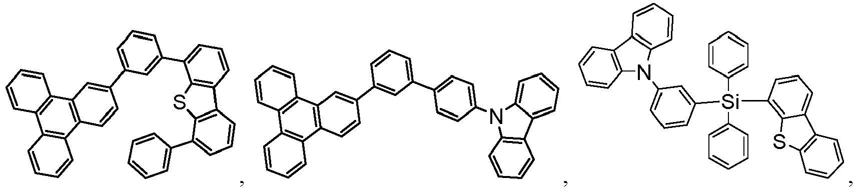 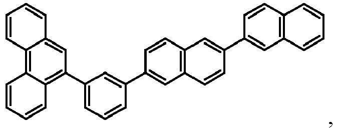 and combinations thereof.

In some embodiments of the first device, the organic layer further comprises a host, wherein the host comprises a metal complex.

A consumer product comprising an OLED is disclosed, where the OLED comprises an anode; a cathode; and an organic layer, disposed between the anode and the cathode, comprising a compound having a formula M(L_{A})ₓ(L_{B})_{y}(L_{c})_{z}:
where the ligand L_{A} has Formula I, 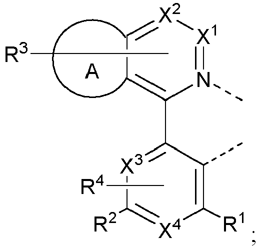
where the ligand L_{B} has Formula II, 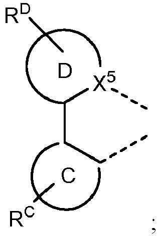
where the ligand L_{C} has Formula III, 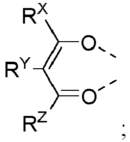
where M is a metal having an atomic weight greater than 40;
where x is 1, 2, or 3;
where y is 0, 1, or 2;
where z is 0, 1, or 2;
where x+y+z is the oxidation state of the metal M;
where X¹ to X⁵ are each independently a carbon or nitrogen;
where ring A is a 5- or 6-membered aromatic ring fused to the ring having X¹;
where rings C and D are each independently a 5 or 6-membered carbocyclic or heterocyclic ring;
where R³, R⁴, R^{C}, and R^{D} each independently represent none to possible maximum number of substitution;
where each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof;
where any adjacent substituents of R³, R⁴, R^{C}, and R^{D}, R^{X}, R^{Y}, and R^{Z} are optionally joined or fused into a ring;
where R¹ and R² are each independently selected from the group consisting of alkyl, cycloalkyl, silyl, germanyl, and substituted variants thereof as defined in claim 1; and
where R¹ is different from R².

In some embodiments, the OLED has one or more characteristics selected from the group consisting of being flexible, being rollable, being foldable, being stretchable, and being curved. In some embodiments, the OLED is transparent or semi-transparent. In some embodiments, the OLED further comprises a layer comprising carbon nanotubes.

In some embodiments, the OLED further comprises a layer comprising a delayed fluorescent emitter. In some embodiments, the OLED comprises a RGB pixel arrangement or white plus color filter pixel arrangement. In some embodiments, the OLED is a mobile device, a hand held device, or a wearable device. In some embodiments, the OLED is a display panel having less than 25,4 cm (10 inch) diagonal or 322,6 cm² (50 square inch) area. In some embodiments, the OLED is a display panel having at least 25,4 cm (10 inch) diagonal or 322,6 cm² (50 square inch) area. In some embodiments, the OLED is a lighting panel.

An emissive region in an OLED is disclosed, where the emissive region comprises a compound having a formula M(L_{A})ₓ(L_{B})_{y}(L_{c})_{z}:
where the ligand L_{A} has Formula I, 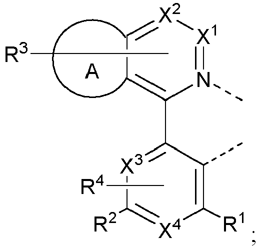
where the ligand L_{B} has Formula II, 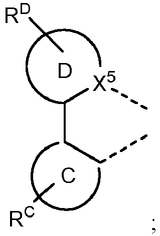
where the ligand L_{C} has Formula III, 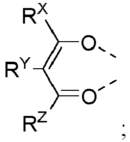
where M is a metal having an atomic weight greater than 40;
where x is 1, 2, or 3;
where y is 0, 1, or 2;
where z is 0, 1, or 2;
where x+y+z is the oxidation state of the metal M;
where X¹ to X⁵ are each independently a carbon or nitrogen;
where ring A is a 5- or 6-membered aromatic ring fused to the ring having X¹;
where rings C and D are each independently a 5 or 6-membered carbocyclic or heterocyclic ring;
where R³, R⁴, R^{C}, and R^{D} each independently represent none to possible maximum number of substitution;
where each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof;
where any adjacent substituents of R³, R⁴, R^{C}, and R^{D}, R^{X}, R^{Y}, and R^{Z} are optionally joined or fused into a ring;
where R¹ and R² are each independently selected from the group consisting of alkyl, cycloalkyl, silyl, germanyl, and substituted variants thereof as defined in claim 1; and
where R¹ is different from R².

In some embodiment of the emissive region, the compound is an emissive dopant or a non-emissive dopant.

In some embodiment of the emissive region, the emissive region further comprises a host, wherein the host comprises at least one selected from the group consisting of metal complex, triphenylene, carbazole, dibenzothiophene, dibenzofuran, dibenzoselenophene, aza-triphenylene, azacarbazole, aza-dibenzothiophene, aza-dibenzofuran, and aza-dibenzoselenophene.

In some embodiment of the emissive region, the emissive region further comprises a host, wherein the host is selected from the group consisting of: 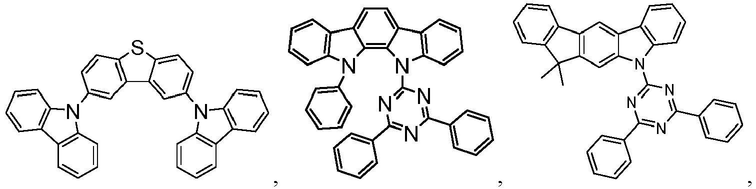 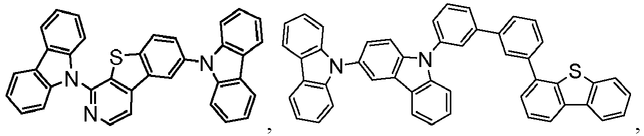 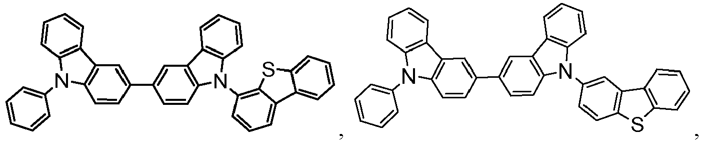 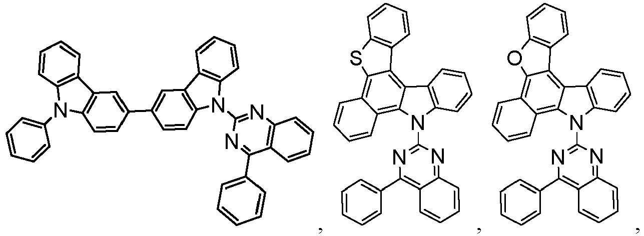 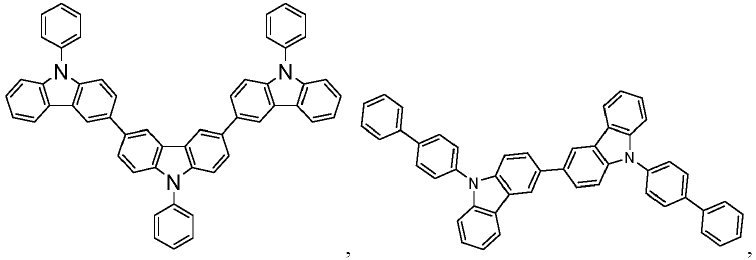 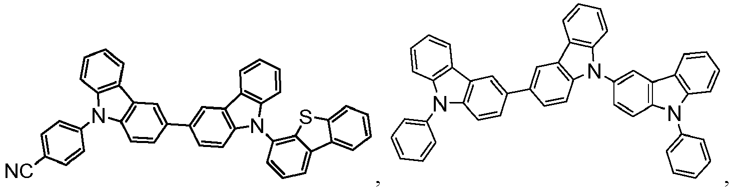 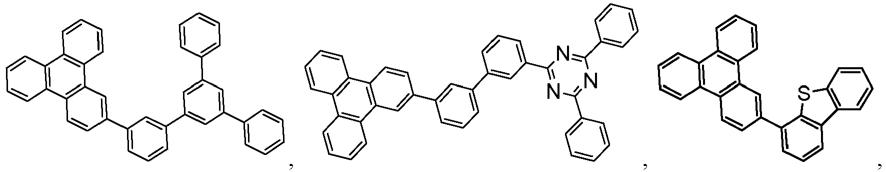 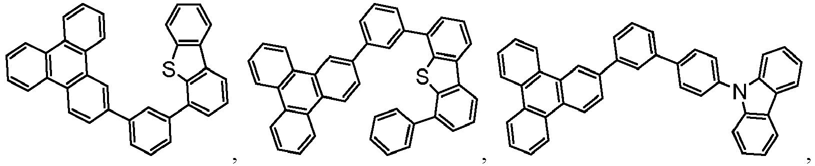 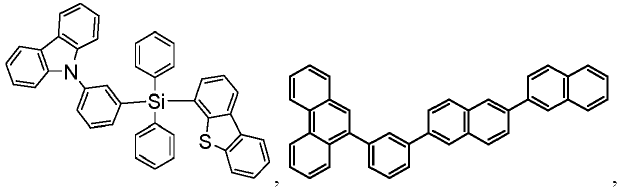 and combinations thereof.

In some embodiments, the compound can be an emissive dopant. In some embodiments, the compound can produce emissions via phosphorescence, fluorescence, thermally activated delayed fluorescence, i.e., TADF (also referred to as E-type delayed fluorescence), triplet-triplet annihilation, or combinations of these processes.

According to another aspect, a formulation comprising the compound described herein is also disclosed.

The OLED disclosed herein can be incorporated into one or more of a consumer product, an electronic component module, and a lighting panel. The organic layer can be an emissive layer and the compound can be an emissive dopant in some embodiments, while the compound can be a non-emissive dopant in other embodiments.

The organic layer can also include a host. In some embodiments, two or more hosts are preferred. In some embodiments, the hosts used maybe a) bipolar, b) electron transporting, c) hole transporting or d) wide band gap materials that play little role in charge transport. In some embodiments, the host can include a metal complex. The host can be a triphenylene containing benzo-fused thiophene or benzo-fused furan. Any substituent in the host can be an unfused substituent independently selected from the group consisting of CₙH₂ₙ₊₁, OCₙH₂ₙ₊₁, OAr₁, N(CₙH₂ₙ₊₁)₂, N(Ar₁)(Ar₂), CH=CH-CₙH₂ₙ₊₁, C≡C-CₙH₂ₙ₊₁, Ar₁, Ar₁-Ar₂, and CₙH₂ₙ₋Ar₁, or the host has no substitutions. In the preceding substituents n can range from 1 to 10; and Ar₁ and Ar₂ can be independently selected from the group consisting of benzene, biphenyl, naphthalene, triphenylene, carbazole, and heteroaromatic analogs thereof. The host can be an inorganic compound. For example a Zn containing inorganic material e.g. ZnS.

The host can be a compound comprising at least one chemical group selected from the group consisting of triphenylene, carbazole, dibenzothiophene, dibenzofuran, dibenzoselenophene, azatriphenylene, azacarbazole, aza-dibenzothiophene, aza-dibenzofuran, and aza-dibenzoselenophene. The host can include a metal complex. The host can be, but is not limited to, a specific compound selected from the group consisting of 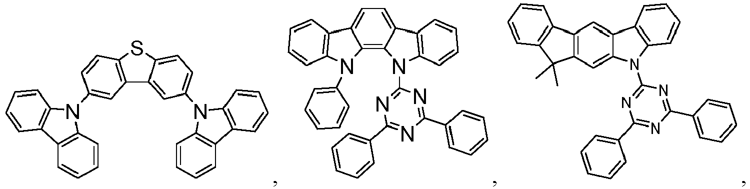 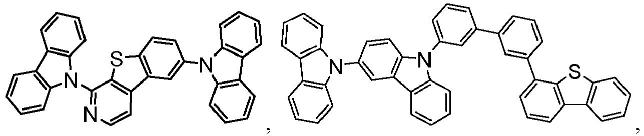 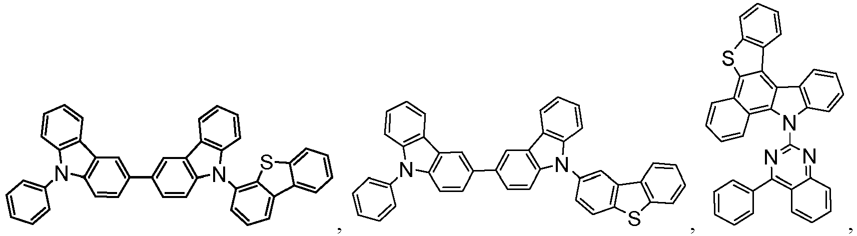 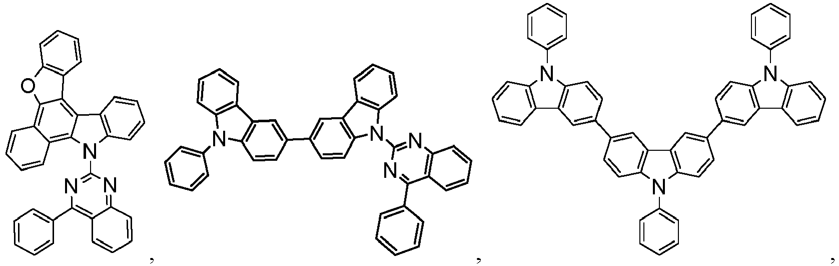 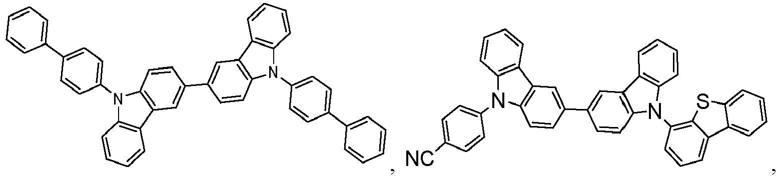 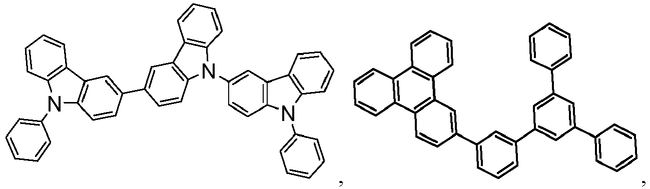 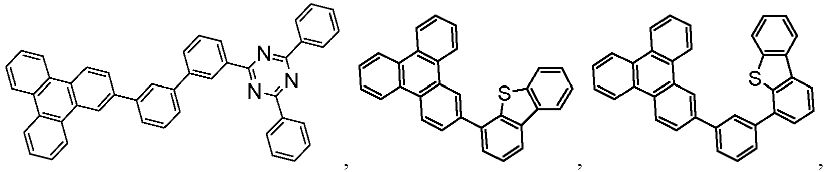 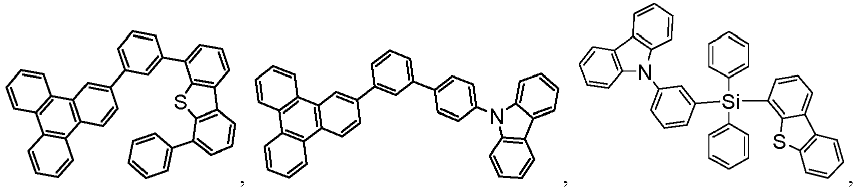 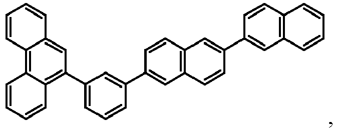 and combinations thereof.
Additional information on possible hosts is provided below.

In yet another aspect of the present disclosure, a formulation that comprises the novel compound disclosed herein is described. The formulation can include one or more components selected from the group consisting of a solvent, a host, a hole injection material, hole transport material, and an electron transport layer material, disclosed herein.

### COMBINATION WITH OTHER MATERIALS

The materials described herein as useful for a particular layer in an organic light emitting device may be used in combination with a wide variety of other materials present in the device. For example, emissive dopants disclosed herein may be used in conjunction with a wide variety of hosts, transport layers, blocking layers, injection layers, electrodes and other layers that may be present. The materials described or referred to below are non-limiting examples of materials that may be useful in combination with the compounds disclosed herein, and one of skill in the art can readily consult the literature to identify other materials that may be useful in combination.

### Conductivity Dopants:

A charge transport layer can be doped with conductivity dopants to substantially alter its density of charge carriers, which will in turn alter its conductivity. The conductivity is increased by generating charge carriers in the matrix material, and depending on the type of dopant, a change in the Fermi level of the semiconductor may also be achieved. Hole-transporting layer can be doped by p-type conductivity dopants and n-type conductivity dopants are used in the electron-transporting layer.

Non-limiting examples of the conductivity dopants that may be used in an OLED in combination with materials disclosed herein are exemplified below together with references that disclose those materials: EP01617493, EP01968131, EP2020694, EP2684932, US20050139810, US20070160905, US20090167167, US2010288362, WO06081780, WO2009003455, WO2009008277, WO2009011327, WO2014009310, US2007252140, US2015060804 and US2012146012.

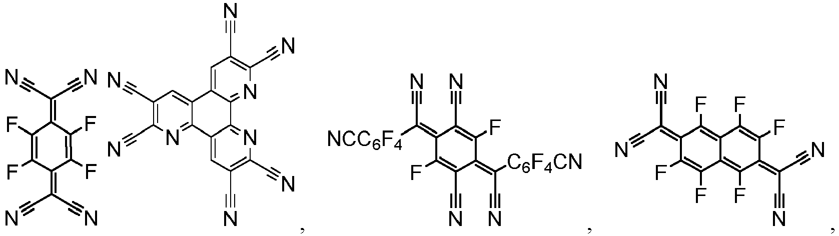

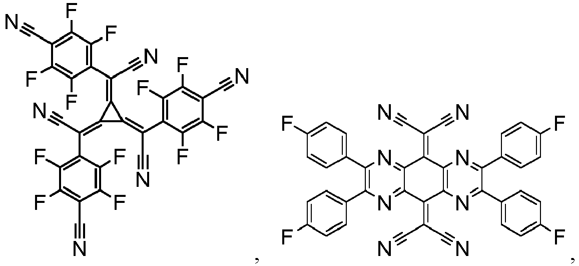

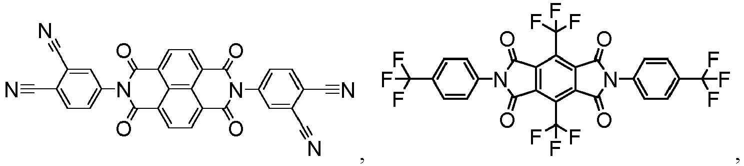

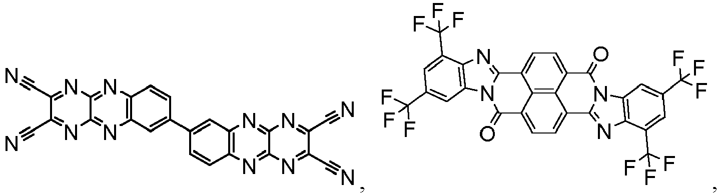

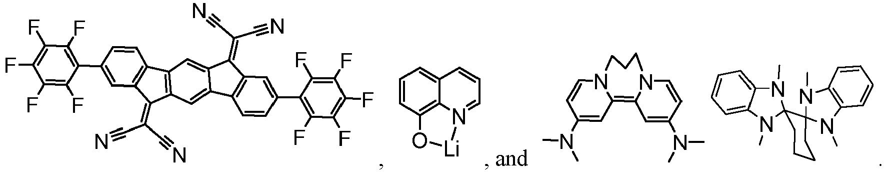

### HIL/HTL:

A hole injecting/transporting material to be used in the present invention is not particularly limited, and any compound may be used as long as the compound is typically used as a hole injecting/transporting material. Examples of the material include, but are not limited to: a phthalocyanine or porphyrin derivative; an aromatic amine derivative; an indolocarbazole derivative; a polymer containing fluorohydrocarbon; a polymer with conductivity dopants; a conducting polymer, such as PEDOT/PSS; a self-assembly monomer derived from compounds such as phosphonic acid and silane derivatives; a metal oxide derivative, such as MoOₓ; a p-type semiconducting organic compound, such as 1,4,5,8,9,12-Hexaazatriphenylenehexacarbonitrile; a metal complex, and a crosslinkable compound.

Examples of aromatic amine derivatives used in HIL or HTL include, but not limit to the following general structures:

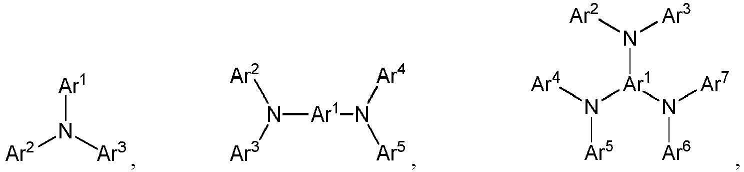

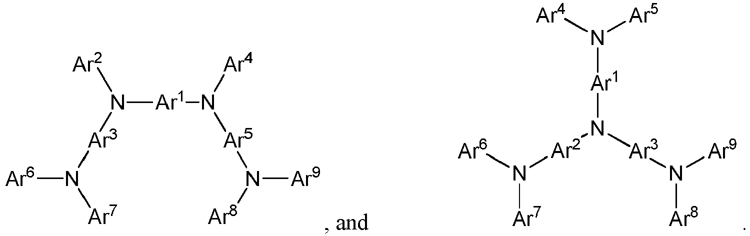

Each of Ar¹ to Ar⁹ is selected from the group consisting of aromatic hydrocarbon cyclic compounds such as benzene, biphenyl, triphenyl, triphenylene, naphthalene, anthracene, phenalene, phenanthrene, fluorene, pyrene, chrysene, perylene, and azulene; the group consisting of aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and the group consisting of 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Each Ar may be unsubstituted or may be substituted by a substituent selected from the group consisting of deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof.

In one aspect, Ar¹ to Ar⁹ is independently selected from the group consisting of: 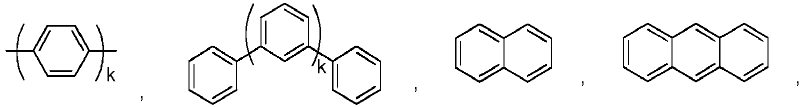 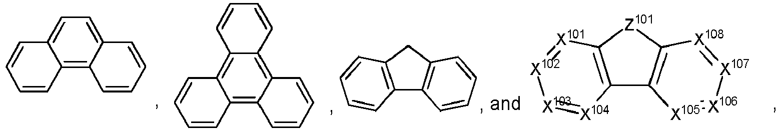 wherein k is an integer from 1 to 20; X¹⁰¹ to X¹⁰⁸ is C (including CH) or N; Z¹⁰¹ is NAr¹, O, or S; Ar¹ has the same group defined above.

Examples of metal complexes used in HIL or HTL include, but are not limited to the following general formula: 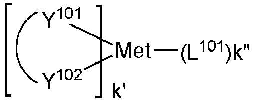 wherein Met is a metal, which can have an atomic weight greater than 40; (Y¹⁰¹-Y¹⁰²) is a bidentate ligand, Y¹⁰¹ and Y¹⁰² are independently selected from C, N, O, P, and S; L¹⁰¹ is an ancillary ligand; k' is an integer value from 1 to the maximum number of ligands that may be attached to the metal; and k'+k" is the maximum number of ligands that may be attached to the metal.

In one aspect, (Y¹⁰¹-Y¹⁰²) is a 2-phenylpyridine derivative. In another aspect, (Y¹⁰¹-Y¹⁰²) is a carbene ligand. In another aspect, Met is selected from Ir, Pt, Os, and Zn. In a further aspect, the metal complex has a smallest oxidation potential in solution vs. Fc⁺/Fc couple less than about 0.6 V.

Non-limiting examples of the HIL and HTL materials that may be used in an OLED in combination with materials disclosed herein are exemplified below together with references that disclose those materials: CN102702075, DE102012005215, EP01624500, EP01698613, EP01806334, EP01930964, EP01972613, EP01997799, EP02011790, EP02055700, EP02055701, EP1725079, EP2085382, EP2660300, EP650955, JP07-073529, JP2005112765, JP2007091719, JP2008021687, JP2014-009196, KR20110088898, KR20130077473, TW201139402, US06517957, US20020158242, US20030162053, US20050123751, US20060182993, US20060240279, US20070145888, US20070181874, US20070278938, US20080014464, US20080091025, US20080106190, US20080124572, US20080145707, US20080220265, US20080233434, US20080303417, US2008107919, US20090115320, US20090167161, US2009066235, US2011007385, US20110163302, US2011240968, US2011278551, US2012205642, US2013241401, US20140117329, US2014183517, US5061569, US5639914, WO05075451, WO07125714, WO08023550, WO08023759, WO2009145016, WO2010061824, WO2011075644, WO2012177006, WO2013018530, WO2013039073, WO2013087142, WO2013118812, WO2013120577, WO2013157367, WO2013175747, WO2014002873, WO2014015935, WO2014015937, WO2014030872, WO2014030921, WO2014034791, WO2014104514, WO2014157018. 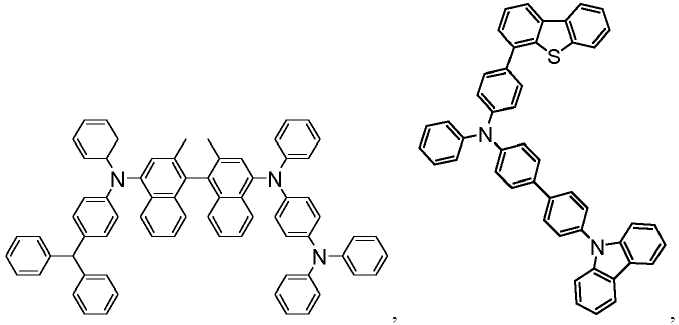 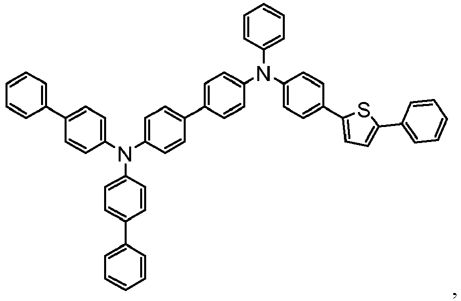 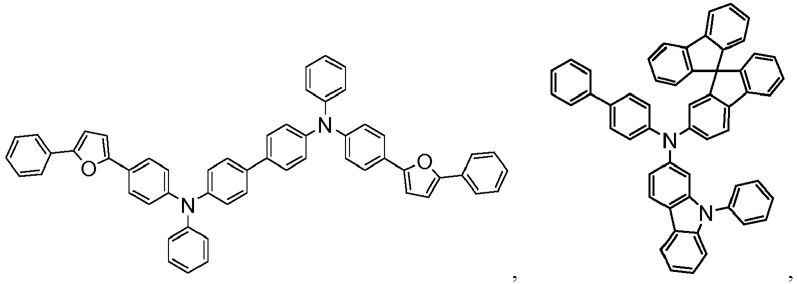 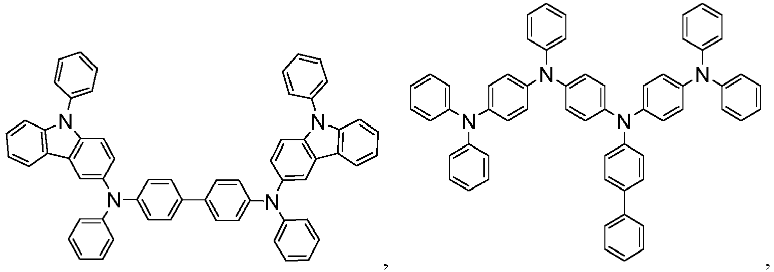 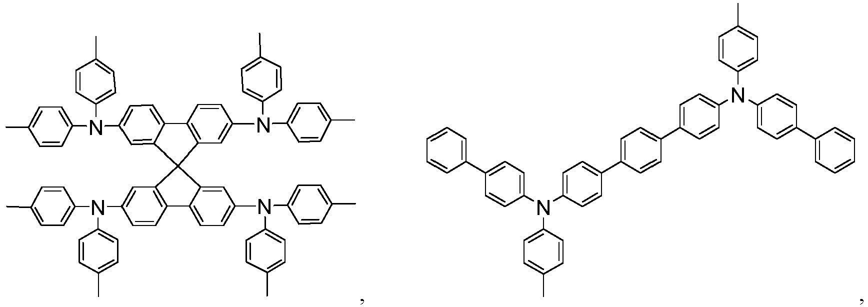 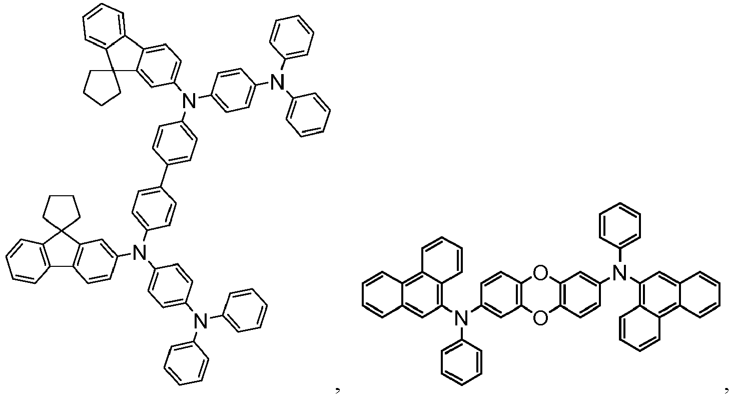 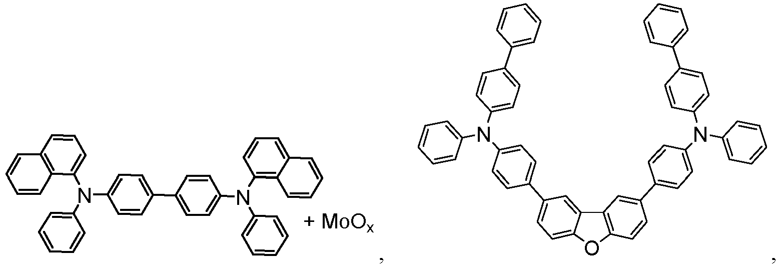 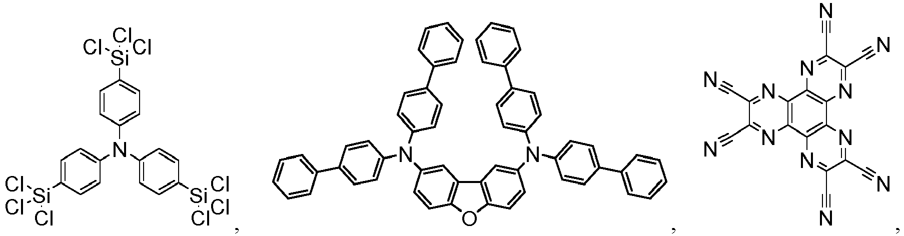 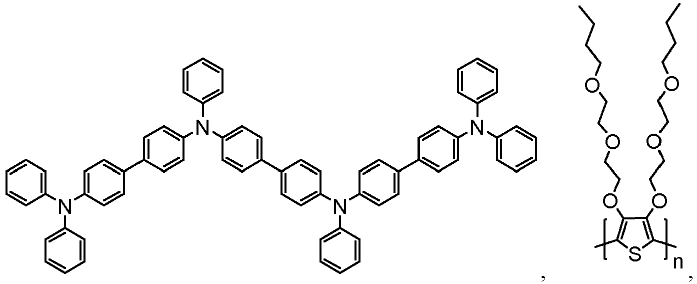 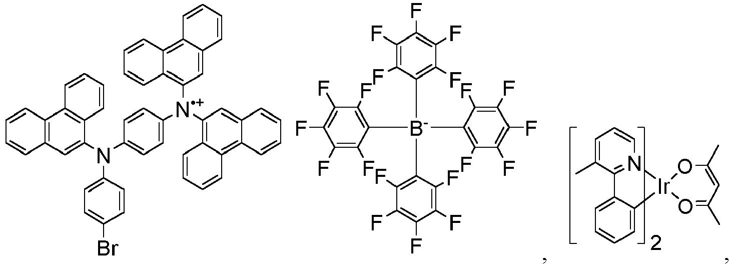 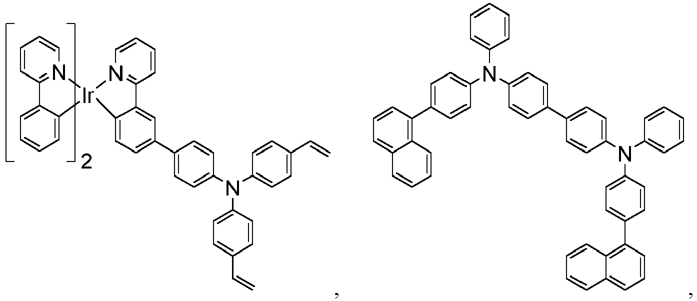 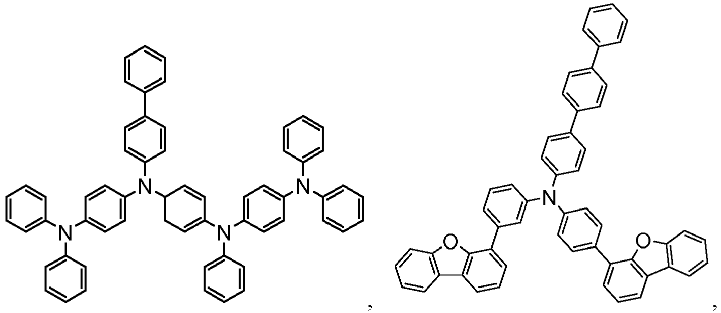 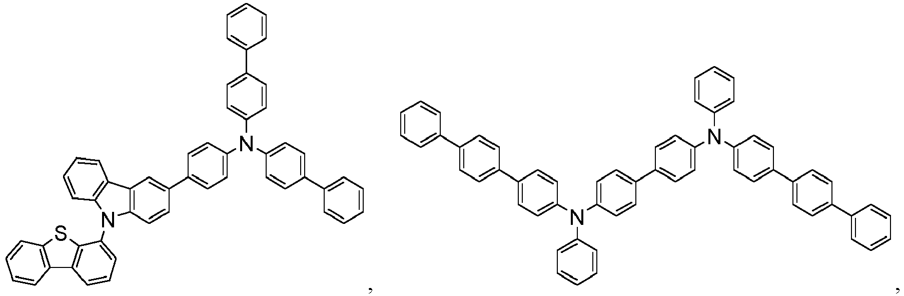 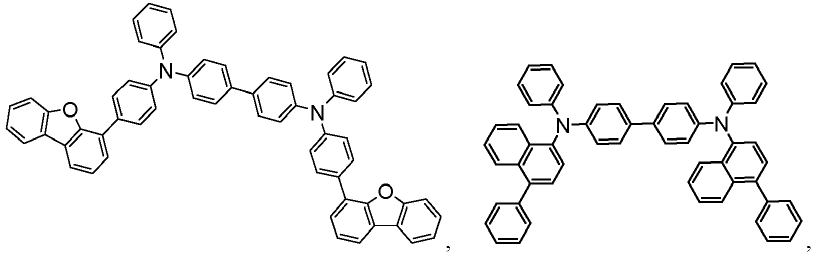 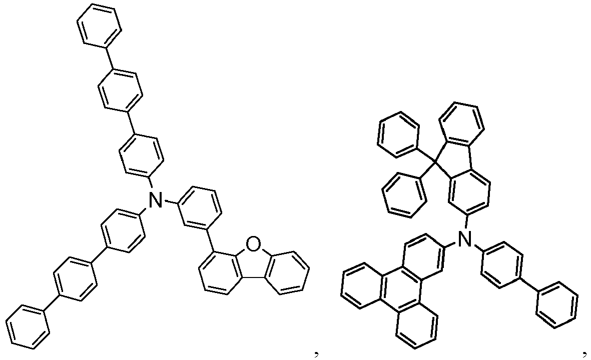 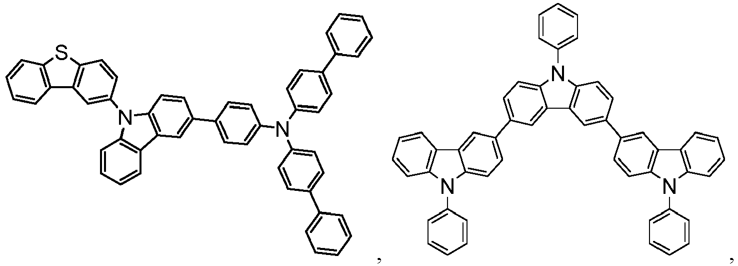 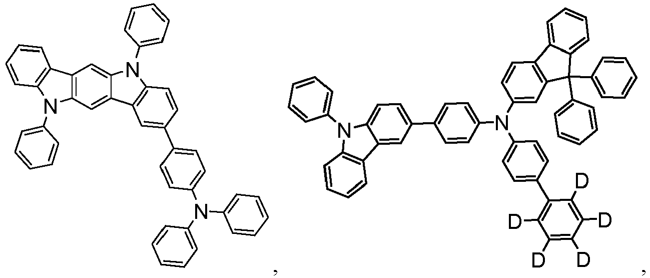 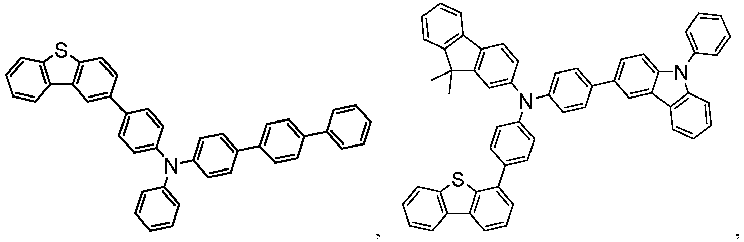 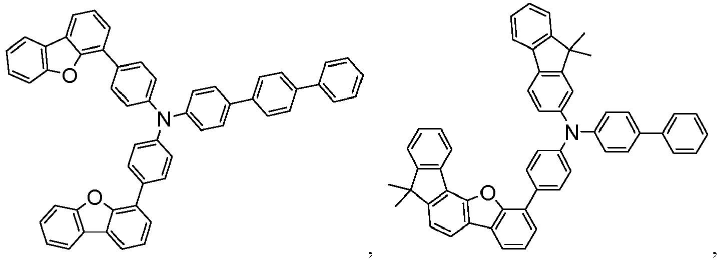 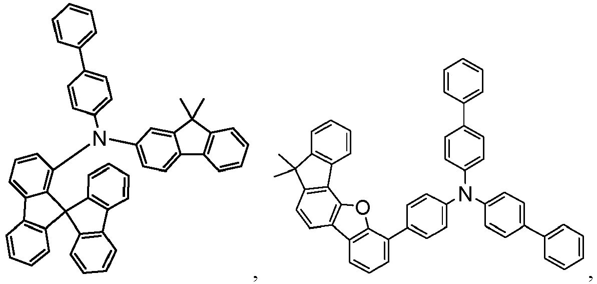 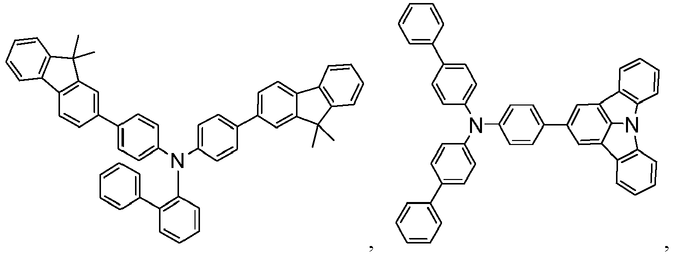 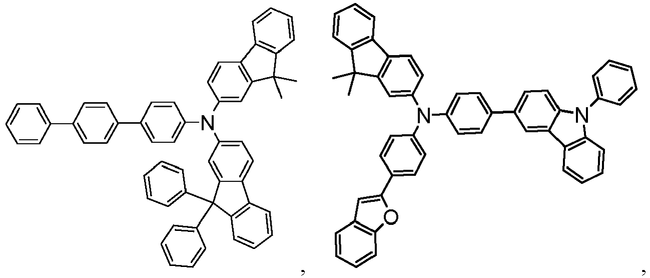 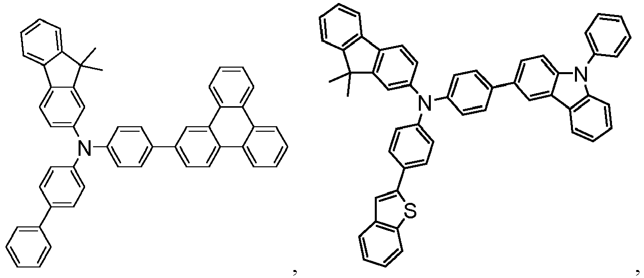 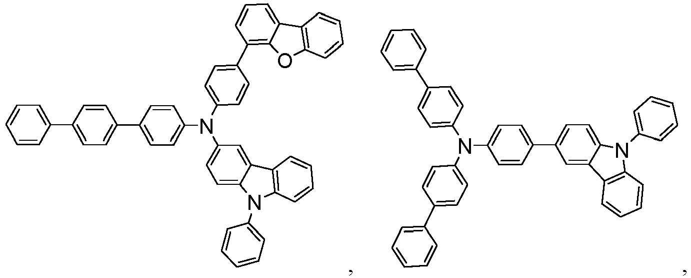 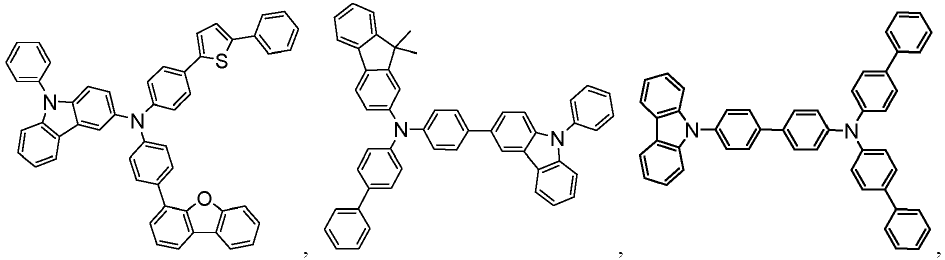 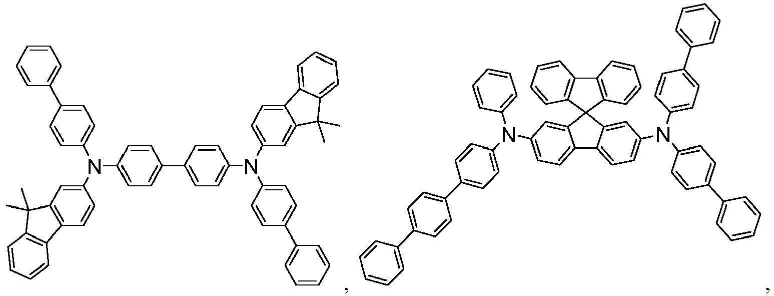 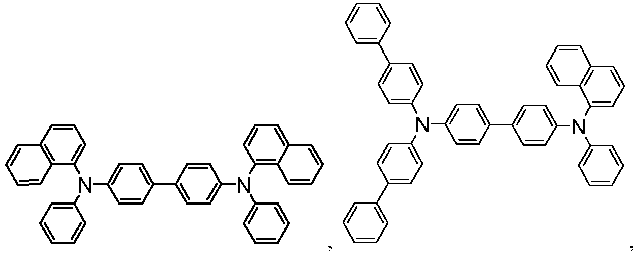 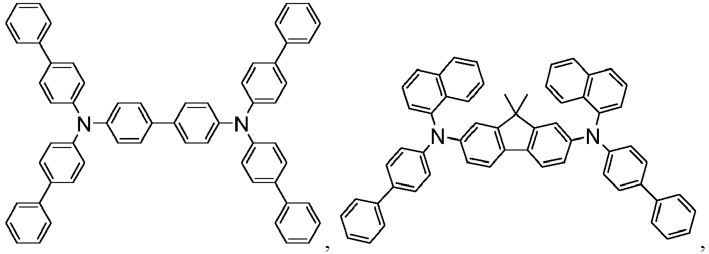 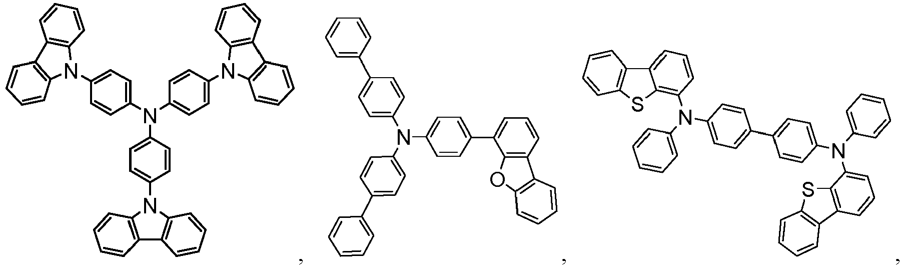 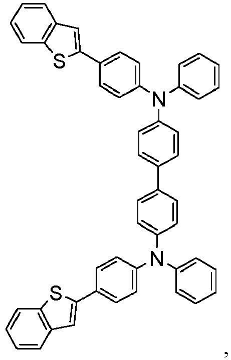 and

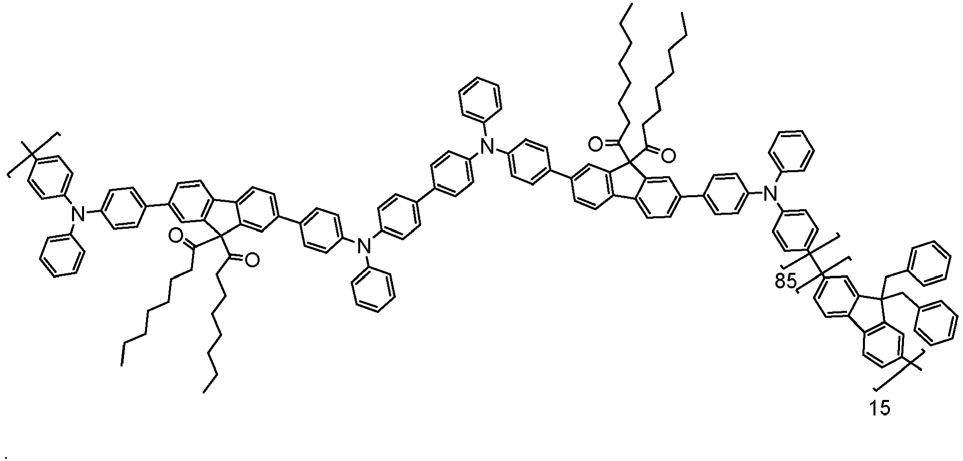

### EBL:

An electron blocking layer (EBL) may be used to reduce the number of electrons and/or excitons that leave the emissive layer. The presence of such a blocking layer in a device may result in substantially higher efficiencies, and or longer lifetime, as compared to a similar device lacking a blocking layer. Also, a blocking layer may be used to confine emission to a desired region of an OLED. In some embodiments, the EBL material has a higher LUMO (closer to the vacuum level) and/or higher triplet energy than the emitter closest to the EBL interface. In some embodiments, the EBL material has a higher LUMO (closer to the vacuum level) and or higher triplet energy than one or more of the hosts closest to the EBL interface. In one aspect, the compound used in EBL contains the same molecule or the same functional groups used as one of the hosts described below.

### Host:

The light emitting layer of the organic EL device of the present invention preferably contains at least a metal complex as light emitting material, and may contain a host material using the metal complex as a dopant material. Examples of the host material are not particularly limited, and any metal complexes or organic compounds may be used as long as the triplet energy of the host is larger than that of the dopant. Any host material may be used with any dopant so long as the triplet criteria is satisfied.

Examples of metal complexes used as host are preferred to have the following general formula: 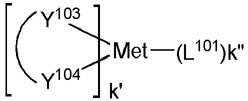 wherein Met is a metal; (Y¹⁰³-Y¹⁰⁴) is a bidentate ligand, Y¹⁰³ and Y¹⁰⁴ are independently selected from C, N, O, P, and S; L¹⁰¹ is an another ligand; k' is an integer value from 1 to the maximum number of ligands that may be attached to the metal; and k'+k" is the maximum number of ligands that may be attached to the metal.

In one aspect, the metal complexes are: 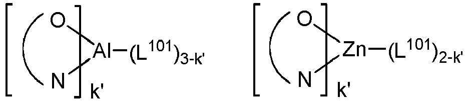 wherein (O-N) is a bidentate ligand, having metal coordinated to atoms O and N.

In another aspect, Met is selected from Ir and Pt. In a further aspect, (Y¹⁰³-Y¹⁰⁴) is a carbene ligand.

Examples of other organic compounds used as host are selected from the group consisting of aromatic hydrocarbon cyclic compounds such as benzene, biphenyl, triphenyl, triphenylene, tetraphenylene, naphthalene, anthracene, phenalene, phenanthrene, fluorene, pyrene, chrysene, perylene, and azulene; the group consisting of aromatic heterocyclic compounds such as dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine; and the group consisting of 2 to 10 cyclic structural units which are groups of the same type or different types selected from the aromatic hydrocarbon cyclic group and the aromatic heterocyclic group and are bonded to each other directly or via at least one of oxygen atom, nitrogen atom, sulfur atom, silicon atom, phosphorus atom, boron atom, chain structural unit and the aliphatic cyclic group. Each option within each group may be unsubstituted or may be substituted by a substituent selected from the group consisting of deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof.

In one aspect, the host compound contains at least one of the following groups in the molecule: 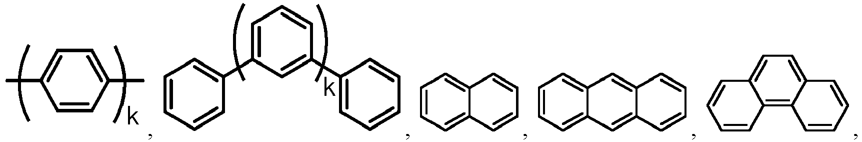 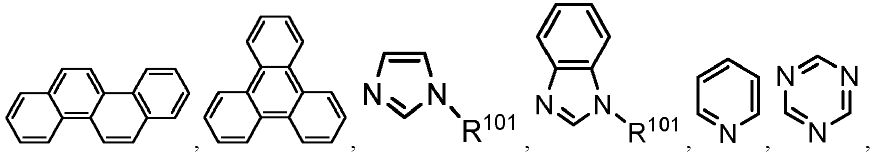 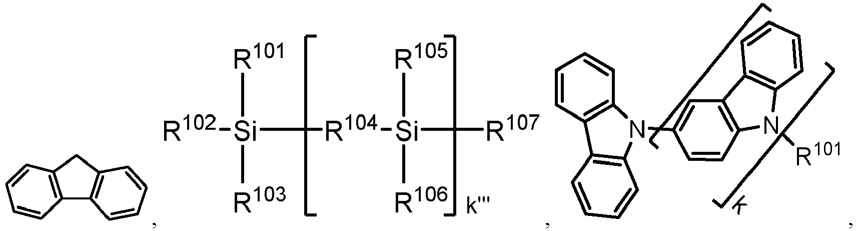 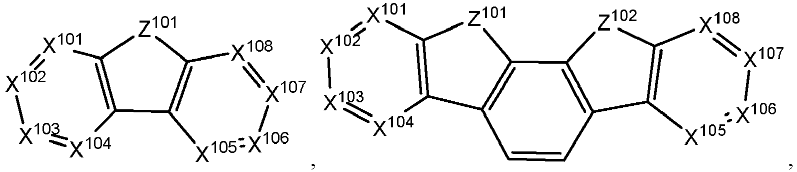 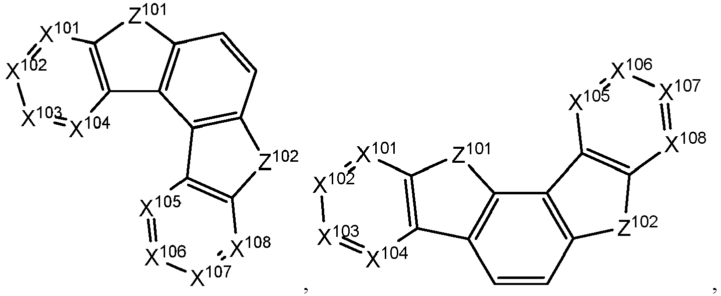 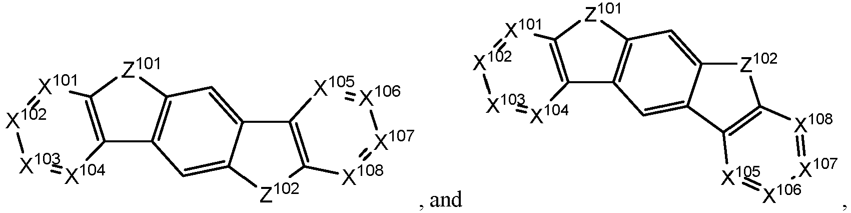
wherein each of R¹⁰¹ to R¹⁰⁷ is independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof, and when it is aryl or heteroaryl, it has the similar definition as Ar's mentioned above. k is an integer from 0 to 20 or 1 to 20; k"' is an integer from 0 to 20. X¹⁰¹ to X¹⁰⁸ is selected from C (including CH) or N.
Z¹⁰¹ and Z¹⁰² is selected from NR¹⁰¹, O, or S.

Non-limiting examples of the host materials that may be used in an OLED in combination with materials disclosed herein are exemplified below together with references that disclose those materials: EP2034538, EP2034538A, EP2757608, JP2007254297, KR20100079458, KR20120088644, KR20120129733, KR20130115564, TW201329200, US20030175553, US20050238919, US20060280965, US20090017330, US20090030202, US20090167162, US20090302743, US20090309488, US20100012931, US20100084966, US20100187984, US2010187984, US2012075273, US2012126221, US2013009543, US2013105787, US2013175519, US2014001446, US20140183503, US20140225088, US2014034914, US7154114, WO2001039234, WO2004093207, WO2005014551, WO2005089025, WO2006072002, WO2006114966, WO2007063754, WO2008056746, WO2009003898, WO2009021126, WO2009063833, WO2009066778, WO2009066779, WO2009086028, WO2010056066, WO2010107244, WO2011081423, WO2011081431, WO2011086863, WO2012128298, WO2012133644, WO2012133649, WO2013024872, WO2013035275, WO2013081315, WO2013191404, WO2014142472,

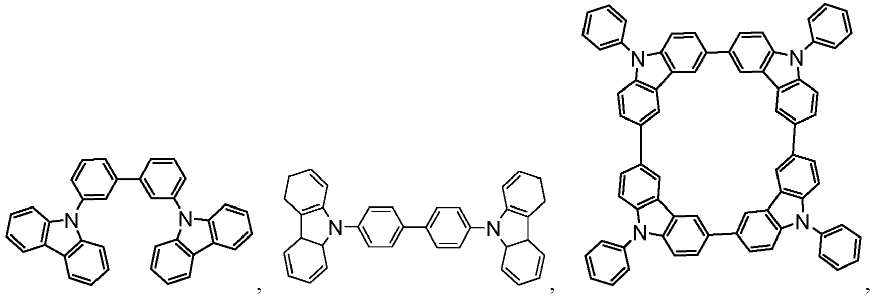

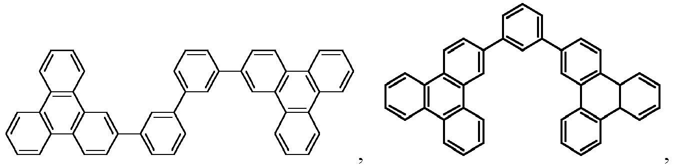

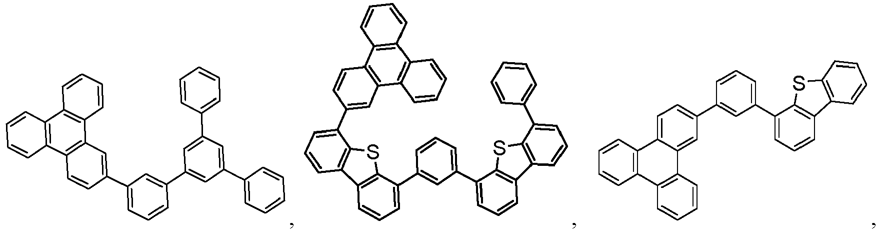

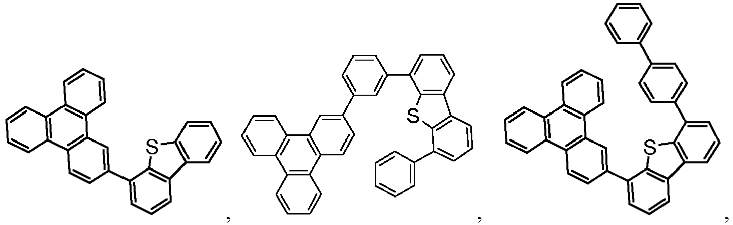

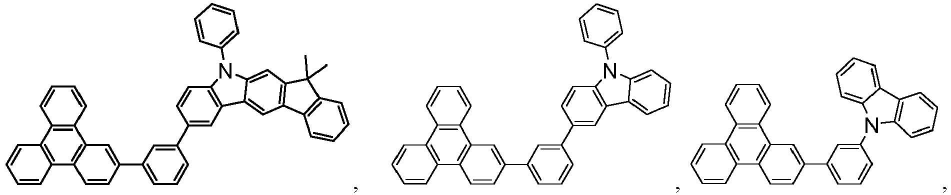

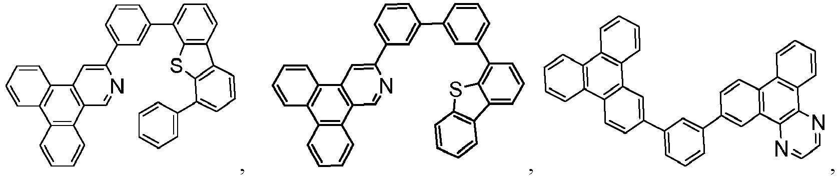

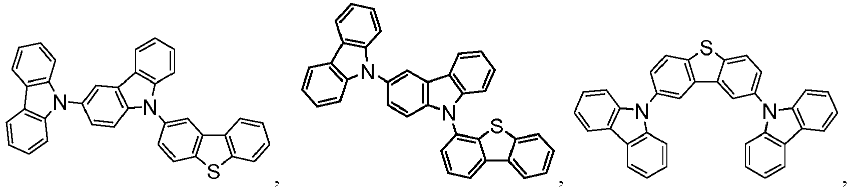

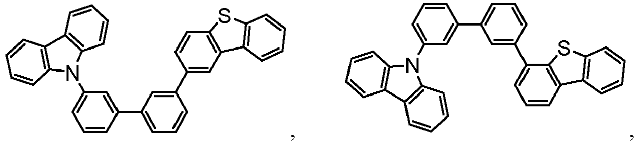

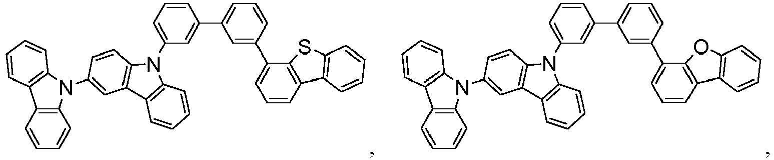

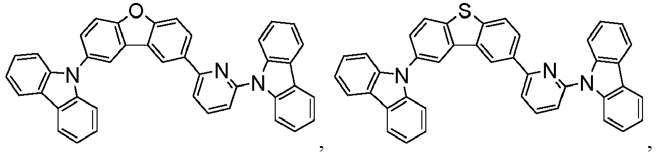

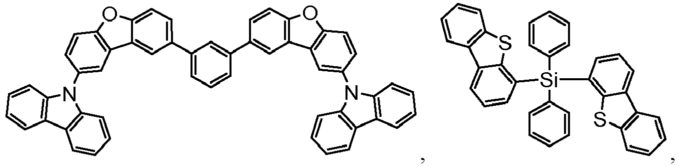

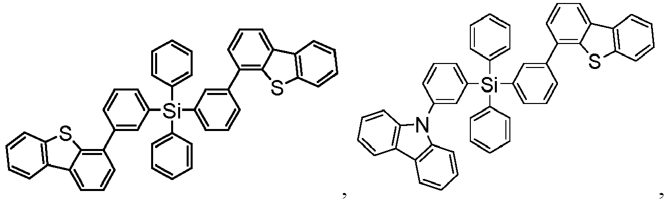

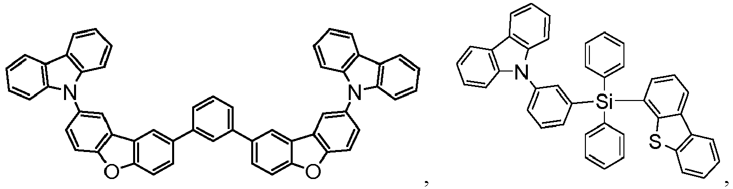

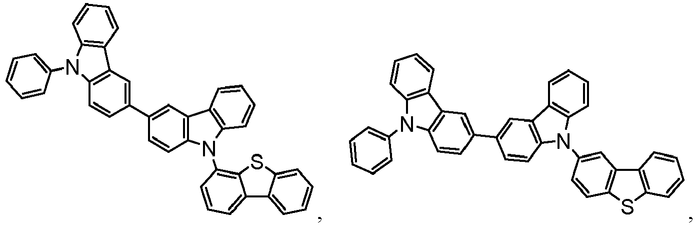

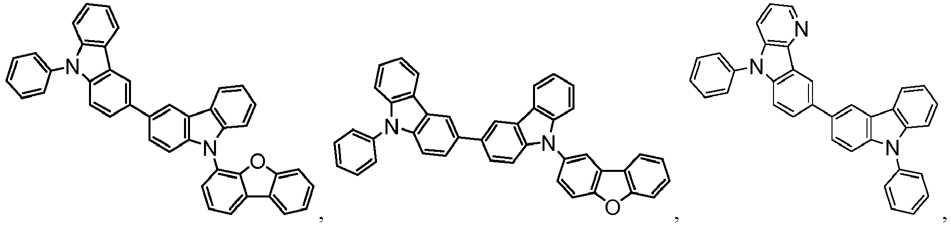

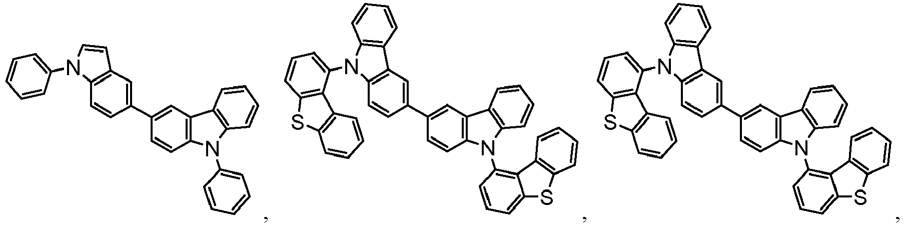

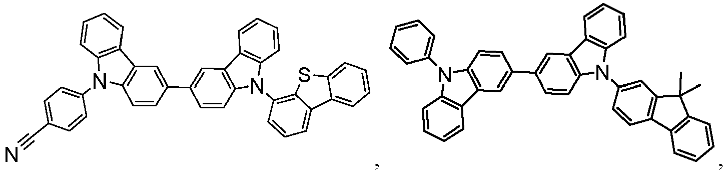

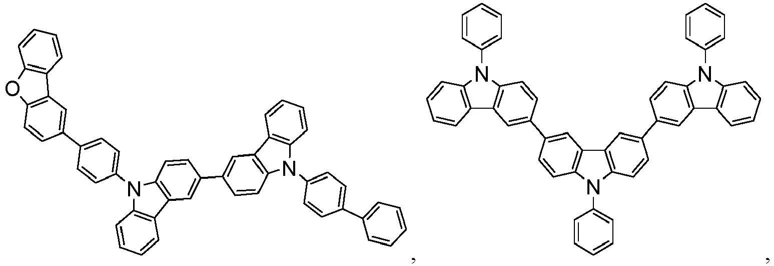

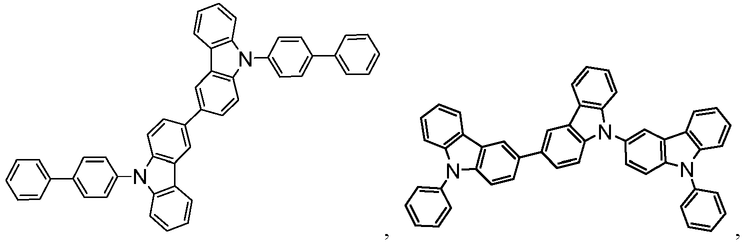

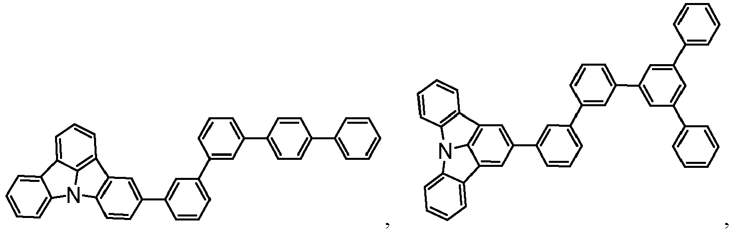

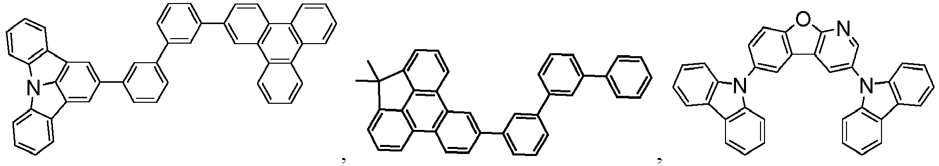

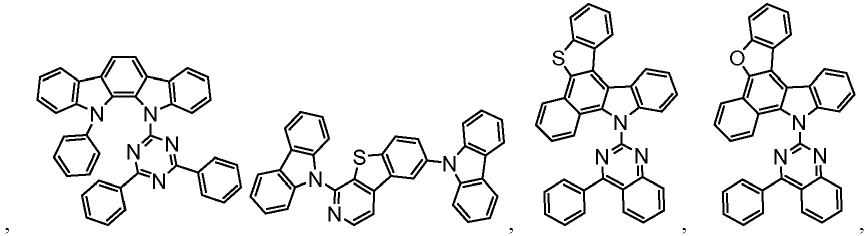

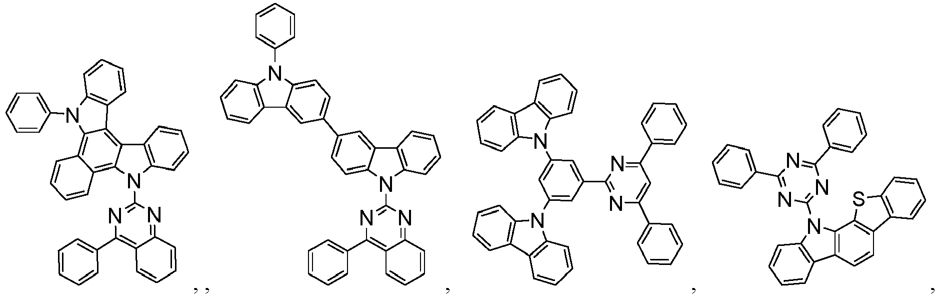

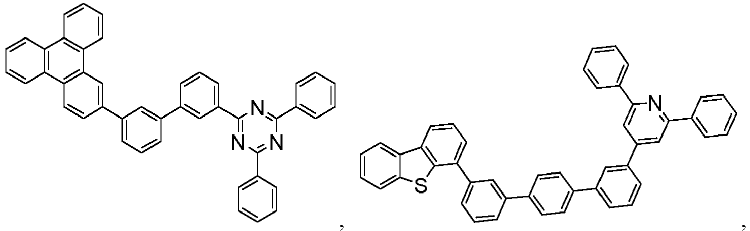

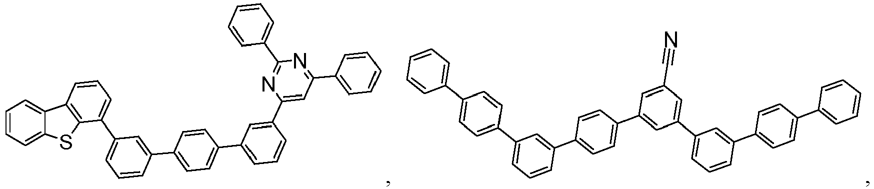

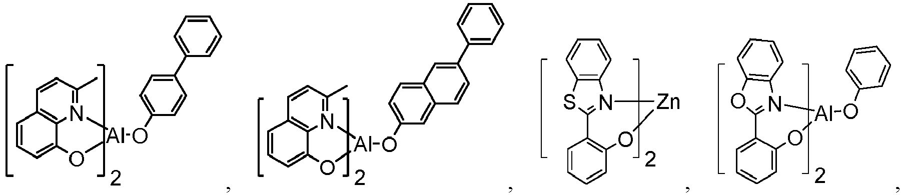

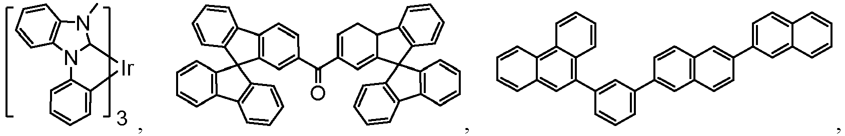

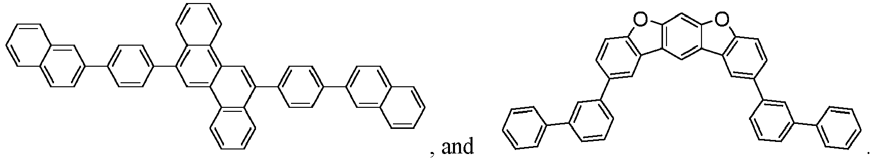

### Additional Emitters:

One or more additional emitter dopants may be used in conjunction with the compound of the present disclosure. Examples of the additional emitter dopants are not particularly limited, and any compounds may be used as long as the compounds are typically used as emitter materials. Examples of suitable emitter materials include, but are not limited to, compounds which can produce emissions via phosphorescence, fluorescence, thermally activated delayed fluorescence, i.e., TADF (also referred to as E-type delayed fluorescence), triplet-triplet annihilation, or combinations of these processes.

Non-limiting examples of the emitter materials that may be used in an OLED in combination with materials disclosed herein are exemplified below together with references that disclose those materials: CN103694277, CN1696137, EB01238981, EP01239526, EP01961743, EP1239526, EP1244155, EP1642951, EP1647554, EP1841834, EP1841834B, EP2062907, EP2730583, JP2012074444, JP2013110263, JP4478555, KR1020090133652, KR20120032054, KR20130043460, TW201332980, US06699599, US06916554, US20010019782, US20020034656, US20030068526, US20030072964, US20030138657, US20050123788, US20050244673, US2005123791, US2005260449, US20060008670, US20060065890, US20060127696, US20060134459, US20060134462, US20060202194, US20060251923, US20070034863, US20070087321, US20070103060, US20070111026, US20070190359, US20070231600, US2007034863, US2007104979, US2007104980, US2007138437, US2007224450, US2007278936, US20080020237, US20080233410, US20080261076, US20080297033, US200805851, US2008161567, US2008210930, US20090039776, US20090108737, US20090115322, US20090179555, US2009085476, US2009104472, US20100090591, US20100148663, US20100244004, US20100295032, US2010102716, US2010105902, US2010244004, US2010270916, US20110057559, US20110108822, US20110204333, US2011215710, US2011227049, US2011285275, US2012292601, US20130146848, US2013033172, US2013165653, US2013181190, US2013334521, US20140246656, US2014103305, US6303238, US6413656, US6653654, US6670645, US6687266, US6835469, US6921915, US7279704, US7332232, US7378162, US7534505, US7675228, US7728137, US7740957, US7759489, US7951947, US8067099, US8592586, US8871361, WO06081973, WO06121811, WO07018067, WO07108362, WO07115970, WO07115981, WO08035571, WO2002015645, WO2003040257, WO2005019373, WO2006056418, WO2008054584, WO2008078800, WO2008096609, WO2008101842, WO2009000673, WO2009050281, WO2009100991,WO2010028151, WO2010054731, WO2010086089, WO2010118029, WO2011044988, WO2011051404, WO2011107491, WO2012020327, WO2012163471, WO2013094620, WO2013107487, WO2013174471, WO2014007565, WO2014008982, WO2014023377, WO2014024131, WO2014031977, WO2014038456, WO2014112450. 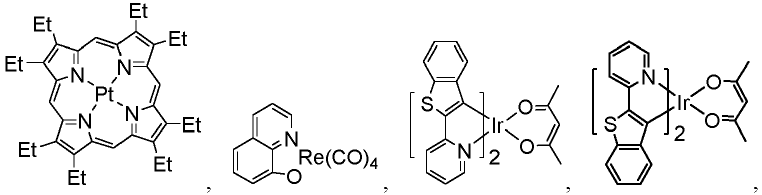 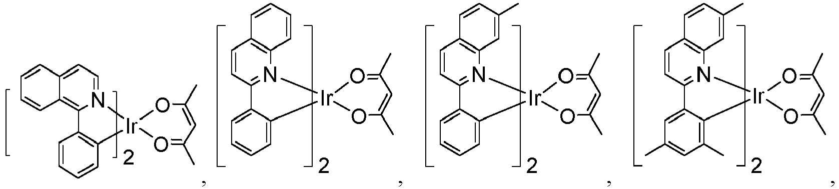 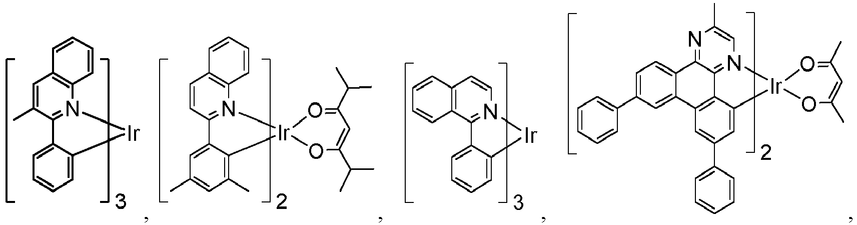 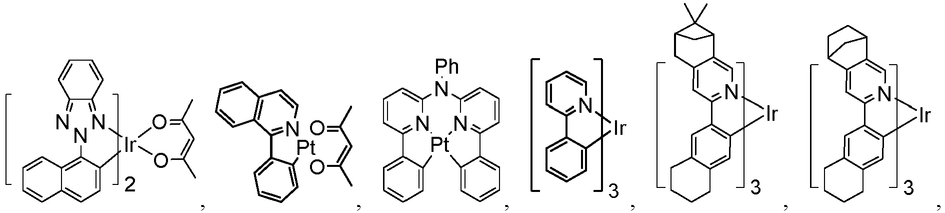 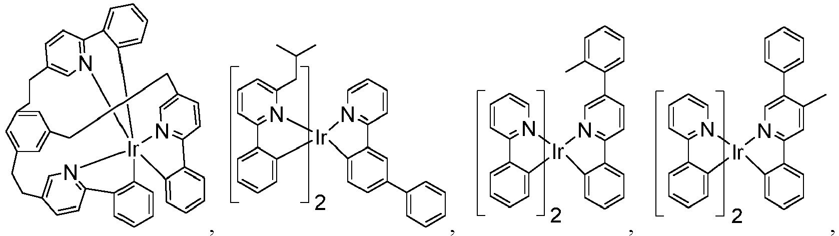 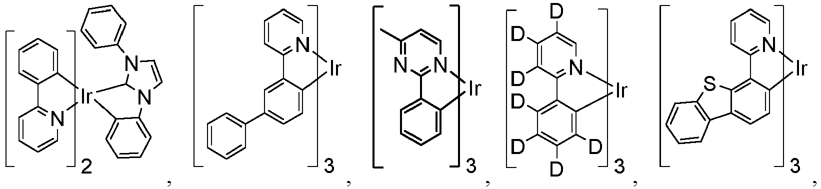 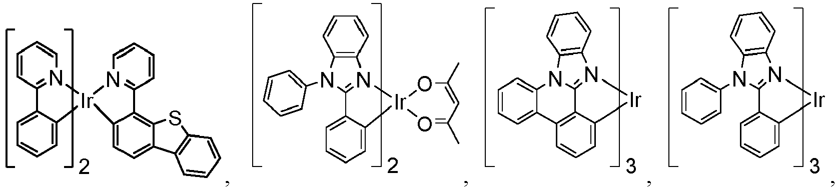 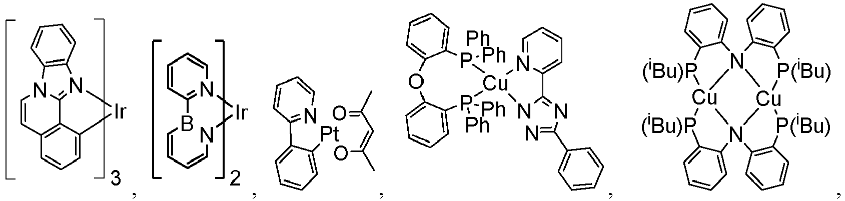 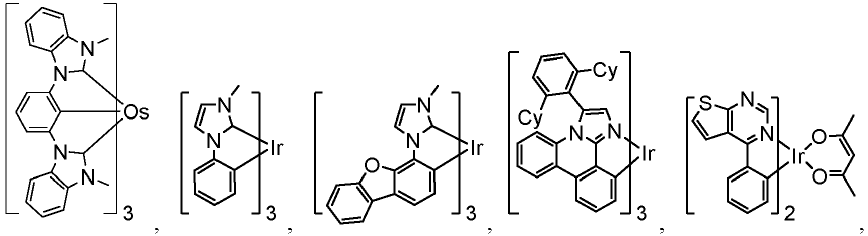 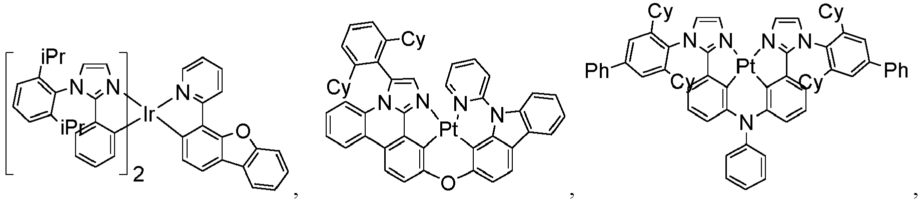 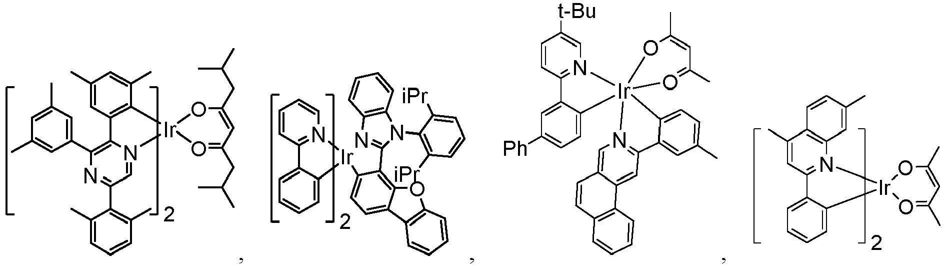 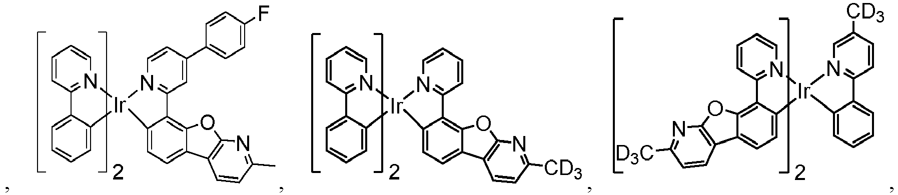 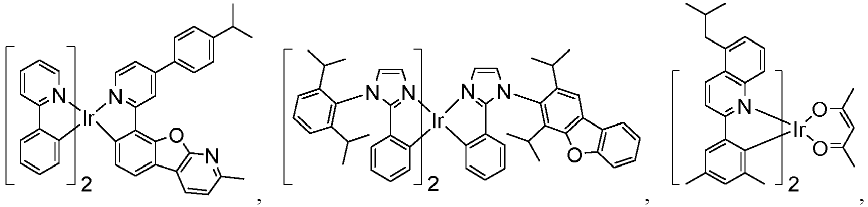 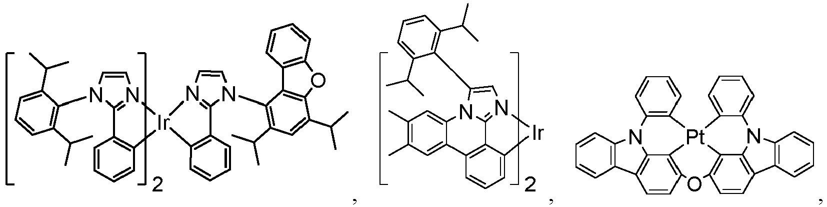 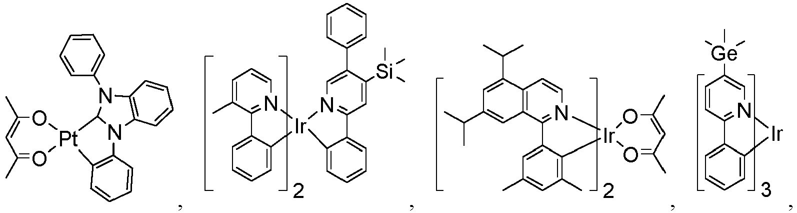 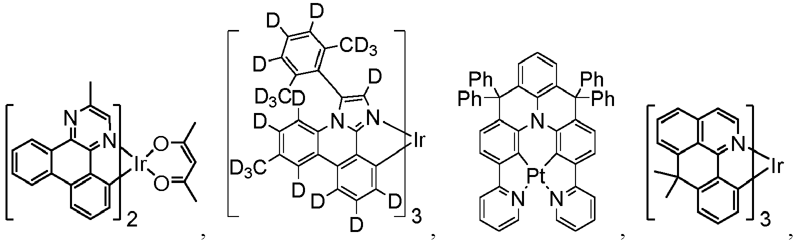 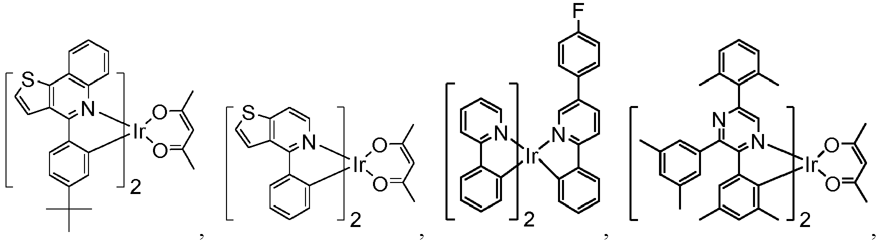 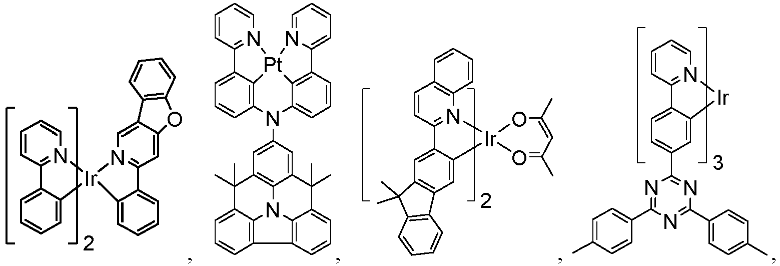 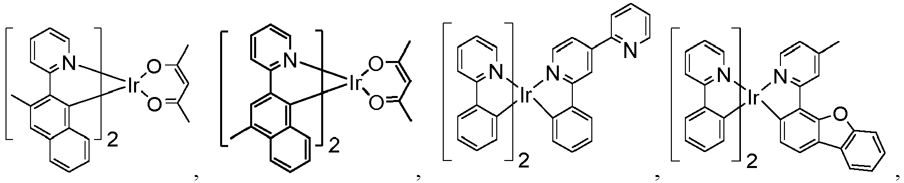 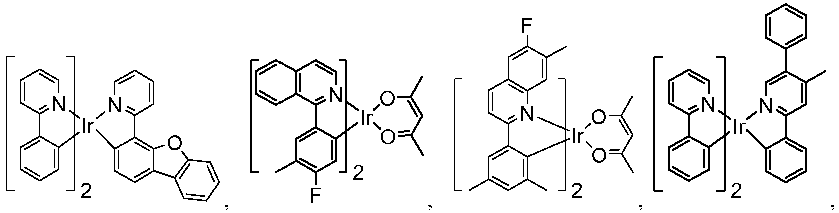 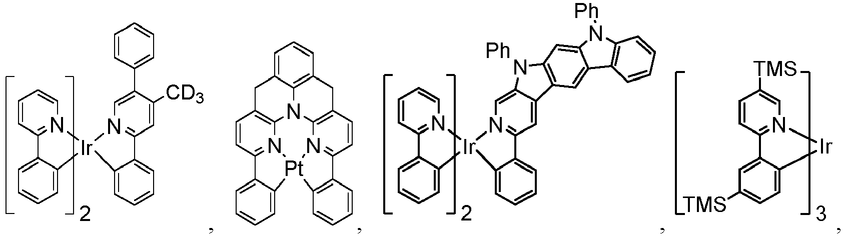 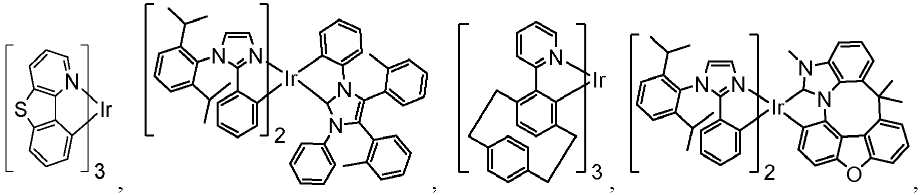 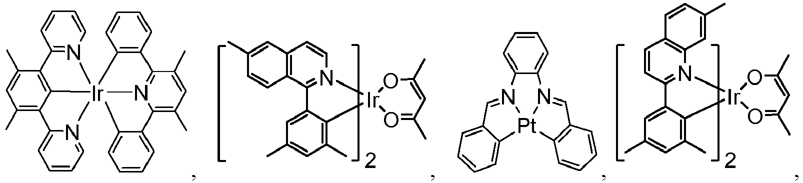 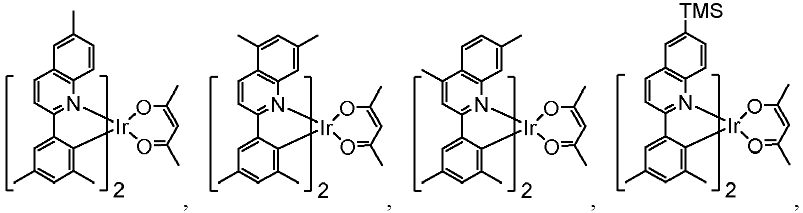 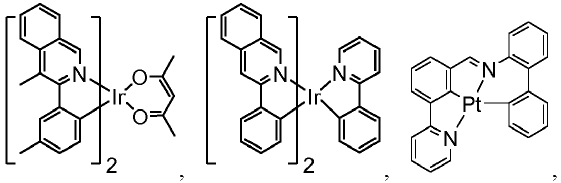 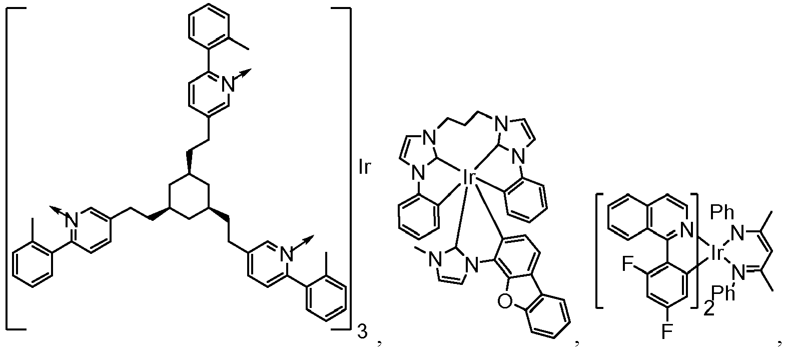 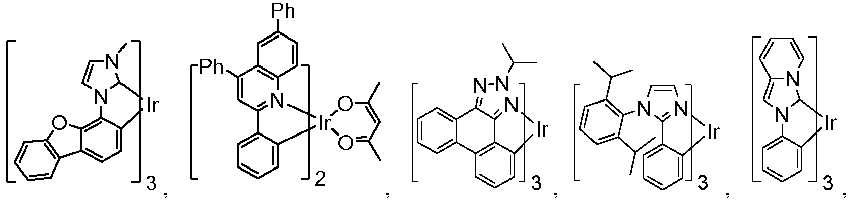 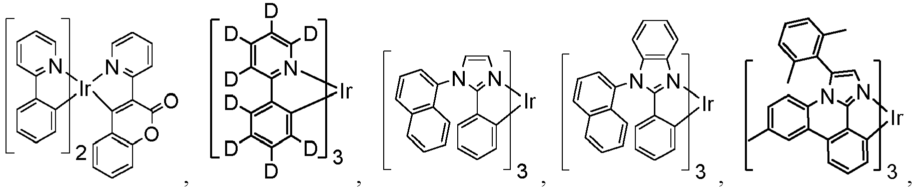 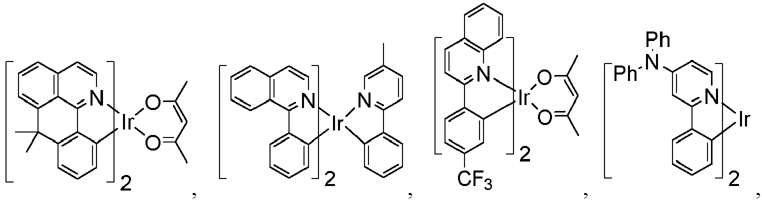 and

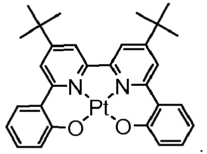

### HBL:

A hole blocking layer (HBL) may be used to reduce the number of holes and/or excitons that leave the emissive layer. The presence of such a blocking layer in a device may result in substantially higher efficiencies and/or longer lifetime as compared to a similar device lacking a blocking layer. Also, a blocking layer may be used to confine emission to a desired region of an OLED. In some embodiments, the HBL material has a lower HOMO (further from the vacuum level) and or higher triplet energy than the emitter closest to the HBL interface. In some embodiments, the HBL material has a lower HOMO (further from the vacuum level) and or higher triplet energy than one or more of the hosts closest to the HBL interface.

In one aspect, compound used in HBL contains the same molecule or the same functional groups used as host described above.

In another aspect, compound used in HBL contains at least one of the following groups in the molecule: 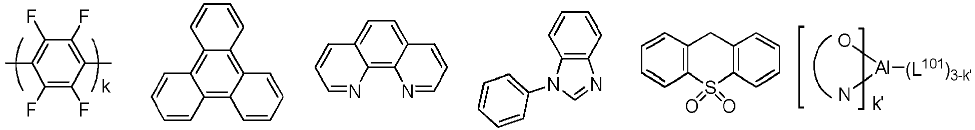 wherein k is an integer from 1 to 20; L¹⁰¹ is an another ligand, k' is an integer from 1 to 3.

### ETL:

Electron transport layer (ETL) may include a material capable of transporting electrons. Electron transport layer may be intrinsic (undoped), or doped. Doping may be used to enhance conductivity. Examples of the ETL material are not particularly limited, and any metal complexes or organic compounds may be used as long as they are typically used to transport electrons.

In one aspect, compound used in ETL contains at least one of the following groups in the molecule: 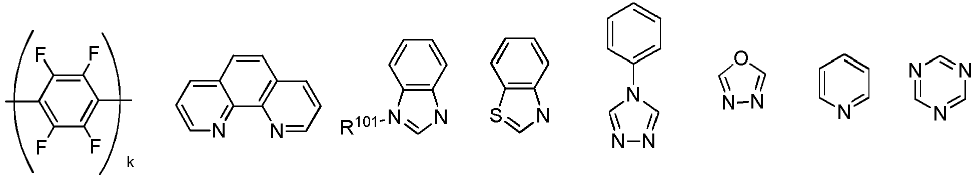 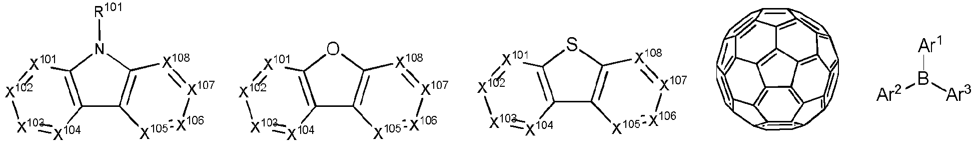 wherein R¹⁰¹ is selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof, when it is aryl or heteroaryl, it has the similar definition as Ar's mentioned above. Ar¹ to Ar³ has the similar definition as Ar's mentioned above. k is an integer from 1 to 20. X¹⁰¹ to X¹⁰⁸ is selected from C (including CH) or N.

In another aspect, the metal complexes used in ETL contains, but not limit to the following general formula: 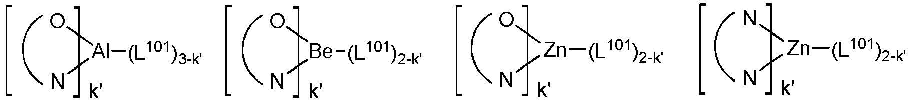 wherein (O-N) or (N-N) is a bidentate ligand, having metal coordinated to atoms O, N or N, N; L¹⁰¹ is another ligand; k' is an integer value from 1 to the maximum number of ligands that may be attached to the metal.

Non-limiting examples of the ETL materials that may be used in an OLED in combination with materials disclosed herein are exemplified below together with references that disclose those materials: CN103508940, EP01602648, EP01734038, EP01956007, JP2004-022334, JP2005149918, JP2005-268199, KR0117693, KR20130108183, US20040036077, US20070104977, US2007018155, US20090101870, US20090115316, US20090140637, US20090179554, US2009218940, US2010108990, US2011156017, US2011210320, US2012193612, US2012214993, US2014014925, US2014014927, US20140284580, US6656612, US8415031, WO2003060956, WO2007111263, WO2009148269, WO2010067894, WO2010072300, WO2011074770, WO2011105373, WO2013079217, WO2013145667, WO2013180376, WO2014104499, WO2014104535,

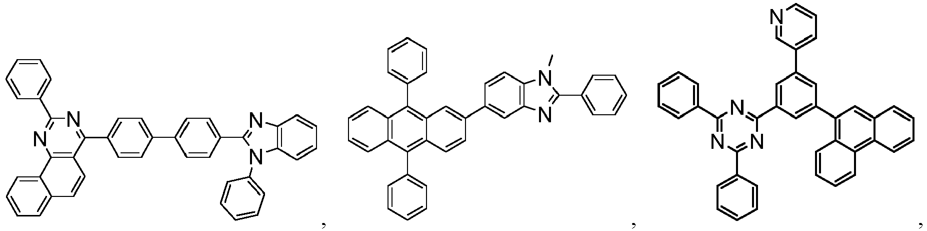

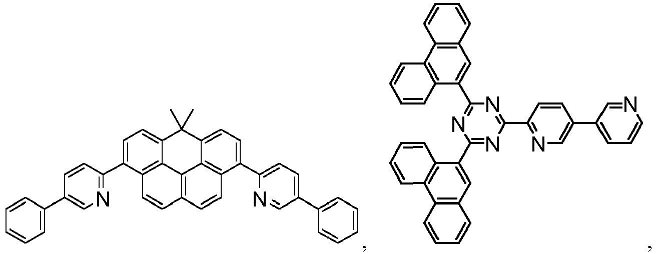

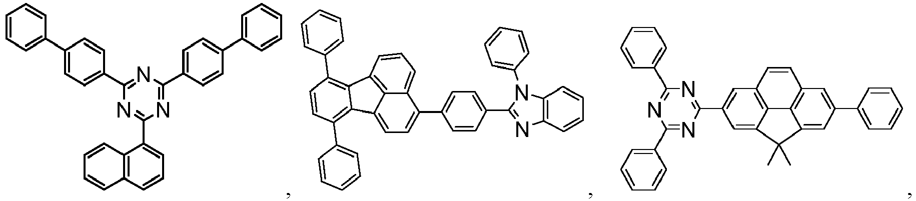

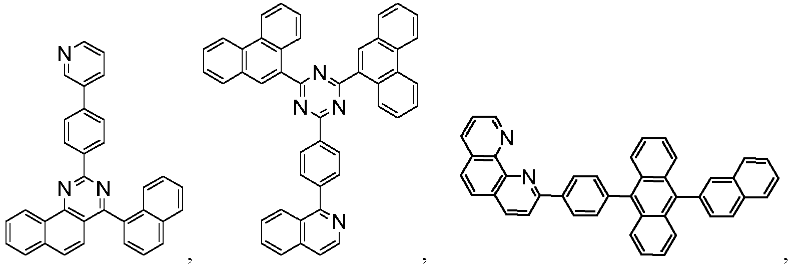

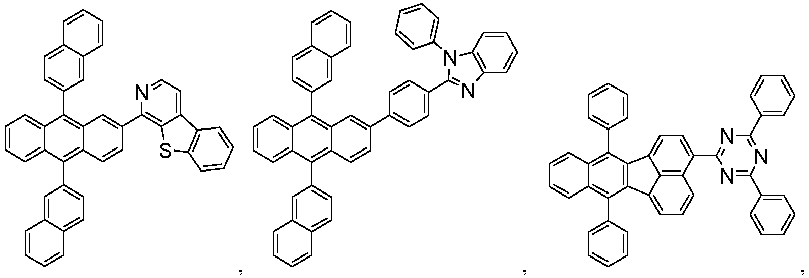

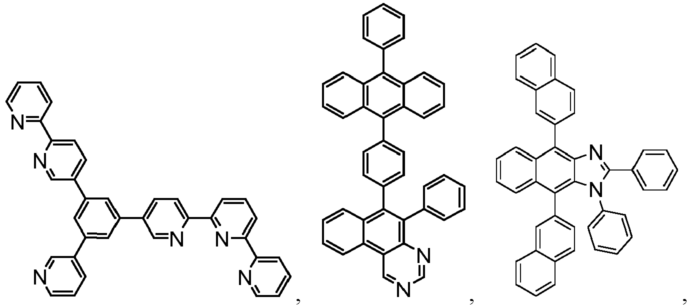

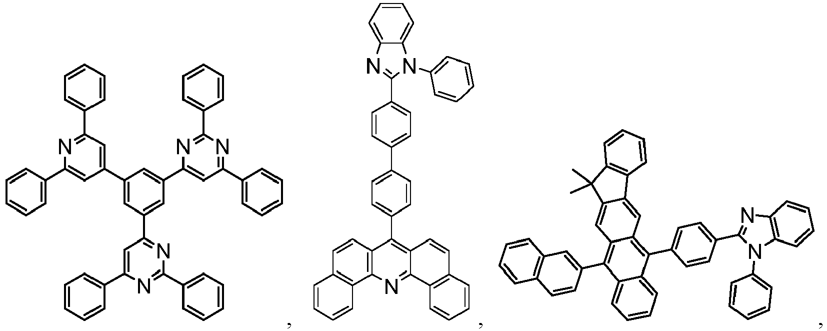

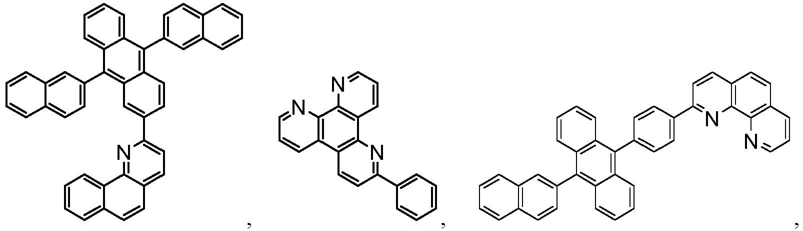

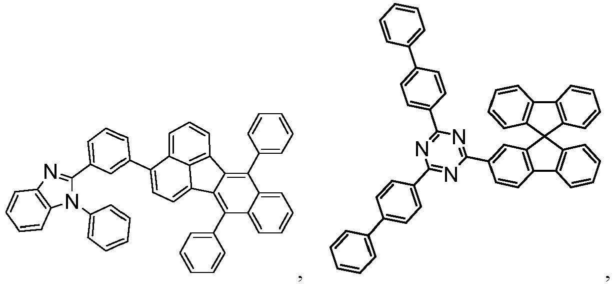

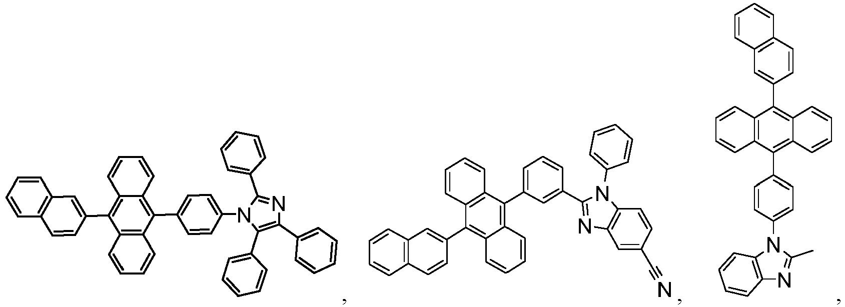

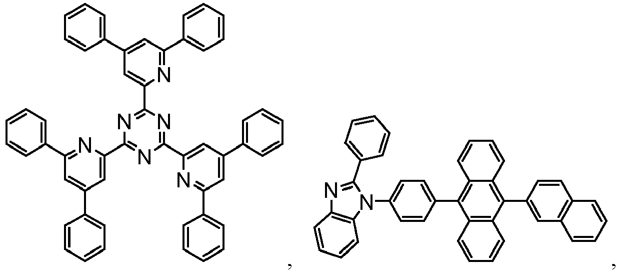

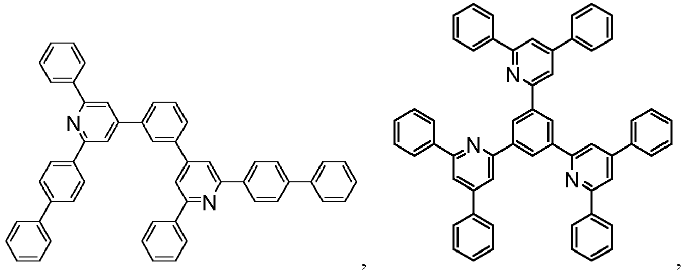

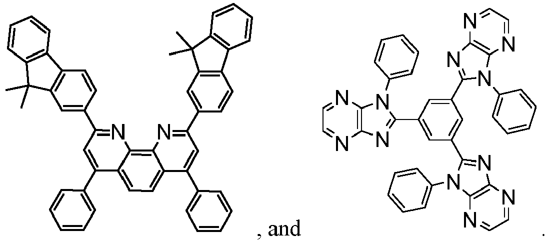

### Charge generation layer (CGL)

In tandem or stacked OLEDs, the CGL plays an essential role in the performance, which is composed of an n-doped layer and a p-doped layer for injection of electrons and holes, respectively. Electrons and holes are supplied from the CGL and electrodes. The consumed electrons and holes in the CGL are refilled by the electrons and holes injected from the cathode and anode, respectively; then, the bipolar currents reach a steady state gradually. Typical CGL materials include n and p conductivity dopants used in the transport layers.

In any above-mentioned compounds used in each layer of the OLED device, the hydrogen atoms can be partially or fully deuterated. Thus, any specifically listed substituent, such as, without limitation, methyl, phenyl, pyridyl, etc. may be undeuterated, partially deuterated, and fully deuterated versions thereof. Similarly, classes of substituents such as, without limitation, alkyl, aryl, cycloalkyl, heteroaryl, etc. also may be undeuterated, partially deuterated, and fully deuterated versions thereof.

### EXPERIMENTAL

### Materials Synthesis

All reactions were carried out under a nitrogen atmosphere unless specified otherwise. All solvents for reactions are anhydrous and used as received from commercial sources.

### Synthesis of Compound 13,376 [Ir(L_{A2496})₂(L_{C5})]

### Synthesis of 4-(3-(tert-butyl)-5-methylphenyl)-7-chlorobenzo[4,5]thieno[3,2-d]pyrimidine

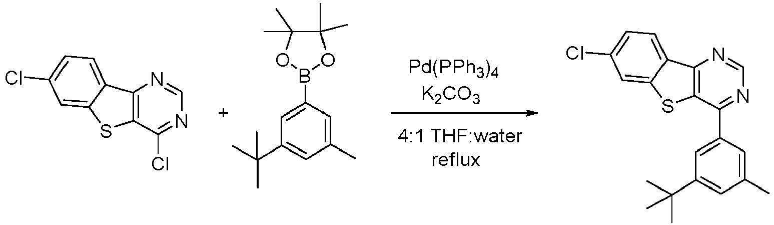

4,7-dichlorobenzo[4,5]thieno[3,2-d]pyrimidine (6.25 g, 24.5 mmol),2-(3-(tert-butyl)-5-methylphenyl)-4,4,5,5-tetramethyl-1,3,2-dioxaborolane (8.06 g, 29.4 mmol), potassium carbonate (10.2 g, 73.5 mmol), THF (200 mL) and water (50 mL) were combined in a flask. The reaction mixture was sparged with nitrogen for 15 minutes, then palladium tetrakis (1.13 g, 0.98 mmol) was added. The reaction was heated to reflux for 16 hours under nitrogen. The mixture was transferred to a separatory funnel with ethyl acetate. The aqueous was partitioned off, then organic phase was washed with brine, dried with sodium sulfate and concentrated down to a brown oil. The pale brown oil was purified with silica gel using 90/8/2 hept/EtOac/DCM solvent system to get 7.3 g of a solid (81% yield).

### Synthesis of 4-(3-(tert-butyl)-5-methylphenyl)-7-(3,3,3-trifluoro-2,2-dimethylpropyl)benzo[4,5]thieno[3,2-d]pyrimidine

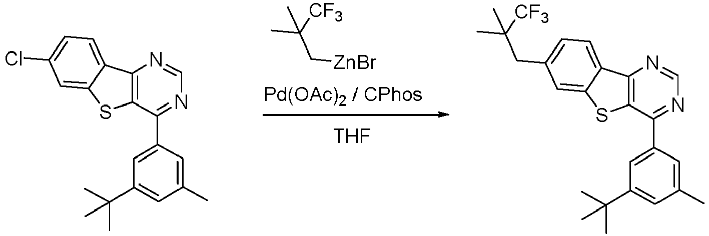

4-(3-(tert-butyl)-5-methylphenyl)-7-chlorobenzo[4,5]thieno[3,2-d]pyrimidine (3.60 g, 9.81 mmol), diacetoxypalladium (0.22 g, 0.98 mmol), and 2,2'-(dicyclohexylphosphanyl)-N2,N2,N6,N6-tetramethyl-[1,1'-biphenyl]-2,6-diamine (0.86 g, 1.96 mmol) were combined in a flask. The system was purged with nitrogen for 15 minutes, then 30 mL THF was added via syringe. The reaction was stirred for 15 minutes to get a light brown solution, then (3,3,3-trifluoro-2,2-dimethylpropyl)zinc(II) bromide (89 mL, 19.6 mmol) was added all at once via syringe. The reaction was stirred at room temperature for 16 hours. The reaction was quenched with sodium bicarbonate solution. The mixture was stirred for 15 minutes, then it was filtered through Celite using ethyl acetate. The aqueous was partitioned off and extracted with ethyl acetate. The combined organics was washed twice with brine, dried with sodium sulfate, filtered and concentrated down to a brown solid. The brown solid was purified with silica gel using 85/15/2 heptane/EtOac/DCM solvent system to get 5.6 g of a brown solid. The brown solid was triturated in hot acetonitrile, then filtered off a nearly white precipitate. The trituration was repeated to get 3.4 g of a white solid. The sample was recrystallized using heptane/ethyl acetate and partially concentrating down on the rotovap to get 1.9 g of a white solid for 42% yield.

### Synthesis of the Ir(III) Dimer

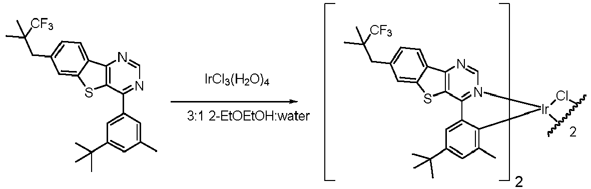

4-(3-(tert-butyl)-5-methylphenyl)-7-(3,3,3-trifluoro-2,2-dimethylpropyl)benzo[4,5]thieno[3,2-d]pyrimidine (1.80 g, 3.95 mmol), 2-ethoxyethanol (21 mL) and water (7 mL) were combined in a flask. The reaction was purged with nitrogen for 15 minutes, then Iridium(III) chloride hydrate (0.45 g, 1.21 mmol) was added. The reaction was heated in an oil bath set at 105°C for 16 hours under nitrogen. The reaction was allowed to cool and then diluted with methanol. The precipitate was filtered off, then dried under vacuum oven for two hours to get 1.70 g (quantitative yield) of an light red solid.

### Synthesis of Compound 13,376

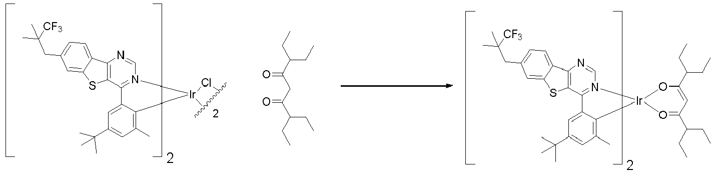

The Ir(III)dimer (1.70 g, 0.75 mmol), 3,7-diethylnonane-4,6-dione (1.3 mL, 5.60 mmol), and 2-ethoxyethanol (20 mL) were combined in a flask. The reaction was sparged with nitrogen for 15 minutes then potassium carbonate (0.77 g, 5.60 mmol) was added. The reaction was stirred at room temperature for 48 hours under nitrogen. The mixture was diluted with methanol then filtered off on a pad of Celite. The precipitate was recovered by adding dichloromethane through the Celite pad. The sample was purified with silica gel using 95/5 heptanes/DCM solvent system to get a red solid which was filtered through an Alumina plug using DCM. Methanol was added to the DCM filtrate, then partially concentrated down on the rotovap. The precipitate was filtered off and dried in the vacuum oven for 16 hours to get 0.54 g (28% yield) of a red solid.

### Synthesis of Comparative Compound 1

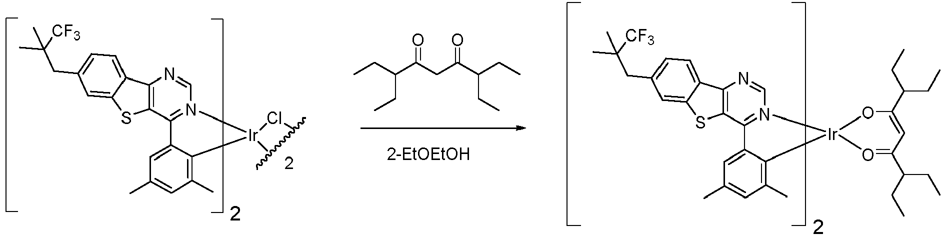

To a 500 mL round bottom flask, the chloro-bridged dimer (1.75 g, 0.95 mmol) and 2-ethoxyethanol (32 mL) were added. Nitrogen was bubbled into the mixture and 1,3-dicyclohexylpropane-1,3-dione (1.7 mL, 7.14 mmol) and potassium carbonate (1.00 g, 7.14 mmol) were added to the mixture. The mixture was stirred for 16 hours at room temperature under nitrogen. The solid was filtered off and washed with ethanol. The solid was taken up in dichloromethane and purified using a silica gel plug wet with heptane and eluted with 50% DCM/heptane. The material was triturated with a DCM/ethanol mixture twice, removing the DCM on a rotovap and filtering off the red solid to afford 1.60 g (77% yield).

### Device Examples

All example devices were fabricated by high vacuum (1,33x 10⁻⁵ Pa (<10⁻⁷ Torr)) thermal evaporation. The anode electrode was 1150 Å of indium tin oxide (ITO). The cathode consisted of 10 Å of Liq (8-hydroxyquinoline lithium) followed by 1,000 Å of Al. All devices were encapsulated with a glass lid sealed with an epoxy resin in a nitrogen glove box (<1 ppm of H₂O and O₂) immediately after fabrication, and a moisture getter was incorporated inside the package. The organic stack of the device examples consisted of sequentially, from the ITO surface, 100Å of HATCN as the hole injection layer (HIL); 450 Å of HTM as a hole transporting layer (HTL); 400 Å of an emissive layer (EML) containing Compound H as a host, a stability dopant (SD) (18%), and Comparative Compound 1 or Compound 13,376 as the emitter (3%); and 350 Å of Liq (8-hydroxyquinoline lithium) doped with 40% of ETM as the ETL. The emitter was selected to provide the desired color, efficiency and lifetime. The stability dopant (SD) was added to the electron-transporting host to help transport positive charge in the emissive layer. The Comparative Example device was fabricated similarly to the device examples except that Comparative Compound 1 was used as the emitter in the EML. Table 1 lists the thicknesses and the materials forming the layers in the devices. The chemical structures of the materials used in the devices are shown after Table 2.

The device performance data are summarized in Table 2. The data for Full Width at Half Maximum (FWHM), Voltage, External Quantum Efficiency (EQE), and LT95% are normalized to Comparative Compound 1. Comparative Compound 1 exhibited a Maximum Wavelength of emission (λ_{MAX}) of 627 nm. The example of the inventive compound, Compound 13,376, showed λ_{MAX} between 621 nm. The FWHM was slightly improved whereas the inventive Compound 13,376 showed a FWHM of 0.96 compared to 1.00 for the Comparative Compound 1. Furthermore, a bulky side chain at the *para* position compared to the Carbon-Iridium bond of the emissive ligand allows good performances for the inventive compound in terms of EQE and lifetime represented by LT95%. The EQE of Compound 13,376 was higher than Comparative Compound 1, going from 1.00 to 1.04. Finally, the device lifetime was much longer for the inventive Compound 13,376 exhibited a LT95% that was 4.5 times higher than Comparative Compound 1.

**Table 1. Device layer materials and thicknesses**

| Layer | Material | Thickness [Å] |
|---|---|---|
| Anode | ITO | 1150 |
| HIL | HATCN | 100 |
| HTL | HTM | 450 |
| EML | Compound H: SD 18%:Emitter 3% | 400 |
| ETL | Liq: ETM 40% | 350 |
| EIL | Liq | 10 |
| Cathode | Al | 1000 |

**Table 2. Performance of the devices with examples of red emitters.**

| **Device Example** | **Emitter** | **1931 CIE** | | **λ max** | **FWHM** | **At 10mA/cm²** | | **At 80 mA/cm2** |
|---|---|---|---|---|---|---|---|---|
| | | **X** | **y** | **[nm]** | **[nm]** | **Voltage [V]** | **EQE [%]** | **LT_{95%} [h]** |
| **Example 1** | Compound 13,376 | 0.66 | 0.34 | 621 | 0.96 | 1.00 | 1.04 | 4.50 |
| **CE1** | Comparative Compound 1 | 0.67 | 0.33 | 627 | 1.00 | 1.00 | 1.00 | 1.00 |

Materials used in the OLED devices in the experiments:

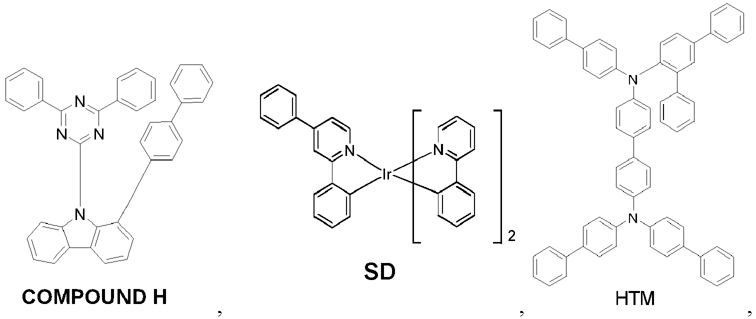

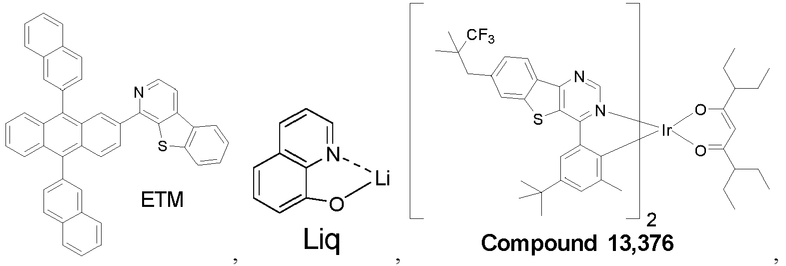

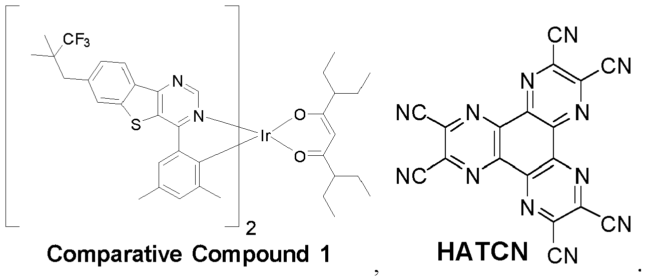

## Claims

1. A compound having a formula M(L_{A})ₓ(L_{B})_{y}(L_{c})_{z}:
wherein the ligand L_{A} has Formula I, 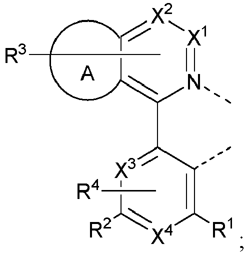
wherein the ligand L_{B} has Formula II, 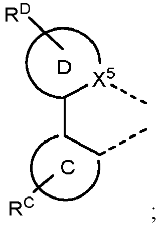
wherein the ligand L_{C} has Formula III, 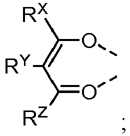
wherein M is a metal having an atomic weight greater than 40;
wherein x is 1, 2, or 3;
wherein y is 0, 1, or 2;
wherein z is 0, 1, or 2;
wherein x+y+z is the oxidation state of the metal M;
wherein X¹ to X⁵ are each independently a carbon or nitrogen;
wherein ring A is a 5- or 6-membered aromatic ring fused to the ring having X¹;
wherein rings C and D are each independently a 5 or 6-membered carbocyclic or heterocyclic ring;
wherein R³, R⁴, R^{C}, and R^{D} each independently represents none to a maximum possible number of substitutions;
wherein each of R³, R⁴, R^{C}, and R^{D}, R^{X}, R^{Y}, and R^{Z} are independently selected from the group consisting of hydrogen, deuterium, halide, alkyl, cycloalkyl, heteroalkyl, arylalkyl, alkoxy, aryloxy, amino, silyl, alkenyl, cycloalkenyl, heteroalkenyl, alkynyl, aryl, heteroaryl, acyl, carbonyl, carboxylic acids, ester, nitrile, isonitrile, sulfanyl, sulfinyl, sulfonyl, phosphino, and combinations thereof;
wherein any adjacent substituents of R³, R⁴, R^{C}, and R^{D}, R^{X}, R^{Y}, and R^{Z} are optionally joined or fused into a ring;
wherein R¹ and R² are each independently selected from the group consisting of alkyl, cycloalkyl, silyl and germanyl;
wherein the alkyl and cycloalkyl can be unsubstituted or substituted with one or more substituents selected from the group consisting of deuterium, halogen, alkyl, cycloalkyl, heteroalkyl, arylalkyl, alkoxy, aryloxy, amino, cyclic amino, silyl, alkenyl, cycloalkenyl, heteroalkenyl, alkynyl, aryl, heteroaryl, acyl, carbonyl, carboxylic acid, ether, ester, nitrile, isonitrile, sulfanyl, sulfinyl, sulfonyl, phosphino, and combinations thereof; and
wherein silyl and germanyl can be unsubstituted, or substituted with a substituent other than H bonded at the relevant position,
wherein R¹ is different from R².

2. The compound of claim 1, wherein M is selected from the group consisting of Ir, Rh, Re, Ru, Os, Pt, Au, and Cu.

3. The compound of claim 1, wherein the compound has a formula M(L_{A})₂(L_{C}) or M(L_{A})(L_{B})₂.

4. The compound of claim 1, wherein X¹ to X⁴ are each a carbon.

5. The compound of claim 1, wherein ring A is a 5-membered aromatic ring or a 6-membered aromatic ring.

6. The compound of claim 1, wherein R¹ and R² are each independently selected from the group consisting of methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, cyclopentyl, cyclohexyl, norbornyl, adamantyl, trimethylsilyl, triethylsilyl, triisopropylsilyl, trimethylgermyl, triethylgermyl, and triisopropylgermyl, partially or fully deuterated variants thereof, partially or fully fluorinated variants thereof, and combinations thereof.

7. The compound of claim 1, wherein R² has at least two more carbon atoms than R¹.

8. The compound of claim 1, wherein each of R³, R⁴, R^{C}, and R^{D}, R^{X}, R^{Y}, and R^{Z} are independently selected from the group consisting of hydrogen, deuterium, alkyl, cycloalkyl, and combinations thereof.

9. The compound of claim 1, wherein ring C is benzene, and ring D is pyridine of which X⁹ is N.

10. The compound of claim 1, wherein the ligand L_{A} is selected from the group consisting of: 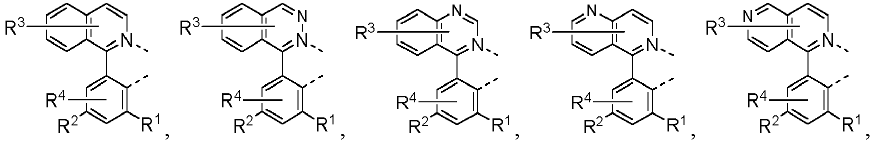 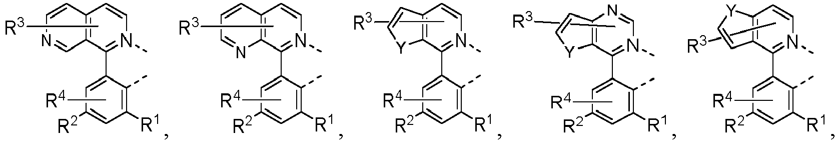 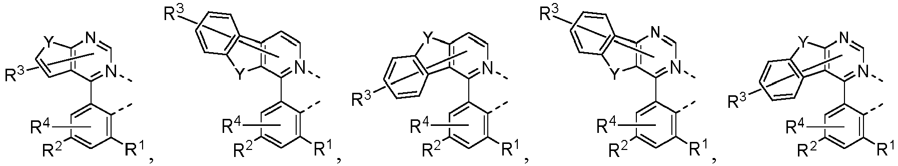 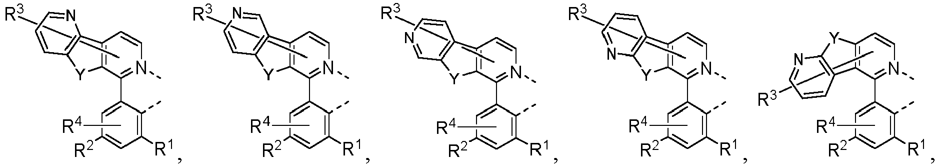 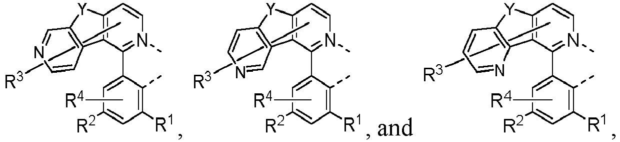 wherein Y is selected from the group consisting of S, O, Se, and N(CH₃).

11. An organic light emitting device (OLED) comprising:
an anode;
a cathode; and
an organic layer, disposed between the anode and the cathode, comprising a compound having a formula M(L_{A})ₓ(L_{B})_{y}(L_{C})_{z} as defined in any one of the previous claims

12. A consumer product comprising an organic light-emitting device (OLED) comprising:
an anode;
a cathode; and
an organic layer, disposed between the anode and the cathode, comprising a compound having a formula M(L_{A})ₓ(L_{B})_{y}(L_{C})_{z} as defined in any one of the claims 1 to 10.

13. The consumer product of claim 12, wherein the consumer product is one of a flat panel display, a computer monitor, a medical monitor, a television, a billboard, a light for interior or exterior illumination and/or signaling, a heads-up display, a fully or partially transparent display, a flexible display, a laser printer, a telephone, a cell phone, a tablet, a phablet, a personal digital assistant (PDA), a wearable device, a laptop computer, a digital camera, a camcorder, a viewfinder, a micro-display, a 3-D display, a virtual reality or augmented reality display, a vehicle, a large area wall, a theater or stadium screen, and a sign.

## Patentansprüche

1. Verbindung mit der Formel M(L_{A})ₓ(L_{B})_{y}(L_{c})_{z}:
wobei der Ligand L_{A} die Formel I, 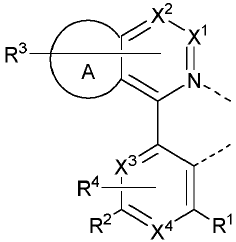 hat;
wobei der Ligand L_{B} die Formel II, 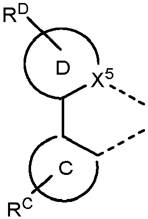 hat;
wobei der Ligand L_{C} die Formel III, 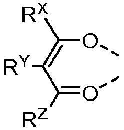 hat;
wobei M ein Metall mit einem Atomgewicht von mehr als 40 ist;
wobei x gleich 1, 2 oder 3 ist;
wobei y gleich 0, 1 oder 2 ist;
wobei z gleich 0, 1 oder 2 ist;
wobei x+y+z die Oxidationsstufe des Metalls M ist;
wobei X¹ bis X⁵ jeweils unabhängig voneinander ein Kohlenstoff oder Stickstoff sind;
wobei Ring A ein 5- oder 6-gliedriger aromatischer Ring ist, der an den Ring mit X¹ kondensiert ist;
wobei die Ringe C und D jeweils unabhängig voneinander ein 5- oder 6-gliedriger carbocyclischer oder heterocyclischer Ring sind;
wobei R³, R⁴, R^{C} und R^{D} jeweils unabhängig voneinander keine bis zu einer maximal möglichen Anzahl von Substitutionen darstellen;
wobei jedes von R³, R⁴, R^{C} und R^{D}, R^{X}, R^{Y} und R^{Z} unabhängig voneinander ausgewählt ist aus der Gruppe bestehend aus Wasserstoff, Deuterium, Halogen, Alkyl, Cycloalkyl, Heteroalkyl, Arylalkyl, Alkoxy, Aryloxy, Amino, Silyl, Alkenyl, Cycloalkenyl, Heteroalkenyl, Alkynyl, Aryl, Heteroaryl, Acyl, Carbonyl, Carbonsäuren, Ester, Nitril, Isonitril, Sulfanyl, Sulfinyl, Sulfonyl, Phosphino und Kombinationen davon;
wobei alle benachbarten Substituenten von R³, R⁴, R^{C} und R^{D}, R^{X}, R^{Y} und R^{Z} gegebenenfalls zu einem Ring verbunden oder kondensiert sind;
worin R¹ und R² jeweils unabhängig voneinander ausgewählt sind aus der Gruppe bestehend aus Alkyl, Cycloalkyl, Silyl und Germanyl;
wobei das Alkyl und Cycloalkyl unsubstituiert oder mit einem oder mehreren Substituenten substituiert sein kann, die ausgewählt sind aus der Gruppe bestehend aus Deuterium, Halogen, Alkyl, Cycloalkyl, Heteroalkyl, Arylalkyl, Alkoxy, Aryloxy, Amino, cyclischem Amino, Silyl, Alkenyl, Cycloalkenyl, Heteroalkenyl, Alkynyl, Aryl, Heteroaryl, Acyl, Carbonyl, Carbonsäure, Ether, Ester, Nitril, Isonitril, Sulfanyl, Sulfinyl, Sulfonyl, Phosphino und Kombinationen davon; und
wobei Silyl und Germanyl unsubstituiert oder mit einem anderen Substituenten als H substituiert sein können, der an die entsprechende Position gebunden ist,
wobei R¹ von R² verschieden ist.

2. Verbindung nach Anspruch 1, wobei M ausgewählt ist aus der Gruppe bestehend aus Ir, Rh, Re, Ru, Os, Pt, Au und Cu.

3. Verbindung nach Anspruch 1, wobei die Verbindung die Formel M(L_{A})₂(L_{C}) oder M(L_{A})(L_{B})₂ aufweist.

4. Verbindung nach Anspruch 1, wobei X¹ bis X⁴ jeweils ein Kohlenstoff sind.

5. Verbindung nach Anspruch 1, wobei Ring A ein 5-gliedriger aromatischer Ring oder ein 6-gliedriger aromatischer Ring ist.

6. Verbindung nach Anspruch 1, wobei R¹ und R² jeweils unabhängig voneinander ausgewählt sind aus der Gruppe bestehend aus Methyl, Ethyl, Propyl, 1-Methylethyl, Butyl, 1-Methylpropyl, 2-Methylpropyl, Pentyl, 1-Methylbutyl, 2-Methylbutyl, 3-Methylbutyl, 1,1-Dimethylpropyl, 1,2-Dimethylpropyl, 2,2-Dimethylpropyl, Cyclopentyl, Cyclohexyl, Norbornyl, Adamantyl, Trimethylsilyl, Triethylsilyl, Triisopropylsilyl, Trimethylgermyl, Triethylgermyl und Triisopropylgermyl, teilweise oder vollständig deuterierte Varianten davon, teilweise oder vollständig fluorierte Varianten davon, und Kombinationen davon.

7. Verbindung nach Anspruch 1, wobei R² mindestens zwei Kohlenstoffatome mehr als R¹ aufweist.

8. Verbindung nach Anspruch 1, wobei jeder der Reste R³, R⁴, R^{C} und R^{D}, R^{X}, R^{Y} und R^{Z} unabhängig voneinander ausgewählt ist aus der Gruppe bestehend aus Wasserstoff, Deuterium, Alkyl, Cycloalkyl, und Kombinationen davon.

9. Verbindung nach Anspruch 1, wobei Ring C Benzol ist und Ring D Pyridin ist, bei dem X⁹ N ist.

10. Verbindung nach Anspruch 1, wobei der Ligand L_{A} ausgewählt ist aus der Gruppe bestehend aus: 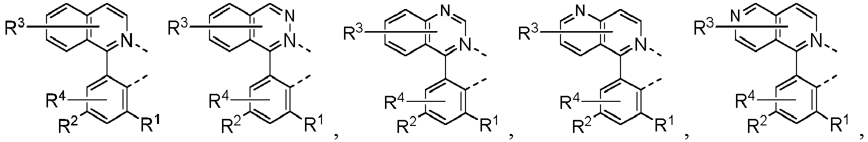 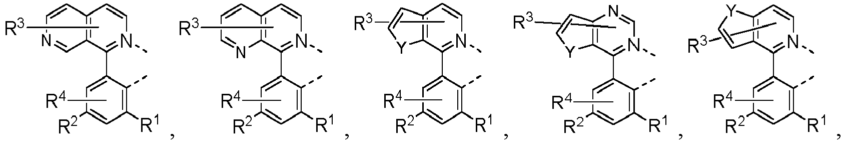 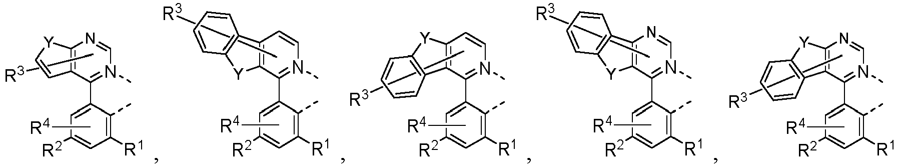 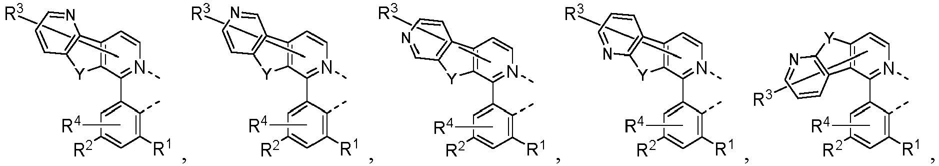 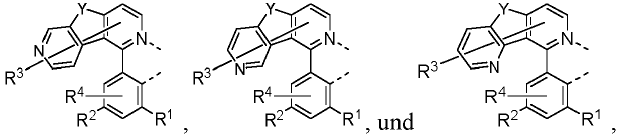 wobei Y ausgewählt ist aus der Gruppe bestehend aus S, O, Se und N(CH₃).

11. Organische lichtemittierende Vorrichtung (OLED), umfassend:
eine Anode;
eine Kathode; und
eine organische Schicht, die zwischen der Anode und der Kathode angeordnet ist und eine Verbindung mit der Formel M(L_{A})ₓ(L_{B})_{y}(L_{C})_{z} wie in einem der vorhergehenden Ansprüche definiert umfasst.

12. Verbraucherprodukt, umfassend eine organische lichtemittierende Vorrichtung (OLED), umfassend:
eine Anode;
eine Kathode; und
eine organische Schicht, die zwischen der Anode und der Kathode angeordnet ist, umfassend eine Verbindung mit einer Formel M(L_{A})ₓ(L_{B})_{y}(L_{C})_{z} wie in einem der Ansprüche 1 bis 10 definiert.

13. Verbraucherprodukt nach Anspruch 12, wobei es sich bei dem Verbraucherprodukt handelt um einen Flachbildschirm, einen Computermonitor, einen medizinischen Monitor, ein Fernsehgerät, eine Werbetafel, eine Leuchte zur Innen- oder Außenbeleuchtung und/oder Signalisierung, ein Heads-up-Display, ein ganz oder teilweise transparentes Display, ein flexibles Display, einen Laserdrucker, ein Telefon, ein Mobiltelefon, ein Tablet, ein Phablet, ein persönlicher digitaler Assistent (PDA), ein tragbares Gerät, ein Laptop, eine Digitalkamera, ein Camcorder, ein Sucher, ein Mikrodisplay, ein 3-D-Display, ein Virtual-Reality- oder Augmented-Reality-Display, ein Fahrzeug, eine großflächige Wand, ein Theater- oder Stadionbildschirm, und ein Schild.

## Revendications

1. Composé de formule M(L_{A})ₓ(L_{B})_{y}(L_{C})_{z} :
dans lequel le ligand L_{A} a la Formule 1 : 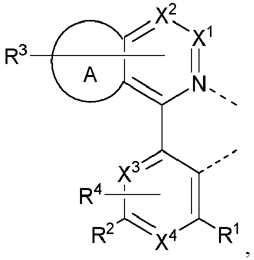
dans lequel le ligand L_{B} a la Formule II : 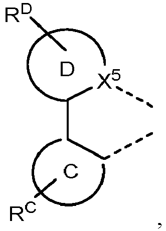
dans lequel le ligand L_{C} a la Formule III: 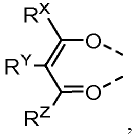
dans lequel M est un métal ayant une masse atomique supérieure à 40,
dans lequel x vaut 1, 2 ou 3,
dans lequel y vaut 0, 1 ou 2,
dans lequel z vaut 0, 1 ou 2,
dans lequel x+y+z est l'état d'oxydation du métal M,
dans lequel X¹ à X⁵ représentent chacun, indépendamment les uns des autres, un carbone ou un azote,
dans lequel le cycle A est un cycle aromatique à 5 ou 6 chaînons fusionné au cycle possédant X¹
dans lequel les cycles C et D représentent chacun, indépendamment l'un de l'autre, un cycle carbocyclique ou hétérocyclique à 5 ou 6 chaînons,
dans lequel R³, R⁴, R^{C} et R^{D} représentent chacun, indépendamment les uns des autres, de zéro substitution à un nombre possible maximal de substitutions,
dans lequel R³, R⁴, R^{C}, et R^{D}, R^{X}, R^{Y}, et R^{Z} sont choisis chacun, indépendamment les uns des autres, parmi le groupe constitué de l'hydrogène, du deutérium, d'un halogénure, d'un alkyle, d'un cycloalkyle, d'un hétéroalkyle, d'un arylalkyle, d'un alcoxy, d'un aryloxy, d'un amino, d'un silyle, d'un alcényle, d'un cycloalcényle, d'un hétéroalcényle, d'un alcynyle, d'un aryle, d'un hétéroaryle, d'un acyle, d'un carbonyle, d'acides carboxyliques, d'un ester, d'un nitrile, d'un isonitrile, d'un sulfanyle, d'un sulfinyle, d'un sulfonyle, d'un phosphino et de combinaisons de ceux-ci,
dans lequel tout substituant adjacent de R³, R⁴, R^{C}, et R^{D}, R^{X}, R^{Y}, et R^{Z} est facultativement lié ou fusionné dans un cycle,
dans lequel R¹ et R² sont choisis chacun indépendamment l'un de l'autre parmi le groupe constitué d'un alkyle, d'un cycloalkyle, d'un silyle et d'un germanyle,
dans lequel l'alkyle et le cycloalkyle peuvent être non substitués ou substitués par un ou plusieurs substituants choisis parmi le groupe constitué du deutérium, d'un halogène, d'un alkyle, d'un cycloalkyle, d'un hétéroalkyle, d'un arylalkyle, d'un alcoxy, d'un aryloxy, d'un amino, d'un amino cyclique, d'un silyle, d'un alcényle, d'un cycloalcényle, d'un hétéroalcényle, d'un alcynyle, d'un aryle, d'un hétéroaryle, d'un acyle, d'un carbonyle, d'un acide carboxylique, d'un éther, d'un ester, d'un nitrile, d'un isonitrile, d'un sulfanyle, d'un sulfinyle, d'un sulfonyle, d'un phosphino et de combinaisons de ceux-ci, et
dans lequel le silyle et le germanyle peuvent être non substitués ou substitués par un substituant autre que le H lié à la position correspondante,
dans lequel R¹ est différent de R².

2. Composé selon la revendication 1, dans lequel M est choisi parmi le groupe constitué d'Ir, Rh, Re, Ru, Os, Pt, Au et Cu.

3. Composé selon la revendication 1, dans lequel le composé a pour formule M(L_{A})₂(L_{C}) ou M(L_{A})(L_{B})₂.

4. Composé selon la revendication 1, dans lequel X¹ à X⁴ représentent chacun un carbone.

5. Composé selon la revendication 1, dans lequel le cycle A est un cycle aromatique à 5 chaînons ou un cycle aromatique à 6 chaînons.

6. Composé selon la revendication 1, dans lequel R¹ et R² sont choisis chacun indépendamment l'un de l'autre parmi le groupe constitué d'un méthyle, éthyle, propyle, 1-méthyléthyle, butyle, 1-méthylpropyle, 2-méthylpropyle, pentyle, 1-méthylbutyle, 2-méthylbutyle, 3-méthylbutyle, 1,1-diméthylpropyle, 1,2-diméthylpropyle, 2,2-diméthylpropyle, cyclopentyle, cyclohexyle, norbornyle, adamantyle, triméthylsilyle, triéthylsilyle, triisopropylsilyle, triméthylgermyle, triéthylgermyle et triisopropylgermyle, des variantes de ceux-ci partiellement ou entièrement deutérées, des variantes de ceux-ci partiellement ou entièrement fluorées, et des combinaisons de ceux-ci.

7. Composé selon la revendication 1, dans lequel R² possède au moins deux atomes de carbone de plus que R¹.

8. Composé selon la revendication 1, dans lequel R³, R⁴, R^{C}, et R^{D}, R^{X}, R^{Y}, et R^{Z} sont choisis chacun indépendamment les uns des autres parmi le groupe constitué de l'hydrogène, du deutérium, d'un alkyle, d'un cycloalkyle et de combinaisons de ceux-ci.

9. Composé selon la revendication 1, dans lequel le cycle C est un benzène et le cycle D est une pyridine dont X⁹ est N.

10. Composé selon la revendication 1, dans lequel le ligand L_{A} est choisi parmi le groupe constitué de : 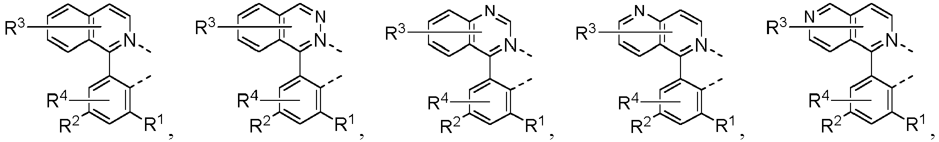 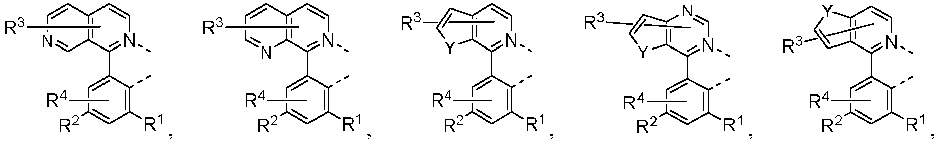 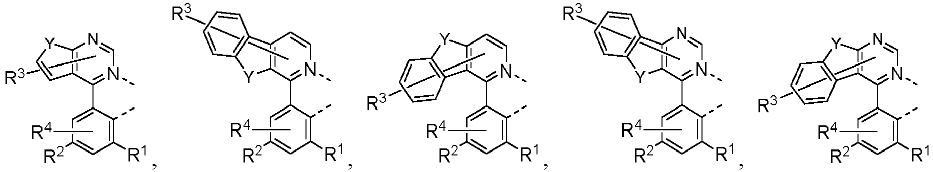 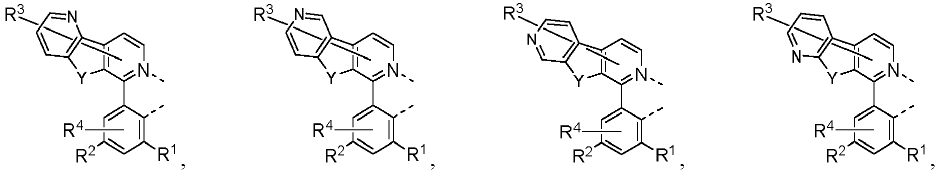 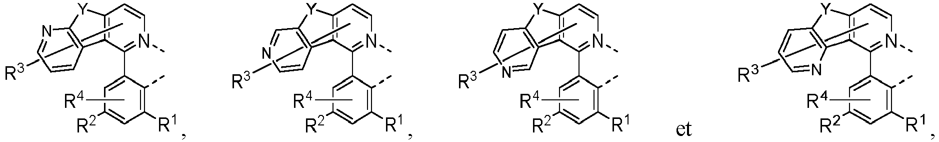 dans lequel Y est choisi parmi le groupe constitué de S, O, Se et N(CH₃).

11. Dispositif électroluminescent organique (OLED) comportant :
une anode,
une cathode, et
une couche organique, disposée entre l'anode et la cathode, comportant un composé de formule M(L_{A})ₓ(L_{B})_{y}(L_{C})_{z} selon l'une quelconque des revendications précédentes.

12. Produit de consommation comportant un dispositif électroluminescent organique (OLED) comportant :
une anode,
une cathode, et
une couche organique, disposée entre l'anode et la cathode, comportant un composé de formule M(L_{A})ₓ(L_{B})_{y}(L_{C})_{z} selon l'une quelconque des revendications 1 à 10.

13. Produit de consommation selon la revendication 12, dans lequel le produit de consommation est un élément parmi un écran plat, un moniteur informatique, un moniteur médical, un téléviseur, un panneau d'affichage, une lumière pour une signalisation et/ou un éclairage intérieur ou extérieur, un affichage tête haute, un écran entièrement ou partiellement transparent, un écran souple, une imprimante laser, un téléphone, un téléphone cellulaire, une tablette, un téléphone-tablette, un assistant numérique personnel (PDA), un dispositif portable, un ordinateur portable, un appareil de prise de vues numérique, un caméscope, un viseur, un micro-écran, un affichage en 3D, un affichage de réalité virtuelle ou de réalité augmentée, un véhicule, un mur de grande surface, un écran de salle de spectacle ou de stade, et une enseigne.
